# EUROPEAN PATENT SPECIFICATION

(11) **EP 3 864 174 B1**
(45) Date of publication and mention of the grant of the patent: **04.12.2024**
(21) Application number: 19779946.3
(22) Date of filing: 08.10.2019
(51) Int. Cl.: C12Q 1/6883, G16B 20/00

(54) **METHOD AND DEVICES FOR AGE DETERMINATION**
VERFAHREN UND VORRICHTUNG ZUR ALTERSBESTIMMUNG
PROCÉDÉ ET DISPOSITIF DE DÉTERMINATION D'ÂGE

(30) Priority: 08.10.2018 EP 18199156
(43) Date of publication of application: 18.08.2021
(73) Proprietor: Thomas J.C. Matzen GmbH, 20148 Hamburg (DE)
(72) Inventor: SCHIEDERIG, Tim, 22523 Hamburg (DE); GUL, Sheraz, London E10 6HT (GB); ZALIANI, Andrea, 22359 Hamburg (DE); CHACHULSKI, Laura, 28329 Bremen (DE); CLAUSSEN, Carsten, 22605 Hamburg (DE)
(74) Representative: Vossius & Partner Patentanwälte Rechtsanwälte mbB
(86) International application number: PCT/EP2019/077252
(87) International publication number: WO 2020/074533

(56) References cited:
- EP-A1- 2 711 431
- WO-A1-2012/162139
- WO-A1-2018/009696
- WO-A1-2018/139826
- WO-A2-2015/048665
- CN-A- 106 947 830
- STEVE HORVATH: "DNA methylation age of human tissues and cell types", GENOME BIOLOGY, BIOMED CENTRAL LTD, vol. 14, no. 10, 21 October 2013 (2013-10-21), pages R115, XP021165700, ISSN: 1465-6906, DOI: 10.1186/GB-2013-14-10-R115
- GREGORY HANNUM ET AL: "Genome-wide Methylation Profiles Reveal Quantitative Views of Human Aging Rates", MOLECULAR CELL, vol. 49, no. 2, 1 January 2013 (2013-01-01), pages 359 - 367, XP055160619, ISSN: 1097-2765, DOI: 10.1016/j.molcel.2012.10.016
- GOEL NEELAM ET AL: "Role of DNA methylation in human age prediction", MECHANISMS OF AGEING AND DEVELOPMENT, ELSEVIER SEQUOIA, LAUSANNE, CH, vol. 166, 24 August 2017 (2017-08-24), pages 33 - 41, XP085191130, ISSN: 0047-6374, DOI: 10.1016/J.MAD.2017.08.012
- WESSEL N VAN WIERINGEN: "Lecture notes on ridge regression", ARXIV.ORG, CORNELL UNIVERSITY LIBRARY, 201 OLIN LIBRARY CORNELL UNIVERSITY ITHACA, NY 14853, 30 September 2015 (2015-09-30), XP081356502
- FRANCESCO GADALETA: "Are we far from correctly inferring gene interaction networks with Lasso?", ARXIV.ORG, CORNELL UNIVERSITY LIBRARY, 201 OLIN LIBRARY CORNELL UNIVERSITY ITHACA, NY 14853, 23 May 2015 (2015-05-23), XP080983857
- BOCKLANDT S ET AL: "Epigenetic Predictor of Age", PLOS ONE,, vol. 6, no. 6, 22 June 2011 (2011-06-22), pages E14821 - 1, XP002687317, ISSN: 1932-6203, DOI: 10.1371/JOURNAL.PONE.0014821
- ALMÉN MARKUS SÄLLMAN ET AL: "Genome-wide analysis reveals DNA methylation markers that vary with both age and obesity", GENE, ELSEVIER, AMSTERDAM, NL, vol. 548, no. 1, 8 July 2014 (2014-07-08), pages 61 - 67, XP029009763, ISSN: 0378-1119, DOI: 10.1016/J.GENE.2014.07.009
- RICCARDO E MARIONI ET AL: "The epigenetic clock is correlated with physical and cognitive fitness in the Lothian Birth Cohort 1936", INTERNATIONAL JOURNAL OF EPIDEMIOLOGY, vol. 44, no. 4, 22 January 2015 (2015-01-22), GB, pages 1388 - 1396, XP055672400, ISSN: 0300-5771, DOI: 10.1093/ije/dyu277
- ELISA GRAZIOLI ET AL: "Physical activity in the prevention of human diseases: role of epigenetic modifications", BMC GENOMICS, BIOMED CENTRAL LTD, LONDON, UK, vol. 18, no. 8, 14 November 2017 (2017-11-14), pages 111 - 123, XP021250742, DOI: 10.1186/S12864-017-4193-5
- FIELD ADAM E ET AL: "DNA Methylation Clocks in Aging: Categories, Causes, and Consequences", MOLECULAR CELL, ELSEVIER, AMSTERDAM, NL, vol. 71, no. 6, 20 September 2018 (2018-09-20), pages 882 - 895, XP085481275, ISSN: 1097-2765, DOI: 10.1016/J.MOLCEL.2018.08.008
- ADIV A. JOHNSON ET AL: "The Role of DNA Methylation in Aging, Rejuvenation, and Age-Related Disease", REJUVENATION RESEARCH, vol. 15, no. 5, 1 October 2012 (2012-10-01), US, pages 483 - 494, XP055672408, ISSN: 1549-1684, DOI: 10.1089/rej.2012.1324
- NAUE JANA ET AL: "Chronological age prediction based on DNA methylation: Massive parallel sequencing and random forest regression", FORENSIC SCIENCE INTERNATIONAL: GENETICS, vol. 31, 1 August 2017 (2017-08-01), pages 19 - 28, XP085298174, ISSN: 1872-4973, DOI: 10.1016/J.FSIGEN.2017.07.015
- PARK JONG-LYUL ET AL: "Identification and evaluation of age-correlated DNA methylation markers for forensic use", FORENSIC SCIENCE INTERNATIONAL: GENETICS, ELSEVIER BV, NETHERLANDS, vol. 23, 17 March 2016 (2016-03-17), pages 64 - 70, XP029567602, ISSN: 1872-4973, DOI: 10.1016/J.FSIGEN.2016.03.005

## Description

The present invention relates to the determination of ages. Specifically, the present invention relates to a method for determining an age indicator, and a method for determining the age of an individual. Said methods are based on data comprising the DNA methylation levels of a set of genomic DNA sequences. Said age indicator is determined by applying on the data a regression method comprising a Least Absolute Shrinkage and Selection Operator (LASSO) in combination with subsequent stepwise regression. Furthermore, the invention relates to the use of an age indicator comprising an ensemble of genomic DNA sequences as set out in the claims for diagnosing the health state of an individual. In further aspects, the invention relates to a chip or a kit, in particular which can be used for detecting the DNA methylation levels of said ensemble of genomic DNA sequences.

### Background

When human beings grow older, their body changes in numerous ways, for example with respect to wear of teeth, joints, weakness of muscles, decrease of the mental capabilities and so forth. However, while health may generally deteriorate as a person grows old, even for persons having the same birth date, there are still large differences in health from individual to individual. Accordingly, some people age faster than others.

Also, it has been found in a study observing ages of twins that only about 25% of the average lifetime is determined by genetic heritage while lifestyle and environmental factors account for the remaining 75% of lifetime variation.

It has been found that some diseases occur more often in human beings with increasing chronological age. However, the chronological age is not the ideal indicator for the age-associated health state of an individual which is often called the "biological age". Determination of an age which is more similar to the biological age might be helpful in assessing whether or not an individual has a higher risk for ageing-related diseases such as Alzheimer's disease. If the determined age is higher than the chronological age, preventive measures, e.g. a change in life-style, might be indicated to prevent or slow down the course of ageing-related diseases. The determination of an alternative age might be also useful for improving diagnostics, for example, evaluating, if a focus should be put on ageing-related diseases or not.

Furthermore, if the chronological age of an individual is not known, the alternative age - despite not being the same - could be used as an indicator of the chronological age. If the alternative age determination is based on a biological sample, it may be used, for example, also in forensics, where traces of blood from an offender are found at a crime scene.

It has further been proposed that certain groups of individuals age slower than others, for example, people in certain countries having specific local habits with respect to nutrition and so forth. Determination of the age of individuals of different groups may help identifying factors influencing the biological age. Reference is made to Alegria-Torres et al., Epigenomics, 2011 June; 3(3): 267-277.

It is noted that where both the chronological age and an age different from the chronological age are known, what could be indicated is a difference to the chronological age rather than the absolute value.

It has been suggested to determine the age of a human being based on the levels of methylation of genomic DNA sequences found in that individual. In particular, reference is made to WO 2012/162139. In WO 2012/162139 it has been suggested to observe cytosine methylation of one or more of CG loci in the genomic DNA selected from a large group of CG loci designations.

Reference is also made to WO 2015/048665 where additional CpG loci are listed.

It has also been suggested in document WO 2012/162139 that one could collect a reference (training) data set of, for example, 100 individuals of varying chronological ages using specific technology platforms and tissues and to then design a specific multivariate linear model that is fit to this reference data set comprising the methylation levels of CpG loci obtained for each individual. For estimating the coefficients, for example, least squares regression has been suggested. The coefficients assigned to each CpG locus would then be used to determine the unknown alternative ages of individuals not included in the training data set. It has been suggested to use a "leave-one-out analysis" in determining these coefficients. In such a "leave-one-out analysis" the multivariate regression model is fit on all but one subject of the reference data set and the prediction is then compared to the chronological age of the left-out subject. Also, tests have been suggested by WO 2012/162139 to screen for top predictors so as to improve the accuracy of the model.

Nonetheless, despite the use of a very large number of CpG loci and substantive experimental and computational effort deriving an age indicator from the very large number of corresponding methylation level measurement values, the average accuracy obtained by WO 2012/162139 is stated to still be only in a range of 3 to 5 years. This demonstrates, that the accuracy and/or efficiency of current age determination methods is suboptimal.

Furthermore, measuring and evaluating a large number of methylation levels is costly.

In this respect it is to be noted that about 28 Million CpG loci can be found in the human genome. Even if it is considered that methylation levels of some of these CpG loci might not be affected by aging, a very large number of CpG loci remain that have methylation levels affected by age. While it is believed that the detection methods used in determining methylation levels might improve over time, allowing to determine the methylation levels of a growing number of CpG loci, it is currently possible already to determine the methylation levels of at least app. 800.000 (800000) CpG loci using commercially available instruments and methods. Still, such measurements are expensive, and thus, the determination of an age based on measuring a very large number of CpG loci would be very expensive. Thus, current age determination methods are based on a few hundreds of CpG loci. However, the costs, equipment and expertise required for determining the age based on a few hundreds of CpG loci are still a road-block for the wide-spread use of current age determining methods.

Accordingly, there is a need for improved age determination methods. In particular, there is a need for improved age determination methods which require less data input while having at least about the same accuracy.

There is further a need for improved means for screening drugs for treating or preventing an ageing-related disease or cancer, or a phenotype associated with an ageing-related disease, or cancer. In particular, such means are also desirable for diagnosing the health state or fitness state of an individual.

It is also desirable to determine an age in a cost-effective manner.

It would also be desirable to allow a determination of an age that even if not very cost-effective and/or not very precise at least allows an independent evaluation of other methods of age determination. In other words, there is a need for an alternative age indicator which can be used to validate the age determined with other age indicators. Such a cross-validation is very important in diagnostics.

### Summary

Means to address the technical problem above are provided in the claims and outlined herein below.

The present invention provides a method for determining an age indicator, and a method for determining the age of an individual. Further disclosed is an ensemble of genomic DNA sequences.

Accordingly, the present invention relates to a computer-implemented method for determining an age indicator comprising the steps of
(a) providing a training data set of a plurality of individuals comprising for each individual
   (i) the DNA methylation levels of a set of genomic DNA sequences and
   (ii) the chronological age, and
(b) applying on the training data set a regression method comprising
   (i) a Least Absolute Shrinkage and Selection Operator (LASSO), thereby determining a reduced training data set, and
   (ii) subsequent stepwise regression, thereby determining the age indicator, preferably wherein the stepwise regression is applied on said reduced training data set,
   wherein the independent variables are the methylation levels of the genomic DNA sequences and preferably wherein the dependent variable is the age,

wherein the age indicator comprises
   (i) a subset of the set of genomic DNA sequences as ensemble and
   (ii) at least one coefficient per genomic DNA sequence contained in the ensemble, and
wherein the reduced training data set comprises all data of the training data set except the DNA methylation levels of the genomic DNA sequences which are eliminated by the LASSO.

The present invention further relates to a computer-implemented method for determining the age of an individual comprising the steps of
(a) providing a training data set of a plurality of individuals comprising for each individual
   (i) the DNA methylation levels of a set of genomic DNA sequences and
   (ii) the chronological age, and
(b) applying on the training data set a regression method comprising
   (i) a Least Absolute Shrinkage and Selection Operator (LASSO), thereby determining a reduced training data set, and
   (ii) subsequent stepwise regression, thereby determining the age indicator, preferably wherein the stepwise regression is applied on said reduced training data set,
   wherein the independent variables are the methylation levels of the genomic DNA sequences and preferably wherein the dependent variable is the age,
   wherein the age indicator comprises
   (i) a subset of the set of genomic DNA sequences as ensemble and
   (ii) at least one coefficient per genomic DNA sequence contained in the ensemble, and wherein the reduced training data set comprises all data of the training data set except the DNA methylation levels of the genomic DNA sequences which are eliminated by the LASSO, and
(c) providing the DNA methylation levels of the individual for whom the age is to be determined of the genomic DNA sequences comprised in the age indicator, and
(d) determining the age of the individual based on its DNA methylation levels and the age indicator.

Furthermore, the present invention relates to a computer-implemented method for determining the age of an individual comprising the steps of
(a) providing an age indicator comprising
   (I) an ensemble of genomic DNA sequences comprising
      at least 70 DNA sequences selected from the group consisting of: cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, and cg23128025; and
   (II) at least one coefficient per genomic DNA sequence contained in the ensemble, wherein said age indicator comprises less than 300 genomic DNA sequences, and preferably wherein said age indicator is obtained by the method for determining an age indicator according to the present invention,
(b) providing the DNA methylation levels of the individual for whom the age is to be determined of the genomic DNA sequences comprised in the age indicator, and
(c) determining the age of the individual based on its DNA methylation levels and the age indicator.

The present invention also relates to a computer-implemented use of an age indicator for diagnosing the health state of an individual, said age indicator comprising
(I) an ensemble of genomic DNA sequences comprising at least 70 DNA sequences selected from the group consisting of: cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, and cg23128025; and
(II) at least one coefficient per genomic DNA sequence contained in the ensemble; and
wherein said age indicator comprises less than 300 genomic DNA sequences; and
preferably, wherein the health state relates to the state of at least one ageing-related disease such as Alzheimer's disease, Parkinson's disease, atherosclerosis, cardio-vascular disease, cancer, arthritis, cataracts, osteoporosis, type 2 diabetes, hypertension, Age-Related Macular Degeneration and/or Benign Prostatic Hyperplasia, at least one phenotype associated with said ageing-related disease(s), and/or cancer.

In addition, the present invention relates to a chip comprising an ensemble of genomic DNA sequences comprising at least 70 DNA sequences selected from the group consisting of: cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, and cg23128025, wherein the chip comprises less than 500 spots and each sequence is contained in a separate spot, and wherein the chip is suitable for detecting the DNA methylation levels of said ensemble of genomic DNA sequences.

A method for determining an age indicator disclosed herein for reference comprises the steps of
(a) providing a training data set of a plurality of individuals comprising for each individual
   (i) the DNA methylation levels of a set of genomic DNA sequences and
   (ii) the chronological age, and
(b) applying on the training data set a regression method comprising a Least Absolute Shrinkage and Selection Operator (LASSO), thereby determining the age indicator and a reduced training data set,
   wherein the independent variables are the methylation levels of the genomic DNA sequences and preferably wherein the dependent variable is the age,
   wherein the age indicator comprises
      (i) a subset of the set of genomic DNA sequences as ensemble and
      (ii) at least one coefficient per genomic DNA sequence contained in the ensemble, and wherein the reduced training data set comprises all data of the training data set except the DNA methylation levels of the genomic DNA sequences which are eliminated by the LASSO.

A method for determining the age of an individual disclosed herein for reference comprises the steps of
(a) providing a training data set of a plurality of individuals comprising for each individual
   (i) the DNA methylation levels of a set of genomic DNA sequences and
   (ii) the chronological age, and
(b) applying on the training data set a regression method comprising a Least Absolute Shrinkage and Selection Operator (LASSO), thereby determining the age indicator and a reduced training data set,
   wherein the independent variables are the methylation levels of the genomic DNA sequences and preferably wherein the dependent variable is the age,
   wherein the age indicator comprises
      (i) a subset of the set of genomic DNA sequences as ensemble and
      (ii) at least one coefficient per genomic DNA sequence contained in the ensemble, and wherein the reduced training data set comprises all data of the training data set except the DNA methylation levels of the genomic DNA sequences which are eliminated by the LASSO, and
(c) providing the DNA methylation levels of the individual for whom the age is to be determined of at least 80%, preferably 100% of the genomic DNA sequences comprised in the age indicator, and
(d) determining the age of the individual based on its DNA methylation levels and the age indicator,
preferably wherein the determined age can be different from the chronological age of the individual.

An ensemble of genomic DNA sequences disclosed herein for reference comprises least one, preferably at least 10, preferably at least 50, preferably at least 70, preferably all of cg1 1330075, cg25845463, cg22519947, cg21807065, cg09001642, cg18815943, cg06335143, cg01636910, cg10501210, cg03324695, cg19432688, cg22540792, cg11176990, cg00097800, cg27320127, cg09805798, cg03526652, cg09460489, cg18737844, cg07802350, cg10522765, cg12548216, cg00876345, cg15761531, cg05990274, cg05972734, cg03680898, cg16593468, cg19301963, cg12732998, cg02536625, cg24088134, cg24319133, cg03388189, cg05106770, cg08686931, cg25606723, cg07782620, cg16781885, cg14231565, cg18339380, cg25642673, cg10240079, cg19851481, cg17665505, cg13333913, cg07291317, cg12238343, cg08478427, cg07625177, cg03230469, cg13154327, cg16456442, cg26430984, cg16867657, cg24724428, cg08194377, cg10543136, cg12650870, cg00087368, cg17760405, cg21628619, cg01820962, cg16999154, cg22444338, cg00831672, cg08044253, cg08960065, cg07529089, cg11607603, cg08097417, cg07955995, cg03473532, cg06186727, cg04733826, cg20425444, cg07513002, cg14305139, cg13759931, cg14756158, cg08662753, cg13206721, cg04287203, cg18768299, cg05812299, cg04028695, cg07120630, cg17343879, cg07766948, cg08856941, cg16950671, cg01520297, cg27540719, cg24954665, cg05211227, cg06831571, cg19112204, cg12804730, cg08224787, cg13973351, cg21165089, cg05087008, cg05396610, cg23677767, cg21962791, cg04320377, cg16245716, cg21460868, cg09275691, cg19215678, cg08118942, cg16322747, cg12333719, cg23128025, cg27173374, cg02032962, cg18506897, cg05292016, cg16673857, cg04875128, cg22101188, cg07381960, cg06279276, cg22077936, cg08457029, cg20576243, cg09965557, cg03741619, cg04525002, cg15008041, cg16465695, cg16677512, cg12658720, cg27394136, cg14681176, cg07494888, cg14911690, cg06161948, cg15609017, cg10321869, cg15743533, cg19702785, cg16267121, cg13460409, cg19810954, cg06945504, cg06153788, and cg20088545, or a fragment thereof which comprises at least 70%, preferably at least 90% of the continuous nucleotide sequence.

Preferably, said ensemble of genomic DNA sequences is comprised in the reduced training data set and/or the age indicator obtained by said method for determining an age indicator.

Further disclosed herein for reference is a gene set comprising at least one, preferably at least 10, preferably at least 30, preferably at least 50, preferably at least 70, preferably all of SIM bHLH transcription factor 1 (SIM1), microtubule associated protein 4 (MAP4), protein kinase C zeta (PRKCZ), glutamate ionotropic receptor AMPA type subunit 4 (GRIA4), BCL10, immune signaling adaptor (BCL10), 5'-nucleotidase domain containing 1 (NT5DC1), suppression of tumorigenicity 7 (ST7), protein kinase C eta (PRKCH), glial cell derived neurotrophic factor (GDNF), muskelin 1 (MKLN1), exocyst complex component 6B (EXOC6B), protein S (PROS1), calcium voltage-gated channel subunit alpha1 D (CACNA1D), kelch like family member 42 (KLHL42), OTU deubiquitinase 7A (OTUD7A), death associated protein (DAP), coiled-coil domain containing 179 (CCDC179), iodothyronine deiodinase 2 (DIO2), transient receptor potential cation channel subfamily V member 3 (TRPV3), MT-RNR2 like 5 (MTRNR2L5), filamin B (FLNB), furin, paired basic amino acid cleaving enzyme (FURIN), solute carrier family 25 member 17 (SLC25A17), G-patch domain containing 1 (GPATCH1), UDP-GlcNAc:betaGal beta-1,3-N-acetylglucosaminyltransferase 9 (B3GNT9), zyg-11 family member A, cell cycle regulator (ZYG11A), seizure related 6 homolog like (SEZ6L), myosin X (MYO10), acetyl-CoA carboxylase alpha (ACACA), G protein subunit alpha i1 (GNAI1), CUE domain containing 2 (CUEDC2), homeobox D13 (HOXD13), Kruppel like factor 14 (KLF14), solute carrier family 1 member 2 (SLC1A2), acetoacetyl-CoA synthetase (AACS), ankyrin repeat and sterile alpha motif domain containing 1A (ANKS1A), microRNA 7641-2 (MIR7641-2), collagen type V alpha 1 chain (COL5A1), arsenite methyltransferase (AS3MT), solute carrier family 26 member 5 (SLC26A5), nucleoporin 107 (NUP107), long intergenic non-protein coding RNA 1797 (LINC01797), myosin IC (MYO1C), ankyrin repeat domain 37 (ANKRD37), phosphodiesterase 4C (PDE4C), EF-hand domain containing 1 (EFHC1), un-characterized LOC375196 (LOC375196), ELOVL fatty acid elongase 2 (ELOVL2), WAS protein family member 3 (WASF3), chromosome 17 open reading frame 82 (C17orf82), G protein-coupled receptor 158 (GPR158), F-box and leucine rich repeat protein 7 (FBXL7), ripply transcriptional repressor 3 (RIPPLY3), VPS37C subunit of ESCRT-I (VPS37C), polypeptide N-acetylgalactosaminyltransferase like 6 (GALNTL6), DENN domain containing 3 (DENND3), nuclear receptor corepressor 2 (NCOR2), endothelial PAS domain protein 1 (EPAS1), PBX homeobox 4 (PBX4), long intergenic non-protein coding RNA 1531 (LINC01531), family with sequence similarity 110 member A (FAM110A), glycosyltransferase 8 domain containing 1 (GLT8D1), G protein subunit gamma 2 (GNG2), MT-RNR2 like 3 (MTRNR2L3), zinc finger protein 140 (ZNF140), kinase suppressor of ras 1 (KSR1), protein disulfide isomerase family A member 5 (PDIA5), spermatogenesis associated 7 (SPATA7), pantothenate kinase 1 (PANK1), ubiquitin specific peptidase 4 (USP4), G protein subunit alpha q (GNAQ), potassium voltage-gated channel modifier subfamily S member 1 (KCNS 1), DNA polymerase gamma 2, accessory subunit (POLG2), storkhead box 2 (STOX2), neurexin 3 (NRXN3), BMS1, ribosome biogenesis factor (BMS1), forkhead box E3 (FOXE3), NADH:ubiquinone oxidoreductase subunit A10 (NDUFA10), relaxin family peptide receptor 3 (RXFP3), GATA binding protein 2 (GATA2), isoprenoid synthase domain containing (ISPD), adenosine deaminase RNA specific B1 (ADARB1), Wnt family member 7B (WNT7B), pleckstrin and Sec7 domain containing 3 (PSD3), membrane anchored junction protein (MAJIN), pyridine nucleotide-disulphide oxidoreductase domain 1 (PYROXD1), cingulin like 1 (CGNL1), chromosome 7 open reading frame 50 (C7orf50), MORN repeat containing 1 (MORN1), atlastin GTPase 2 (ATL2), WD repeat and FYVE domain containing 2 (WDFY2), transmembrane protein 136 (TMEM136), inositol polyphosphate-5-phosphatase A (INPP5A), TBC1 domain family member 9 (TBC1D9), interferon regulatory factor 2 (IRF2), sirtuin 7 (SIRT7), collagen type XXIII alpha 1 chain (COL23A1), guanine monophosphate synthase (GMPS), potassium two pore domain channel subfamily K member 12 (KCNK12), SIN3-HDAC complex associated factor (SINHCAF), hemoglobin subunit epsilon 1 (HBE1), and tudor domain containing 1 (TDRD1).

Preferably, said gene set is obtained by selecting from said ensemble of genomic DNA sequences those genomic DNA sequences which encode a protein, or a microRNA or long non-coding RNA.

Further disclosed for reference is the use of the ensemble of genomic DNA sequences or the gene disclosed herein for diagnosing the health state and/or the fitness state of an individual.

Also disclosed for reference is an *in silico* and/or *in vitro* screening method for identifying a molecule which affects ageing comprising a step of providing the ensemble of genomic DNA sequences or the gene set disclosed herein, wherein the molecule ameliorates, prevents and/or reverses at least one ageing-related disease, at least one phenotype associated with at least one ageing-related disease, and/or cancer when administered to an individual.

Further disclosed for reference is a chip or a kit, in particular which can be used for detecting the DNA methylation levels of the ensemble of genomic DNA sequences or the gene set disclosed herein.

In particular, the chip comprises the genomic DNA sequences or the gene set disclosed herein, wherein each sequence is contained in a separate spot.

As disclosed for reference, the kit may comprise
(a) at least one unique primer pair,
   wherein of each primer pair one primer is a forward primer binding to the reverse strand and the other primer is a reverse primer binding to the forward strand of one the genomic DNA sequences comprised in the ensemble of genomic DNA sequences or one of the genes comprised in the gene set disclosed herein,
   and wherein the two nucleotides which are complementary to the 3' ends of the forward and reverse primers are more than 30 and less than 3000, preferably less than 1000 nucleotides apart, or
(b) at least one probe which is complementary to one of the genomic DNA sequences comprised in the ensemble of genomic DNA sequences or one of the genes comprised in the gene set disclosed herein.

The invention is, at least partly, based on the surprising discovery that an age indicator comprising a further reduced set of genomic DNA sequences, but still having an acceptable quality, could be determined by applying a regression method comprising a Least Absolute Shrinkage and Selection Operator (LASSO), wherein the independent variables are the methylation levels of the genomic DNA sequences and the dependent variable is the age. This was especially surprising as the ridge regression (L2 parameter), which was required in previous methods, was omitted. Further surprisingly, there was very little overlap between the set of genomic DNA sequences determined in the present invention and previously determined sets of genomic DNA sequences. It is thus further surprising that an age indicator comprising very different genomic DNA sequences than known age indicators, but also performing well, could be found.

Reducing the number of genomic DNA sequences while ensuring accurate age determination has many advantages. One advantage is reducing costs, efforts and/or necessary expertise for determining the DNA methylation levels of the genomic DNA sequences, in particular because it allows to use simpler laboratory methods. Another advantage is to narrow down drug target candidates which are encoded by the reduced ensemble of genomic DNA sequences. A further advantage is to provide an alternative or improved tool for diagnosing the health state of an individual. Thus, a method for determining alternative or improved age indicators is also useful for validating the results obtained by other methods, i.e. a diagnosis or drug candidates.

### General terms

Unless defined otherwise, all technical and scientific terms used herein have the same meaning as commonly understood by those skilled in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein refer to one or to more than one (i.e. to at least one) of the grammatical object of the article. By way of example, "an element" means one element or more than one element.

As used herein, "and/or" refers to and encompasses any and all possible combinations of one or more of the associated listed items, as well as the lack of combinations when interpreted in the alternative (or).

It is an object of the invention to provide novelties for the industrial application.

This object is achieved by what is claimed in the independent claims.

Some preferred embodiments are described in the dependent claims.

### Detailed description

In the following described are embodiments and definitions relating to the method for determining an age indicator according to the present invention, the age indicator obtained by said method, the ensemble of genomic DNA sequences comprised in said age indicator, and the method for determining the age of an individual according to the present invention.

As used herein, an age indicator refers to a statistical model which can be used for determining the age of an individual based on the DNA methylation levels of certain genomic DNA sequences of said individual.

The determined age of an individual, as used herein, is not necessarily the same age as the chronological age of said individual. Usually, the determined age and the chronological age of an individual are different, and it is coincidence when they are the same. The determined age is also termed "alternative age" herein. Any age may be counted in "years" and/or, preferably, in "days". The determined age of an individual, as used herein, is a better indicator of the biological age of said individual than his chronological age. The chronological age of an individual refers to the time which has passed since birth of the individual. The biological age, as used herein, relates to the health state of an individual. Preferably, the health state relates to the state of at least one ageing-related disease, at least one phenotype associated with at least one ageing-related disease, and/or cancer, wherein the state indicates the absence, presence, or stage of the disease or the phenotype associated with a disease. Thus, the age indicator of the invention can be used for diagnosing the health state of an individual.

In particular, the age indicator, as used herein, refers to a linear model which comprises independent variables. Herein, an independent variable comprised in the age indicator or a linear model used for generating the age indicator refers to the DNA methylation level of a certain genomic DNA sequence.

Preferably, the dependent variable of the age indicator of the invention and/or the linear model used for generating the age indicator of the invention is the age.

In the linear model, the age of a plurality of individuals is predicted by a set of independent variables (the methylation levels of certain genomic DNA sequences), wherein each independent variable has at least one coefficient. The predicted age and the chronological age preferably correlate very well or, in other words, are preferably in average very similar. However, the predicted age, also termed herein the "determined age", of one individual, may differ, for example for several years, from his chronological age.

Specifically, the methylation level, as used herein, refers to the beta value. The beta value, as used herein, describes the ratio of methylated cytosines over the sum of methylated and unmethylated cytosines among all relevant cytosines within a certain part of the genomic DNA of all alleles of all cells contained in a sample. The methylation state of one particular cytosine molecule is binary and is either unmethylated (0; 0%) or methylated (1; 100%). A methylated cytosine is also termed "5'mC". In consequence, the beta value for a cytosine at a particular position in the genomic DNA of a single cell having two alleles is thus usually either 0, 0.5 or 1. Thus, the beta value at a particular CpG position in the genomic DNA of a population of cells (regardless of the allele number) can take a value between 0 and 1. Furthermore, the beta value when considering all CpGs within a certain genomic DNA sequence of a single allele can take a value between 0 and 1. Preferably herein, only one CpG is considered within a certain genomic DNA sequence. Herein, the sample comprises preferably more than one cell which might comprise more than one allele. Thus, it is evident that the beta value of a genomic DNA sequence, as used herein, can virtually take any value between 0 and 1. Herein, the methylation level of a CpG is defined by the cytosine, and not the guanine, comprised in said CpG.

Preferably herein, CGs/CpGs correspond to Illumina^{™} probes specified by so called Cluster CG numbers (Illumina^{™} methylation probe ID numbers). The methylation levels of a preselected set of CpGs can be measured using an Illumina^{™} DNA methylation array. To quantify the methylation level of a CpG, one can use software to calculate the beta value of methylation.

An Illumina^{™} methylation probe ID is characterized by the term "cg" followed by a number, for example cg11330075 or cg25845463. The terms "CG", "cg", "CpG", "CpG locus", "CpG site", and "eg site" are used interchangeably herein. Determination of DNA methylation levels with Illumina^{™} DNA methylation array is well known, established and can be used in the present invention, although other methods will be described and might be preferred for reasons indicated. Thus, alternatively or in addition, methylation levels of CpGs can be quantified using other methods known in the art as well. Nonetheless, unless indicated otherwise, the CGs/CpGs identified in the present invention correspond to the Illumina^{™} methylation probe IDs.

Furthermore, although possible, it is not required for determining the methylation level of a genomic DNA sequence to determine the methylation of cytosines at a single-nucleotide resolution, but the average methylation signal of relevant cytosines within said sequence is sufficient. Preferably, only cytosines which are followed by a guanine (CpG dinucleotides) are considered relevant herein. The common names for bases and nucleotides, e.g. cytosine and cytidine, respectively, are used interchangeably herein and refer to a specific nucleotide comprising the respective base. Herein, the terms "methylation level" and "DNA methylation level" are used interchangeably. The ranges of 0% to 100% and 0 to 1 are used interchangeably herein when referring to methylation levels.

As used herein, a genomic DNA sequence refers to a coherent part of the genomic DNA of an individual. Herein, a certain genomic DNA sequence does not have to be necessarily identical to the reference sequence of the genomic DNA sequence it relates to, but it may be a variant thereof. Preferably, the genomic DNA sequence is a unique sequence. A skilled person can easily determine if a sequence is a variant of a certain reference genomic DNA sequence by interrogating databases such as "GenBank" or "EMBL-NAR" and using general knowledge.

Herein, the methylation level of a genomic DNA sequence refers to the methylation level of at least one cytosine within at least one CpG dinucleotide comprised in said genomic DNA sequence.

Preferably herein, the methylation level of a genomic DNA sequence refers to the methylation level of exactly one cytosine within exactly one CpG dinucleotide comprised in said genomic DNA sequence. Preferably, said genomic DNA sequence comprises further nucleotides whereof the methylation levels are not considered, but which allow identification of said CpG dinucleotide. Thus herein, a genomic DNA sequence may be defined by a CpG locus.

Very preferably herein, a genomic DNA sequence is defined by an Illumina^{™} methylation probe ID. The terms "Illumina^{™} methylation probe ID", "Illumina^{™} CpG cluster ID", "Illumina^{™} Cluster CG number", "Illumina^{™} probe", Illumina^{™} methylation probe ID number, with or without the terms "Illumina^{™}" or "^{™}", and equivalents thereof, are used interchangeably herein.

A plurality of individuals, as used herein, refers to more than one individual. An individual, as used herein, refers to a living being which has 5'-methylated cytosines (5'-mc) within its genomic DNA. Preferably a living being is a vertebrate, more preferably a mammal, most preferably a human. Preferably, the methylation level of at least one genomic DNA sequence of the individual is associable with ageing and/or the health state of the individual. As used herein, an individual can have any sex, for example, it may be a male, a female, a hermaphrodite or others. Thus, the terms "he", "she", "it", or "his", "her", "its" are used interchangeably herein in the context of an individual.

Usually, the identity of an individual is known, but this is not required. In particular, the age of an individual can be determined by the method of the invention even if the identity and/or the chronological age of the individual is unknown. Thus, the method for determining the age of an individual according to the present invention allows to predict the chronological age of an individual whereof only a biological sample is available. Such a biological sample comprises, for example, hair cells, buccal cells, saliva, blood and/or sperm. Thus, the method for determining the age of an individual is useful for estimating the chronological age of an individual who was present at a crime scene and has left some of his/her biological material there. Furthermore, the method for determining the age of an individual is useful for estimating the chronological age of an individual when no data about the chronological age of said individual have been recorded or are available.

A regression method, as used herein, refers to a statistical process for estimating the relationships among variables, in particular the relationship between a dependent variable and one or more independent variables. Regression analysis is also used to understand which among the independent variables are related to the dependent variable, and to explore the forms of these relationships. Preferably, the regression method comprises a linear regression. Preferably, the regression method comprises a linear regression that uses shrinkage. Shrinkage is where data values are shrunk towards a central point, like the mean. Herein, the regression method comprises a Least Absolute Shrinkage and Selection Operator (LASSO).

LASSO encourages simple, sparse models (i.e. models with fewer parameters). This particular type of regression is well-suited for models showing high levels of multicollinearity or when automation of certain parts of the model selection is desired, like variable selection and/or parameter elimination. LASSO regression performs L1 regularization, which adds a penalty equal to the absolute value of the magnitude of coefficients. This type of regularization can result in sparse models with few coefficients; some coefficients can become zero and eliminated from the model. Larger penalties result in coefficient values closer to zero, which is the ideal for producing simpler models. In other words, LASSO can be used for reducing the number of independent variables of a linear model. The terms "LASSO", "lasso" and "Lasso regression" are used synonymously herein.

In preferred embodiments, the LASSO is performed with the biglasso R package, preferably by applying the command "cv.biglasso". Preferably, the "nfold" is 20.

In preferred embodiments, the LASSO L1 regularization parameter/alpha parameter is 1.

Preferably, the regression method of the invention does not comprise a Ridge regression (L2 regularization) or the L2 regularization parameter/lambda parameter is 0.

In contrast, in the Elastic Net method, the L1 regularization parameter or alpha parameter is not 1, but around 0.1 to 0.9. Furthermore, the Elastic Net method comprises a Ridge regression. Thus, preferably, the regression method of the invention does not comprise an Elastic Net method. Furthermore, the age indicator of the invention is preferably not determined by applying an Elastic Net method.

The regression method of the invention further comprises applying a stepwise regression subsequently to the LASSO. Preferably, the stepwise regression is applied on the reduced training data set.

Thus, the method for determining an age indicator comprises the steps of
(a) providing a training data set of a plurality of individuals comprising for each individual
   (i) the DNA methylation levels of a set of genomic DNA sequences and
   (ii) the chronological age, and
(b) applying on the training data set a regression method comprising
   (i) a Least Absolute Shrinkage and Selection Operator (LASSO), thereby determining a reduced training data set, and
   (ii) subsequent stepwise regression, thereby determining the age indicator, preferably

   wherein said stepwise regression is applied on said reduced training data set,
   wherein the independent variables are the methylation levels of the genomic DNA sequences and preferably wherein the dependent variable is the age,
   wherein the age indicator comprises
      (i) a subset of the set of genomic DNA sequences as ensemble and
      (ii) at least one coefficient per genomic DNA sequence contained in the ensemble, and
   wherein the reduced training data set comprises all data of the training data set except the DNA methylation levels of the genomic DNA sequences which are eliminated by the LASSO.

As disclosed herein for reference the method for determining the age of an individual may comprise the steps of
(a) providing a training data set of a plurality of individuals comprising for each individual
   (i) the DNA methylation levels of a set of genomic DNA sequences and
   (ii) the chronological age, and
(b) applying on the training data set a regression method comprising
   (i) a Least Absolute Shrinkage and Selection Operator (LASSO), thereby determining a reduced training data set, and
   (ii) subsequent stepwise regression, thereby determining the age indicator, preferably

   wherein said stepwise regression is applied on said reduced training data set,
   wherein the independent variables are the methylation levels of the genomic DNA sequences and preferably wherein the dependent variable is the age,
   wherein the age indicator comprises
      (i) a subset of the set of genomic DNA sequences as ensemble and
      (ii) at least one coefficient per genomic DNA sequence contained in the ensemble, and
   wherein the reduced training data set comprises all data of the training data set except the DNA methylation levels of the genomic DNA sequences which are eliminated by the LASSO, and
(c) providing the DNA methylation levels of the individual for whom the age is to be determined of at least 80%, preferably 100% of the genomic DNA sequences comprised in the age indicator, and
(d) determining the age of the individual based on its DNA methylation levels and the age indicator,
preferably wherein the determined age can be different from the chronological age of the individual.

Stepwise regression, as used herein, is a method of fitting regression models in which the choice of predictive variables is carried out by an automatic procedure. In each step, a variable is considered for addition to or subtraction from the set of explanatory variables based on some prespecified criterion. This may take the form of a sequence of F-tests or t-tests, but other techniques are possible, such as adjusted R2, Akaike information criterion (AIC), Bayesian information criterion, Mallows's Cp, PRESS, or false discovery rate. The main approaches are forward selection, backward elimination and bidirectional elimination. Forward selection involves testing the addition of each variable using a chosen model fit criterion, adding the variable (if any) whose inclusion gives the most statistically significant improvement of the fit, and repeating this process until none improves the model to a statistically significant extent. Backward elimination involves testing the deletion of each variable using a chosen model fit criterion, deleting the variable (if any) whose loss gives the most statistically insignificant deterioration of the model fit, and repeating this process until no further variables can be deleted without a statistically significant loss of fit. Bidirectional elimination is a combination of forward selection and backward elimination, testing at each step for variables to be included or excluded. Preferably herein, the variables considered by the stepwise regression are the variables which are selected by the LASSO regression.

In a preferred embodiment, the stepwise regression is a bidirectional elimination. Preferably, statistically insignificant independent variables are removed when applying said stepwise regression. Preferably, the significance level for determining if a variable is added/included or removed/excluded is 0.05.

For determining an age indicator according to the invention, a set of genomic DNA sequences is reduced by the regression method according to the invention in at least one step, preferably in two steps. Preferably, the starting set of genomic DNA sequences is preselected from genomic DNA sequences whereof the methylation level is associable with chronological age. Such a preselected set is, for example, an Illumina^{™} DNA methylation array. Then, the LASSO is applied thereby determining an age indicator and a reduced training data set, which both comprise an ensemble of genomic DNA sequences.

In certain embodiments, the set of genomic DNA sequences comprised in the training data set is preselected from genomic DNA sequences whereof the methylation level is associable with chronological age. Preferably, the preselected set comprises at least 400000, preferably at least 800000 genomic DNA sequences. Particularly suitable are sequences assayed by the Infinium MethylationEPIC BeadChip Kit.

In certain embodiments, the genomic DNA sequences comprised in the training data set are not overlapping with each other and/or only occur once per allele. This is particularly preferred, when only a comparably small set of genomic DNA sequence is preselected, i.e. less than 10000.

In a preferred embodiment, the stepwise regression is applied on the reduced training data set, thereby determining an age indicator comprising an ensemble of genomic DNA sequences.

It has been further surprisingly found that the ensemble of genomic DNA sequences determined by applying LASSO and subsequent stepwise regression is smaller and the respective age indicator has a better performance than the ensemble of genomic DNA sequences or the age indicator determined by applying only LASSO without stepwise regression.

It was further surprisingly found that, although having less variables, an age indicator determined by applying LASSO and subsequent stepwise regression had an accuracy which was at least about as high or even improved compared to prior art methods such as in Horvath, Genome Biology 2013, 14:R115.

Herein, the subset comprised in the age indicator is also termed "ensemble" or "ensemble of genomic DNA sequences". As used herein, the subset of genomic DNA sequences (ensemble) is maximally as big as the set of genomic DNA sequences.

Preferably, the ensemble comprised in the age indicator of the invention is smaller than the set of genomic DNA sequences used for determining said age indicator.

Preferably, the ensemble comprised in the age indicator of the invention is smaller than the set of genomic DNA sequences comprised in the reduced training data set used for determining said age indicator.

In certain embodiments, the reduced training data set comprises at least 90, preferably at least 100, preferably at least 140 genomic DNA sequences.

In certain embodiments, the reduced training data set comprises less than 5000, preferably less than 2000, preferably less than 500, preferably less than 350, preferably less than 300 genomic DNA sequences.

The set of genomic DNA sequences comprised in the reduced training data set is preferably much reduced compared to the preselected set of genomic DNA sequences, preferably more than 90%, preferably more than 99%, preferably more than 99.9%. However, said set of genomic DNA sequences must be large enough to not prematurely limit the optimization potential of subsequent stepwise regression and/or to not obtain an age indicator with a weak performance. Herein, it is contemplated that an age indicator comprising less than 30 genomic DNA sequences has a rather weak performance compared to an age indicator comprising at least 30, preferably at least 50, preferably at least 60, preferably at least 80 genomic DNA sequences. However, an age indicator comprising as few genomic DNA sequences as possible, is preferred.

Thus, in certain embodiments, the age indicator comprises at least 80 genomic DNA sequences.

In preferred embodiments, the age indicator comprises less than 300, preferably less than 150, preferably less than 110, preferably less than 100, preferably less than 90 genomic DNA sequences.

In very preferred embodiments, the age indicator comprises 80 to 100, preferably 80 to 90, preferably 88 genomic DNA sequences.

Furthermore, the age indicator of the invention comprises at least one coefficient per genomic DNA sequence contained in the ensemble. Since one coefficient is sufficient, the age indicator preferably comprises exactly one coefficient per genomic DNA sequence contained in the ensemble.

A coefficient, as used herein, refers to the weight of an independent variable, which herein is the methylation level of a certain genomic DNA sequence. For predicting or determining the age of an individual, the coefficient is multiplied with the methylation level of the genomic DNA sequence or, in other words, a weight is put on each genomic DNA sequence and its methylation level; and then all weighted methylation levels are summed up. Preferably, the methylation level is between 0 and 1 (unmethylated and fully methylated, respectively).

Herein, the data set which is used for generating an age indicator is also called the "training data set". As used herein, a reduced training data set refers to a training data set whereof the data of certain genomic DNA sequences are eliminated or not considered. Herein, a reduced training data set is determined by applying a regression method comprising LASSO on a training data set.

In preferred embodiments, the training data set comprises a matrix comprising as columns the methylation levels of the genomic DNA sequences comprised in the set of genomic DNA sequences and as rows the plurality of individuals. Preferably, the chronological age of said individuals is comprised in a further column of the matrix.

In certain embodiments, the age indicator of the invention is iteratively updated comprising adding the data of at least one further individual to the training data in each iteration, thereby iteratively expanding the training data set.

It is expected that said iterative updating of the age indicator iteratively improves the performance of the age indicator, in particular its accuracy.

Herein, iterative updating refers to consecutive rounds of updating the age indicator. As used herein, one round of updating or updating round is specified in certain or preferred embodiments of the invention. As used herein, different rounds of updating may refer to the same or different embodiments. Preferably, each updating round of the iteration is specified by the same embodiments of the invention. A further individual, with regard to updating the age indicator, refers to an individual which has not contributed data to the training data set, but his data are added in an updating round. Expanding the training data set, as used herein, refers to adding the data of at least one further individual to the training data set.

In one updating round the added data of each further individual may comprise the individual's DNA methylation levels of
(i) at least 5%, preferably 50%, more preferably 100% of the set of genomic DNA sequences comprised in the initial or any of the expanded training data sets, and/or
(ii) the genomic DNA sequences contained in the reduced training data set.

Said option (i) refers in particular to the starting set of genomic DNA sequences, in particular to the preselected set of genomic DNA sequences. Typically, this starting set of genomic DNA sequences is large and comprises, for example, at least 800000 genomic DNA sequences. Thus, adding the methylation levels of at least 5% of the starting set to the training data set, provides a sufficiently large training data set which can be used for determining an age indicator. Preferably, the training data set is restricted to the genomic DNA sequences whereof the DNA methylation levels of all individuals comprised therein are present.

Preferably, all genomic DNA sequences (independent variables) which are not present for all individuals who contribute data to the expanded training data set are removed from the expanded training data set. Preferably, updating the age indicator according to said option (i) comprises adding at least 50%, preferably 100% of the set of genomic DNA sequences comprised in the initial or any of the expanded training data sets, in particular if several or many updating rounds are done.

Preferably, in one updating round the set of genomic DNA sequences whereof the methylation levels are added is identical for each of the further individual(s). This is particularly useful to avoid excessive removal of genomic DNA sequences within one round of updating.

Herein, updating of the age indicator can change the ensemble of genomic DNA sequences (independent variables) comprised therein and/or the coefficient(s) of each said genomic DNA sequence. Of note, said option (i) allows to extend, restrict and/or alter said ensemble of genomic DNA sequences, whereas option (ii) only allows to restrict said ensemble of genomic DNA sequences. Both, options (i) and (ii) allow said coefficients to change. An advantage of option (ii) however is, that only the methylation levels of a reduced set of genomic DNA sequences of the at least one further individual must be provided. Furthermore, said option (ii) is particularly useful for further reducing the size of said ensemble of genomic DNA sequences. In other words, option (i) is particularly useful for generating different age indicators for different purposes, for example, age indicators for certain groups of individuals, or to determine different age indicators as a basis for further refinements; option (ii) is particularly useful for fine-tuning and optimizing a generally already useful age indicator, i.e. for further reducing the number of independent variables, for example for its non-personalized off-the-shelf use. Both options (i) and (ii) can be combined to combine the flexibility of option (i), and the streamlining of option (ii).

In certain embodiments, one updating round comprises applying the LASSO on the expanded training data set, thereby determining an updated age indicator and/or an updated reduced training data set.

The training data set to which the data of the at least one further individual are added may be the reduced training data set, which can be the initial or any of the updated reduced training data sets. Preferably, the reduced training data set is the previous reduced training data set in the iteration.

Thus, an updated reduced training data set can result from applying the LASSO on an expanded training data set and/or from adding data of at least one further individual to a reduced training data set.

In preferred embodiments, one updating round comprises applying the stepwise regression on the reduced training data set thereby determining an updated age indicator.

In one updating round, the data of at least one individual may be removed from the training data set and/or the reduced training data set.

In certain embodiments, the training data set, reduced training data set and/or added data, further comprise at least one factor relating to a life-style or risk pattern associable with the individual(s) and/or a characteristic of the individual(s). Preferably, the factor is selected from drug consumption, environmental pollutants, shift work and stress.

The preselection of genomic DNA sequences, and/or the addition and/or removal of the data of an individual may depend on at least one characteristic of the individual. Herein, the characteristic of an individual, is, for example, the ethnos, the sex, the chronological age, the domicile, the birth place, at least one disease and/or at least one life style factor. As used herein, a life style factor may be selected from drug consumption, exposure to an environmental pollutant, shift work or stress.

The training data set and/or the reduced training data set may be restricted to genomic DNA sequences whereof the DNA methylation level and/or the activity/level of an encoded protein is associated with at least one of said characteristics and/or life-style factors.

Selecting the data in the training data set and/or the reduced training data set at any step, i.e. at the start during preselecting genomic DNA sequences and/or during updating said data sets and/or the age indicator, based on life-style factors and/or characteristics of the individuals, allows to determine age indicators which are particularly well suited for determining the age of an individual or a certain group of individuals having a certain combination of said characteristics and/or life-style factors. Furthermore, the application of different age indicators for age determination may be useful for determining certain predispositions of an individual or a group of individuals, for example, a predominant effect of stress or drug consumption. For example, if the determined age of an individual is much higher than expected when using an age indicator which has been optimized for smoking-related ageing than when using an age indicator which has been optimized for shift work related ageing, this may indicate that smoking is a more important factor for the ageing related health state of the individual than the shift work.

Furthermore, the quality of the age indicator may be determined, wherein the determination of said quality may comprise the steps of
(a) providing a test data set of a plurality of individuals who have not contributed data to the training data set comprising for each said individual
   (i) the DNA methylation levels of the set of genomic DNA sequences comprised in the age indicator and
   (ii) the chronological age; and
(b) determining the quality of the age indicator by statistical evaluation and/or evaluation of the domain boundaries,

wherein the statistical evaluation comprises
   (i) determining the age of the individuals comprised in the test data set,
   (ii) correlating the determined age and the chronological age of said individual(s) and determining at least one statistical parameter describing this correlation, and
   (iii) judging if the statistical parameter(s) indicate(s) an acceptable quality of the age indicator or not, preferably wherein the statistical parameter is selected from a coefficient of determination (R²) and a mean absolute error (MAE), wherein a R² of greater than 0.50, preferably greater than 0.70, preferably greater than 0.90, preferably greater than 0.98 and/or a MAE of less than 6 years, preferably less than 4 years, preferably at most 1 year, indicates an acceptable quality, and
wherein evaluation of the domain boundaries comprises
   (iv) determining the domain boundaries of the age indicator,
   wherein the domain boundaries are the minimum and maximum DNA methylation levels of each genomic DNA sequence comprised in the age indicator and
   wherein said minimum and maximum DNA methylation levels are found in the training data set which has been used for determining the age indicator, and
   (v) determining if the test data set exceeds the domain boundaries, wherein not exceeding the domain boundaries indicates an acceptable quality.

As used herein, a test data set is a data set which can be used for evaluating an age indicator that has been determined based on a training data set. Usually, said training data set and test data set have the same structure. In particular, the test data set and the training data set comprise the same set of genomic DNA sequences. As essential difference however, the test data set only contains data of individuals who have not contributed data to the respective training data set.

Evaluation of an age indicator, as used herein, comprises statistical evaluation and/or evaluation of the domain boundaries.

For the statistical evaluation, the age of the individuals of the test data set is determined and compared to the chronological age of said individuals. Any statistical measurement or parameter which is commonly used to describe the correlation of two variables can be applied. Preferably, the statistical parameter is selected from a coefficient of determination (R²) and a mean absolute error (MAE). Preferably, a R² of greater than 0.50, preferably greater than 0.70, preferably greater than 0.90, preferably greater than 0.98 and/or a MAE of less than 6 years, preferably less than 4 years, preferably at most 1 year, indicates an acceptable quality. If not specified herein, a skilled person can evaluate the result of the measurement or the parameter based on common knowledge. In case of doubt, the quality should be judged as not acceptable.

If the test data set is not fully contained within the boundaries of the domain of an age indicator, the age indicator is judged to not have an acceptable quality. The domain boundaries of an age indicator, as used herein, refer to the minimum and maximum DNA methylation levels of each genomic DNA sequence comprised in the age indicator. More specifically, said minimum and maximum DNA methylation levels are found in the training data set which has been used for determining the age indicator.

The test data set should have a reasonable size. In particular for the statistical evaluation, it should not be too small, but comprise at least 10, preferably at least 30 individuals, preferably at least 200 individuals. For determination of the domain boundaries, the test data set should additionally not be too large, and thus comprise at most 1000 individuals, preferably at most 200 individuals. If it is larger, some violations of the domain boundaries may be allowable, for example for 5%, preferably for 1% of the individuals of the test data set.

In certain embodiments, the training data set and/or the test data set comprises at least 10, preferably at least 30 individuals, preferably at least 200 individuals. Preferably, the training data set comprises at least 200 individuals and the test data set comprises at least 30 individuals.

Of note, an age indicator which has been judged to not have an acceptable quality, can still be useful for the determination of the age on an individual. The term "acceptable quality", as used herein, refers to the determination of an optimal age indicator, in particular through updating. Thus, an acceptable or unacceptable quality of an age indicator, as used herein, does not relate to the absolute quality of the age indicator, but to its relative quality compared to other age indicators, in particular to age indicators determined in different rounds of updating according to the method of the invention.

In preferred embodiments, the age indicator is updated when its quality is not acceptable. The quality is judged to be acceptable or not acceptable as explained above in the context of evaluation of the age indicator.

In certain embodiments, the age indicator is not further updated when the number of individuals comprised in the data has reached a predetermined value and/or a predetermined time has elapsed since a previous update. The predetermined time may also refer to the number of quality evaluations for potential updating rounds.

For example, if an age indicator comprises already data of many thousands or even millions of individuals, or the last 10 or even 100 evaluations with new test data sets have indicated an acceptable quality, further optimization of the age indicator is not to be expected and the updating may stop.

In certain embodiments, the DNA methylation levels of the genomic DNA sequences of an individual are measured in a sample of biological material of said individual comprising said genomic DNA sequences. Preferably, the sample comprises buccal cells.

Suitable methods for determining DNA methylation levels are, for example, methylation sequencing, bisulfate sequencing, a PCR method, high resolution melting analysis (HRM), methylation-sensitive single-nucleotide primer extension (MS-SnuPE), methylation-sensitive single-strand conformation analysis, methyl-sensitive cut counting (MSCC), base-specific cleavage/MALDI-TOF, combined bisulfate restriction analysis (COBRA), methylated DNA immunoprecipitation (MeDIP), micro array-based methods, bead array-based methods, pyrosequencing and/or direct sequencing without bisulfate treatment (nanopore technology).

The DNA methylation levels of an individual may be measured with a DNA methylation array such as an Illumina^{™} DNA methylation array, preferably an Infinium MethylationEPIC BeadChip Kit. A DNA methylation array is particularly suitable when the DNA methylation levels of a very large number of genomic DNA sequences are to be measured, in particular for starting and/or preselected genomic DNA sequences.

Furthermore, the DNA methylation levels of genomic DNA sequences of an individual may be measured by base-specific cleavage/MALDI-TOF and/or a PCR method, preferably wherein base-specific cleavage/MALDI-TOF is the Agena technology and the PCR method is methylation specific PCR. Base-specific cleavage/MALDI-TOF and/or a PCR method is particularly suitable when DNA methylation levels of a reduced set of genomic DNA sequences is to be measured, in particular for adding data to the reduced training data set and/or providing the methylation levels of an individual for whom the age is to be determined with the age indicator of the invention.

Further details on the determination of DNA methylation levels are explained further below and in the Examples.

The method for determining an age indicator and/or the method for determining the age of an individual disclosed herein may further comprise a step of obtaining a sample of biological material of an individual. The biological material may be derived from any part of the individual, but preferably the sample is obtained non-invasively. Preferably, the individual is not an embryo.

In a preferred embodiment, the sample is obtained from a buccal swab.

Herein, the age indicator of the invention can be used as a tool for determining the age of an individual. Thus, the method for determining the age of an individual according to the invention comprises all steps of the method of the invention for determining an age indicator or comprises a step of providing an age indicator according to the invention. Further, said method of determining the age of an individual comprises the steps of providing the DNA methylation levels of the individual for whom the age is to be determined of the genomic DNA sequences comprised in said age indicator and determining the age of the individual based on its DNA methylation levels and said age indicator.

In other words, the methylation levels of the genomic DNA sequences comprised in the provided age indicator are to be provided for an individual for whom the age is to be determined. The methylation levels of the genomic DNA sequences of said individual which are missing can be imputed, for example by using the median or mean of the provided methylation levels.

In certain embodiments, the age of the individual is determined based on its DNA methylation levels and the updated age indicator. In particular, the age is determined on the updated age indicator when the quality of the initially provided age indicator is not acceptable.

Preferably, the age of the individual is only determined with the age indicator when he/she has not contributed data to the training data set which is or has been used for generating said age indicator.

In certain embodiments, the method for determining the age of an individual further comprises a step of determining at least one life-style factor which is associated with the difference between the determined and the chronological age of said individual.

The ensemble of genomic DNA sequences according to the invention comprises at least 70of cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, and cg23128025.

Preferably, said ensemble of genomic DNA sequences is comprised in the age indicator obtained by the method for determining an age indicator, wherein said method comprises applying a stepwise regression subsequently to the LASSO.

Herein, a gene refers to a genomic DNA sequence which encodes a protein (coding sequence; CDS), or a microRNA or long non-coding RNA. Herein, a genomic DNA sequence which encodes a protein also encodes the mRNA for the translation of said protein. A microRNA (miRNA) is a small non-coding RNA molecule (containing about 22 nucleotides) that functions in RNA silencing and post-transcriptional regulation of gene expression. Long non-coding RNAs (long ncRNAs, IncRNA) are a type of transcripts with typically more than 200 nucleotides which are not translated into proteins (but possibly into peptides). Still, the majority of long non-coding RNAs are likely to be functional, i.e. in transcriptional regulation.

The gene set disclosed herein for reference may comprise at least one, preferably at least 5, preferably at least 10, preferably at least 30, preferably all of ISPD, KCNK12, GNG2, SIRT7, GPATCH1, GRIA4, LINC01531, LOC101927577, NCOR2, WASF3, TRPV3, ACACA, GDNF, EFHC1, MYO10, COL23A1, TDRD1, ELOVL2, GNAI1, MAP4, CCDC179, KLF14, ST7, INPP5A, SIM1, SLC1A2, AS3MT, KSR1, DSCR6, IRF2, KCNS1, NRXN3, C11orf85, HBE1, FOXE3, TMEM136, HOXD13, LOC375196, PANK1, MIR107, COL5A1, PBX4, ZNF140, GALNTL6, NUP107, LOC100507250, MTRNR2L5, C17orf82, MKLN1, FURIN, KLHL42, MORN1, ANKS1A, BCL10, DENND3, FAM110A, PROS1, WNT7B, FBXL7, GATA2, VPS37C, NRP1, POLG2, ANKRD37, GMPS, and WDFY2.

Preferably, said gene set is obtained by selecting from the ensemble of genomic DNA sequences those which encode a protein, or a microRNA or long non-coding RNA, wherein said ensemble of genomic DNA sequences is comprised in the age indicator obtained by the method for determining an age indicator, wherein said method comprises applying a stepwise regression subsequently to the LASSO.

The ensemble of genomic DNA sequences disclosed herein may comprise at least one, preferably at least 4, preferably at least 10, preferably all of cg1 1330075, cg00831672, cg27320127, cg27173374, cg14681176, cg06161948, cg08224787, cg05396610, cg15609017, cg09805798, cg19215678, cg12333719, cg03741619, cg03230469, cg19851481, cg10543136, cg07291317, cg26430984, cg16950671, cg16867657, cg13973351, cg16781885, cg04287203, cg27394136, cg10240079, cg02536625, and cg23128025.

Preferably, said ensemble of genomic DNA sequences is comprised in the age indicator obtained by the method for determining an age indicator, wherein said method comprises applying a stepwise regression subsequently to the LASSO, and wherein each coefficient of said genomic DNA sequences comprised in said age indicator has an absolute value of more than 20.

Furthermore, the ensemble of genomic DNA sequences disclosed herein may comprises at least one, preferably at least 4, preferably all of cg11330075, cg00831672, cg27320127, cg10240079, cg02536625, and cg23128025.

Preferably, said ensemble of genomic DNA sequences is comprised in the age indicator obtained by the method for determining an age indicator, wherein said method comprises applying a stepwise regression subsequently to the LASSO, and wherein each coefficient of said genomic DNA sequences comprised in said age indicator has an absolute value of more than 40.

Preferably, the genomic DNA sequences comprised in the ensemble of genomic DNA sequences disclosed herein, are the full sequences and not the fragments thereof.

The ensemble of genomic DNA sequences disclosed herein may comprise the complementary sequences thereof in addition and/or in place of said ensemble of genomic DNA sequences. Herein, a genomic DNA sequence refers to the sequence as described and/or the reverse complementary sequence thereof. The skilled person can easily judge if the sequence as described or the reverse complementary sequence thereof should be used. By default, and for most applications, the sequence as described is to be used, but for some applications, for example, for determining the methylation level of said sequence with a probe, the complementary sequence thereof is used for the probe.

The gene set disclosed herein may comprise at least one, preferably at least 5, preferably at least 10, preferably at least 20, preferably all of:
microtubule associated protein 4 (MAP4), protein kinase C zeta (PRKCZ), glutamate ionotropic receptor AMPA type subunit 4 (GRIA4), suppression of tumorigenicity 7 (ST7), protein kinase C eta (PRKCH), calcium voltage-gated channel subunit alpha1 D (CACNA1D), death associated protein (DAP), transient receptor potential cation channel subfamily V member 3 (TRPV3), furin, paired basic amino acid cleaving enzyme (FURIN), acetyl-CoA carboxylase alpha (ACACA), G protein subunit alpha i1 (GNAI1), solute carrier family 1 member 2 (SLC1A2), phosphodiesterase 4C (PDE4C), ELOVL fatty acid elongase 2 (ELOVL2), nuclear receptor corepressor 2 (NCOR2), endothelial PAS domain protein 1 (EPAS1), G protein subunit gamma 2 (GNG2), pantothenate kinase 1 (PANK1), ubiquitin specific peptidase 4 (USP4), G protein subunit alpha q (GNAQ), potassium voltage-gated channel modifier subfamily S member 1 (KCNS 1), DNA polymerase gamma 2, accessory subunit (POLG2), NADH:ubiquinone oxidoreductase subunit A10 (NDUFA10), relaxin family peptide receptor 3 (RXFP3), isoprenoid synthase domain containing (ISPD), inositol polyphosphate-5-phosphatase A (INPPSA), sirtuin 7 (SIRT7), guanine monophosphate synthase (GMPS), SIN3-HDAC complex associated factor (SINHCAF), tudor domain containing 1 (TDRD1).

Preferably, said gene set is obtained from further filtering the gene set disclosed herein on genes which encode a protein whereof the level and/or activity can be determined with an available assay. In other words, said gene set is further enriched for candidate drug targets.

Generally speaking, the method for determining an age indicator according to the invention and the ensemble of genomic DNA sequences according to the invention are tightly linked and are based on a common inventive concept. Thus, the description and definition of the ensemble of genomic DNA sequences according to the invention herein can be used to further specify the age indicator and/or the reduced training data set of the invention, both of which comprise an ensemble of genomic DNA sequences. Furthermore, said age indicator and/or reduced training data set can be used to further specify the method for determining an age indicator and/or the method for determining the age of an individual. Similarly, the ensemble of genomic DNA sequences according to the invention, which may be comprised in the age indicator of the invention, is preferably obtained by the method for determining an age indicator according to the invention. Moreover, this also applies to the gene set disclosed herein which preferably is selected from the ensemble of genomic DNA sequences according to the invention.

Further disclosed is an age indicator obtained by the method for determining an age indicator according to the invention, and/or an ensemble of genomic DNA sequences comprised in said age indicator obtained by said method.

Also disclosed is an age indicator as described in the Examples herein.

As regards the use of the age indicator as described in the examples, the age indicator obtained by the method for determining an age indicator and/or the ensemble of genomic DNA sequences comprised therein, the same applies as is described herein for uses of the ensemble of genomic DNA sequences and/or the gene set disclosed herein, in particular in a method for diagnosing the health state and/or the fitness state of an individual and/or in an *in silico* and/or *in vitro* screening method for identifying a molecule which affects ageing.

Further disclosed herein for reference is a method for diagnosing the health state and/or the fitness state of an individual comprising a step of providing the ensemble of genomic DNA sequences according to the invention, or the gene set disclosed herein.

Preferably herein, the health state comprises the state of at least one ageing-related disease, at least one phenotype associated with at least one ageing-related disease, and/or cancer, wherein the state indicates the absence, presence, or stage of the disease or the phenotype associated with a disease. Thus, the health state, as used herein, is preferably related to ageing.

Herein, a phenotype associated with an ageing-related disease refers preferably to at least one symptom of an ageing-related disease. Furthermore, an ageing-related diseases or cancer or a phenotype associated therewith, usually progresses is certain stages. Thus, herein, an ageing-related diseases or cancer or a phenotype associated therewith, can be absent or present, or be in a certain stage.

In preferred embodiments, the ageing-related disease is Alzheimer's disease, Parkinson's disease, atherosclerosis, cardiovascular disease, cancer, arthritis, cataracts, osteoporosis, type 2 diabetes, hypertension, Age-Related Macular Degeneration and/or Benign Prostatic Hyperplasia.

Preferably herein, the fitness state comprises the blood pressure, body weight, level of immune cells, level of inflammation and/or the cognitive function of the individual.

Preferably herein, the health state and/or fitness state of an individual relates to his biological age. Moreover, the age of the individual which is determined according to the present invention describes said biological age and/or said health state and/or fitness state better than does the chronological age of said individual.

In particular, diagnosing the health state and/or fitness state of an individual is complementary to diagnosing one specific disease and/or health/fitness parameter. Primarily, diagnosing the health state and/or fitness state may provide a holistic or integrated perspective on the individual. For example, in case the diagnosis is rather negative, it may be indicated that the individual changes his life style and/or his environment. Moreover, diagnosing the health state and/or fitness state is particularly useful for evaluating if a certain medical treatment or change in the life style or environment of an individual has improved the overall health state and/or fitness state of the individual. It is obvious that the overall health state and/or fitness state of an individual, in particular when related to ageing, is a crucial factor for the well-being of said individual. In other words, instead of only assaying the state of individual diseases, the method for diagnosing the health state and/or the fitness state according to the invention may allow diagnosing how young or old the individual biologically is.

The method for diagnosing the health state and/or the fitness state of an individual may further comprise a step of determining the methylation levels of the genomic DNA sequences in a biological sample of said individual comprising said genomic DNA sequences.

As regards determining the methylation levels of the genomic DNA sequences and the biological sample, the same applies as has been described above in the context of the methods disclosed herein for determining an age indicator and/or the age of an individual.

The method for diagnosing the health state and/or the fitness state of an individual disclosed herein comprises the medical application and/or the non-medical application of said method.

Further disclosed for reference is an *in silico* and/or *in vitro* screening method for identifying a molecule which affects ageing comprising a step of providing the ensemble of genomic DNA sequences disclosed herein, or the gene set disclosed herein. Preferably, the molecule ameliorates, prevents and/or reverses at least one ageing-related disease, at least one phenotype associated with at least one ageing-related disease, and/or cancer when administered to an individual. Preferably, said screening method is an *in vitro* method.

As regards the ageing-related disease and the phenotype associated therewith, the same applies as has been described above in the context of the method for diagnosing the health state and/or the fitness state of an individual. Furthermore, the prevention of an ageing-related disease and/or the phenotype associated therewith, relates to the maintenance of its absence; the amelioration relates to the slowed down progression through the stages, the maintenance of a stage and/or the regression to an earlier stage; and the reversion relates to the regression to an earlier stage, preferably to the regression to the absence of the disease and/or the phenotype associated therewith.

Herein, cancer is a preferred ageing-related disease.

The screening method disclosed herein may further comprise a step of determining the DNA methylation level of at least one of the genomic DNA sequences comprised in the ensemble of genomic DNA sequences and/or at least one of the genes comprised in the gene set.

The identified molecule may increase and/or decrease the DNA methylation level of at least one of said genomic DNA sequences or genes in an individual when administered to said individual. Preferably, the DNA methylation levels are altered such that they are associated with a younger chronological age than before alteration.

Thus, the ensemble of genomic DNA sequences or the gene set disclosed herein can be used for screening molecules, i.e. drug candidates, which alter the methylation state of said sequences or genes in a way which is associated with a younger chronological age than before alteration. For example, when the methylation level of a genomic DNA sequence increases with chronological age, the drug should decrease the methylation level of said genomic DNA sequence. Similarly, when the methylation level of a genomic DNA sequence decreases with chronological age, the drug should increase the methylation level of said genomic DNA sequence.

The screening method disclosed herein, wherein the gene set disclosed herein is provided, may further comprise a step of determining the activity of at least one protein encoded by the gene set. Preferably, said gene set only comprises genes which encode a protein.

The identified molecules may inhibit and/or enhance the activity of at least one protein encoded by the gene set. Preferably, the protein activities are altered such that they are associated with a younger chronological age than before alteration. For example, when the protein activity of a protein encoded by a genomic DNA sequence increases with chronological age, the drug should decrease/inhibit the activity of said protein. Similarly, when the protein activity of a protein encoded by a genomic DNA sequence decreases with chronological age, the drug should increase/enhance the activity of said protein.

As used herein, the activity of a protein also encompasses the level of said protein, in particular of its active form.

In further preferred aspects, the invention relates to a chip comprising the ensemble of genomic DNA sequences according to the invention as spots, wherein each sequence is contained in a separate spot. Preferably, the chip is a microarray chip.

Furthermore, the present invention relates to a kit comprising the chip of the invention.

Disclosed for reference is further a kit comprising
(a) at least one unique primer pair, wherein of each primer pair one primer is a forward primer binding to the reverse strand and the other primer is a reverse primer binding to the forward strand of one the genomic DNA sequences comprised in the ensemble of genomic DNA sequences disclosed herein or one of the genes comprised in the gene set disclosed herein, and wherein the two nucleotides which are complementary to the 3' ends of the forward and reverse primers are more than 30 and less than 3000, preferably less than 1000 nucleotides apart;
(b) at least one probe which is complementary to one of the genomic DNA sequences comprised in the ensemble of genomic DNA sequences disclosed herein or one of the genes comprised in the gene set disclosed herein; and/or
(c) the chip disclosed herein.

Preferably, said primer pair is used for a polymerase chain reaction (PCR). Said primers may be DNA methylation specific or not. Preferably, said primers are used for a methylation specific PCR method. The DNA methylation levels may be determined by assaying the amplified PCR products, for example by sequencing or by comparing the quantity of the products obtained by different PCRs with primers binding to either methylated or unmethylated sequences. Preferably, said probe is used for a hybridization method, for example an *in-situ* hybridization method, or a microarray method.

The primer or probe may specifically bind to either methylated or unmethylated DNA, wherein unmethylated cytosines have been converted to uracils.

Herein, conversion of unmethylated cytosines to uracils is done preferably by bisulfite treatment.

In certain embodiments, the kit further comprises a container for biological material and/or material for a buccal swab.

In certain embodiments, the kit further comprises material for extracting, purifying and or amplifying genomic DNA from a biological sample, wherein the material is a spin column and/or an enzyme.

In certain embodiments, the kit further comprises hydrogen sulfite.

Further disclosed is the use of the chip of the invention and/or the kit of the invention for determining the DNA methylation levels of at least one of the genomic DNA sequences comprised in the ensemble of genomic DNA sequences according to the invention and/or one of the genes comprised in the gene set disclosed herein.

Further disclosed is the use of the chip of the invention and/or the kit of the invention for diagnosing the health state and/or the fitness state of an individual.

Disclosed for reference is also the use of the chip disclosed herein and/or the kit disclosed herein in an *in silica* and/or *in vitro* screening method for identifying a molecule which affects ageing.

As regards the diagnosing of the health state and/or the fitness state of an individual and the *in silico* and/or *in vitro* screening method for identifying a molecule which affects ageing, the same applies as has been described above in the context of a method for diagnosing the health state and/or the fitness state of an individual and the *in silico* and/or *in vitro* screening method for identifying a molecule which affects ageing.

Further disclosed herein is a data carrier comprising the age indicator of the invention, the ensemble of genomic DNA sequences according to the invention, and/or the gene set disclosed herein.

The kit and/or the data carrier disclosed herein may further comprises a questionnaire for the individual of whom the age is to be determined, wherein the questionnaire can be blank or comprise information about said individual.

Disclosed herein for reference is further a method of age determination of an individual based on the levels of methylation of genomic DNA sequences found in the individual, comprising the steps of preselecting from genomic DNA sequences having levels of methylation associable with an age of the individual a set of genomic DNA sequences; determining for a plurality of individuals levels of methylation for the preselected genomic DNA sequences; selecting from the preselected set an ensemble of genomic DNA sequences such that the number of genomic DNA sequences in the ensemble is smaller than the number of genomic DNA sequences in the preselected set, wherein the ages of the individuals can be calculated based on the levels of methylation of the sequences of the ensemble, and a statistical evaluation of the ages if calculated indicates an acceptable quality of the calculated ages; determining in a sample of biological material from the individual levels of the methylation of the sequences of the ensemble; calculating an age of the individual based on levels of the methylation of the sequences of the ensemble; determining a measure of the quality of the age calculated; judging whether or not the quality determined is acceptable or not; outputting the age of the individual calculated if the quality is judged to be acceptable; re-selecting genomic DNA sequences for the ensemble in view of the judgment; and, depending on the judgment, amending the group of individuals to include the individual; re-selecting an ensemble of genomic DNA sequences from the preselected subset based on determinations of the levels of the methylation of individuals of the amended group.

An ensemble of genomic DNA sequences may be initially used, selected from a number of genomic DNA sequences having levels of methylation associable with an age of the individual; typically, the number of genomic DNA sequences in the ensemble is smaller than the number from which they are selected; then, methylation levels are obtained for the genomic DNA sequences of the ensemble, and from these an age is determined. In the course of a series of age determinations, the composition of the ensemble and/or the way to determine an age based on the methylation levels obtained for the genomic DNA sequences of the ensemble is repeatedly altered based on additional information generated or gained during the series of determinations, in particular based on the methylation levels additionally determined. The determination of an age may be based on an evaluation of methylation levels of specific genomic DNA sequences (or CpG loci) from a plurality of individuals, wherein the plurality of individuals comprises the exact individual, for whom the age is to be determined, although that need not be the case.

Surprisingly, it has been found that in this manner, significant improvements over the prior art can be achieved.

Generally speaking, such an adaption of the ensemble and/or of the (best) way to determine an age based on the respective methylation levels obtained for the genomic DNA sequences of an ensemble currently considered may be altered with every further individual for whom methylation levels and preferably the chronological age are known. Sometimes, this might not be done for every individual, but only for some of these individuals.

The adaption could be effected only after the levels of methylation of genomic DNA sequences have been determined for a plurality of more than one additional individual such as 5, 8, 10, 20, 50 or 100 individuals. This would be particularly advantageous where the effort of statistical evaluation to either select certain genomic DNA sequences into an ensemble and/or to determine the best way of age determination based on the methylation levels of certain genomic DNA sequences is substantive.

Thus, it is not necessary to only reiterate the composition of the ensemble and/or the best way to determine an age based on the methylation levels in case an outlier is measured.

Rather, there is a possibility to judge that the quality according to a (statistical) measure is not acceptable simply because the (statistical) measure indicates that the size of a reference plurality of individuals is smaller than a certain number, for example smaller than the overall number of all individuals for whom methylation levels have been determined and/or for whom methylation levels for the selected genomic DNA sequences have been determined and the chronological age is also known.

It is possible to first reiterate the composition of the ensemble and/or the best way to determine an age based on the methylation levels obtained for the genomic DNA sequences prior to the determination of an age of the specific individual and/or to first calculate the age of the additional individuum and to then reiterate the ensemble and/or the best way later on.

Herein, the terms "individuum" and "individual" are used interchangeably.

If the composition of the ensemble and/or the best way to obtain an age based on the respective methylation levels is to be effected after outputting the age of the individual, the methylation levels can be stored together with additional information about the individuals such as their chronological age (if known), so that the stored information can be used later on in a statistical (re-)evaluation. Accordingly, it is possible to gather such methylation level information for a plurality of additional individuals prior to reiterating the ensemble and/or the best way.

As is obvious from the above, the disclosure basically may suggest to improve a determination of an unknown age based on a statistical evaluation of measurements that themselves will yield the unknown result to be determined. Surprisingly, this is not contradictory in itself as by including such information in a reference group, overall improvements of the reliability of the method can be achieved. Accordingly, it has been found that a self-learning approach can be easily implemented.

On average, the age determined by the method should, for a large group of individuals, correspond to the average of their chronological ages. Note that the age determined will be a biological age or at least be closer to a biological age, which may be different from the chronological age and will oftentimes only be useful as it varies vis-à-vis the chronological age, because then, it can be determined whether or not a particular individual is aging faster than average.

Therefore, any deviation of the age determined according to the best information available vis-à-vis the chronological age is important. The method can be concluded or re-worded to relate to a method of establishing an age difference between a biological and a chronological age known or to assess differences between biological ages obtained by difference measurements and/or methods.

It has been found that using the best information available for such a comparison will typically include the largest number available of individuals rather than a pre-defined, fixed number. Overall, the ages determined for one and the same specimen gained from an individual will be altered if the ensemble and/or the best way of determining in age based on the methylation levels obtained for the genomic sequences is changed.

Due to such changes, the overall precision and/or variation could be effected, but the invention provides for improvements of overall precision and/or variation due to this.

Note that where a specimen is stored in a manner preventing changes to the methylation level, it will be easy to detect changes in an age determined if the measurements are sufficiently free from noise and the changes due to re-iteration are sufficiently large. Accordingly, it has been found that frequently implementation of a self-learning approach can be easily detected.

In a general approach, it is not necessary to actively preselect from all 28 million genomic DNA sequences having levels of methylation known to be associable with an age or adverse health condition of an individual a set of genomic DNA sequences smaller than the known about 28 million sites. Rather, such active preselection should be considered to have been made already if only a limited number of the known sites are evaluated, e.g. due to a method chosen.

A preselection can be made by choosing a specific method of determination of methylation levels such as those provided for example by Illumina^{™} and/or by choosing DNA chips that have a limited collection of spots that each can be used for determination of only one or some but not all genomic DNA sequences found in the individual and having levels of methylation associable with an age. Hence, the decision to use a specific detection method is an implicit preselection.

Also, a preselection can be considered to have been made if only data derived in such a manner is evaluated, i.e. if data is evaluated from less than the full app. 28 million sites constitutes the basis of the ensemble and/or set of the preselected set.

Typically, the preselected set will be significantly smaller than 28 million different genomic DNA sequences. In particular, while commercially available methods allow determination of levels of methylation of 800,000 (800000) or more different genomic DNA sequences, it will be understood that methods using chips that allow determination of levels of methylation for only a very limited number of different genomic DNA sequences using a collection of specific sites or "spots" are significantly cheaper to use in determination of an age of an individual.

For example, in certain methods chips can be used that allow determination of levels of methylation of only one or a few thousand different genomic DNA sequences, preferably even less, in particular not more than 1000 CpG loci, preferably 500 different genomic DNA sequences or CpG loci, preferably less than 200 different genomic DNA sequences or CpG loci, preferably not more than 150 different genomic DNA sequences or CpG loci.

It is possible to only determine levels of methylation for those genomic DNA sequences that constitute part of the ensemble. In this case, during reiteration, the composition of the ensemble may either only be altered in that certain genomic DNA sequences that previously had been considered are disconsidered after the reiteration and/or in that the best way to determine an age based on the methylations levels obtained for the genomic sequences of the ensemble itself is altered, for example where regression coefficients obtained from a multivariate (linear) correlation of methylation levels obtained for the genomic DNA sequences of the ensemble with a chronological age of individuals are changed. Also, it would be possible to carry out a determination of levels of methylation of further sequences prior to re-iteration.

It is possible to include in each determination of levels of methylation of individuals (some) more genomic DNA sequences (or CpG loci) than those currently constituting part of an ensemble, for example about or at least 10 or about or at least 20 or about or at least 50 more sequences or CpG loci.

Still, the number of genomic DNA sequences having levels of methylation associable with an age of the individual and determined for each individual or some individuals without constituting part of a current ensemble will normally be rather small. For example, it is possible to determine the levels of methylation for genomic DNA sequences currently not constituting part of the ensemble for not more than 5 times the number of genomic DNA sequences currently constituting part of the ensemble. Accordingly, where the ensemble for example comprises 100 different genomic DNA sequences, the overall number of different genomic sequences will usually be less than 500. Typically, there are even smaller numbers of additional genomic DNA sequences or CpG loci.

The additional genomic DNA sequences for which levels of methylation associable with an age are determined, although the respective genomic DNA sequences do not constitute part of an ensemble of genomic DNA sequences from which the age is determined may be smaller than 400, preferably smaller than 300, in particular smaller than 100 and in particular less than 60, 50 or 40 CpG loci. In addition and/or as an alternative, the ratio of genomic DNA sequences, not constituting part of the ensemble over genomic DNA sequences in the ensemble is preferably smaller than 5, preferably smaller than 4, preferably smaller than 3, preferably smaller than 2. It is noted that the additional sequences that are currently not constituting part of an ensemble but are used to provide additional levels of methylation only in case these might be helpful in a re-iteration will typically be carefully selected as well. This can be done in the preselection.

For example, CpG loci might be selected to the set that have methylations levels correlating well with methylation levels of CpG loci that, while also selected into the ensemble, have a very low overall methylation or a high variance. Also, CpG loci could be included known to be indicative for specific adverse lifestyles even though such loci would not be predominant in a statistical multivariate analysis. Furthermore, CpG loci could be selected additionally that are relevant to subsets of an initial reference group.

As will be obvious from the above, the exact numbers of the entire set and/or the ensemble will depend on the availability of affordable measurement methods such as sufficiently cheap chips. Also, data processing costs may be prohibitive. It may be preferred to use a chip adapted to determine levels of methylation of genomic DNA sequences having no more than 1000, 500, 200 spots each adapted to be used in determination of methylation levels of a different CpG locus.

This chip may comprise at least one spot, preferably at least 10, in particular at least 20, 30, 40, 50 60, 70, 80, 90 or 100 and in particular all spots allowing determination of levels of methylation of one or more, in particular at least 20, 30, 40, 50 60, 70, 80, 90 or 100 and in particular all of the following genomic DNA sequences or CpG loci: cg11330075, cg25845463, cg22519947, cg21807065, cg09001642, cg18815943, cg06335143, cg01636910, cg10501210, cg03324695, cg19432688, cg22540792, cg11176990, cg00097800, cg09805798, cg03526652, cg09460489, cg18737844, cg07802350, cg10522765, cg12548216, cg00876345, cg15761531, cg05990274, cg05972734, cg03680898, cg16593468, cg19301963, cg12732998, cg02536625, cg24088134, cg24319133, cg03388189, cg05106770, cg08686931, cg25606723, cg07782620, cg16781885, cg14231565, cg18339380, cg25642673, cg10240079, cg19851481, cg17665505, cg13333913, cg07291317, cg12238343, cg08478427, cg07625177, cg03230469, cg13154327, cg16456442, cg26430984, cg16867657, cg24724428, cg08194377, cg10543136, cg12650870, cg00087368, cg17760405, cg21628619, cg01820962, cg16999154, cg22444338, cg00831672, cg08044253, cg08960065, cg07529089, cg11607603, cg08097417, cg07955995, cg03473532, cg06186727, cg04733826, cg20425444, cg07513002, cg14305139, cg13759931, cg14756158, cg08662753, cg13206721, cg04287203, cg18768299, cg05812299, cg04028695, cg07120630, cg17343879, cg07766948, cg08856941, cg16950671, cg01520297, cg27540719, cg24954665, cg05211227, cg06831571, cg19112204, cg12804730, cg08224787, cg13973351, cg21165089, cg05087008, cg05396610, cg23677767, cg21962791, cg04320377, cg16245716, cg21460868, cg09275691, cg19215678, cg08118942, cg16322747, cg12333719, cg23128025, cg27173374, cg02032962, cg18506897, cg05292016, cg16673857, cg04875128, cg22101188, cg07381960, cg06279276, cg22077936, cg08457029, cg20576243, cg09965557, cg03741619, cg04525002, cg15008041, cg16465695, cg16677512, cg12658720, cg27394136, cg14681176, cg07494888, cg14911690, cg06161948, cg15609017, cg10321869, cg15743533, cg19702785, cg16267121, cg13460409, cg19810954, cg06945504, cg06153788, and cg20088545.

In particular, each of said genomic DNA sequences or CpG loci is comprised in a separate spot of said chip. In other words, one spot of said chip is defined by one of said genomic DNA sequences or CpG loci. It will be obvious that it is useful that at least a plurality of those CpG loci are referred to when measuring the methylation level using a chip. In particular, at least 10, preferably at least 20, preferably at least 50, and particularly preferred, all of the above CG loci may constitute part of a set of preselected genomic DNA sequences having levels of methylation associable with an age of the individuum, so that an ensemble of genomic DNA sequences can be easily obtained comprising either all of the above-listed CG loci or, preferably, at least a number or fraction of the above-listed CG loci. Said chip may be used for determining the DNA methylation levels of a set of genomic DNA sequences, in particular the genomic DNA sequences comprised in the reduced training data set disclosed herein.

In some cases, the CpG Loci will additionally comprise cg27320127, the last CpG loci being known inter alia from WO2012/162139. The CpGs identified above are identified using Illumina^{™} methylation probe IDs.

Furthermore, the chip may comprise a low overall number of spots allowing determination of levels of methylation of the following genomic DNA sequences, in particular less than 1600 spots, in particular less than 800 spots, in particular less than 400 spots, preferably less than 200 spots.

It should be noted that when defining a set of genomic DNA sequences having levels of methylation associable with an age of the individuum, the set being different from the entirety of genomic DNA sequences of a human being having levels of methylation associable with an age of the individuum, some or all of the CpG loci assumed to be known in the art to have levels of methylation associable with an age of the individuum known in the art, for example those listed in the WO 2012/162139 A1, could be included. However, it is considered that at least 10, preferably 20, particularly preferred 50, 100 and in particular all of the above-listed CpG loci believed to be novel over those known in the art may constitute part of a preselected set of genomic DNA sequences having levels of methylation associable with an age of the individuum and in particular comprising no more than 5000, in particular no more than 2000, in particular no more than 1000, in particular no more than 250 genomic DNA sequences or CpG loci and/or constituting a fraction of at least 10% of the overall number of genomic DNA sequences in a preselected set, preferably at least 10% thereof, and particularly preferred at least 15%, 20%, 25%, 33%, 50%, 66%, 75%, 80%, 100%. So, in preferred cases the CpG loci listed and newly disclosed herein as being relevant may constitute a significant part of the ensemble.

It will be noted that the overall number of CG loci considered in a set from which the ensemble is to be selected, will be dependent on the number of different loci measurable easily and, in a cost-effective manner, according to a respective state of the art. For example, prices of DNA chips having oligonucleotides that bind in a measurement process to DNA fragments comprising the respective CpG loci vary strongly with a number of different sites, with the costs dropping significantly from chips having 1000 or more sites to chips having 500, 384, 192 or 96 different sites.

It is noted that the numbers of 96 or 384, while in no way restricted, refer to numbers frequently used in current laboratory procedures. It has already been stated that usually the step of preselecting can be considered to have been effected once it has been decided to use not all CpG loci known in the human being but only those easily accessible. Such a step of preselection could thus be made by a referring to the data set comprising only a correspondingly small number of methylation levels.

Also it is noted that, determining in a sample of biological material from an individual levels of the methylation of the sequence of the ensemble can be done by referring to measurements that already have been done on the sample. Accordingly, a determination of levels of the methylation of certain sequences could be effected by opening a corresponding data file. The same holds for selecting from the preselected set an ensemble of genomic DNA sequences in a specific manner. This selection should be considered made in case reference is being had to a data base comprising such an ensemble determined by a preceding analysis of reference data from individuals.

Regarding the calculation of an age, it is noted that most frequently, the number of genomic DNA sequences in the set and/or in the plurality is rather large, for example because they comprise more than 5, in particular more than 10, in particular at least 50 different genomic DNA sequences. Also, the number of individuals in the group is rather large as well, that is comprising preferably at least 10, preferably at least 50, in particular at least 100, in particular at least 200, or, preferably, at least 1000 individuals. Thus, usually, mathematical analysis, in particular statistical analysis is needed to determine the best way of calculating an age of an individual based on the levels of the methylation determined. It should be noted that a "best" way of such calculation may not be the absolute best way but can refer to some very good way. In other words, a way of determination may be stated to be a "best" way even though either the calculations are particularly simple and/or because a local extreme of statistical functions has been used instead of an absolute extreme.

As is obvious from the above, typically the calculation of an age of an individual based on levels of the methylation of the sequences of the ensemble may be done in a manner where values relating to the level of methylation such as percentages are used to calculate the age based in a manner using also regression coefficients from a multivariate regression, and in particular from a multivariate linear regression. Calculating a measure such as a statistical measure can be effected in different ways. For example, it can be determined whether or not the levels of the methylation of the sequences themselves should be considered reliable. Where levels are exceptionally low, it might not be advisable to use the respective sequences and/or levels of the methylation because, for example, an error in the determination might have occurred (e.g. due to noise in the methylation level measurements), so that the measurements should be disregarded or weighed with a lower weight, compared to other levels. Also, where the levels of methylation are particularly high or low, it might well be that an assumption made during initial calculations such as in a multivariate linear regression assuming a linear correlation between a level of methylation and age will not apply.

It should be noted that generally, while the assumption that the levels of methylation correlate linearly with an age of the individual are useful, this need not be the case where very high or very low levels of methylation are observed or where the individual is significantly chronologically younger or older than the average of the individuals in the reference group. It might be useful to determine a more linear correlation by grouping certain individuals before determining how ages of individuals can best be calculated based on the levels of methylation of sequences found in a reference ensemble. For example, it might be advisable to distinguish between male and female individuals, children, teenagers, young adults, middle-aged persons and senior citizens. Also, it might be useful to differentiate e.g. between smokers and non-smokers, between persons having specific, different nutrition habits such as frequently eating very fat or not, frequently eating fish vs. frequently eating red meat, frequently and/or regularly drinking alcohol or specific alcohols such as alcoholic beverages, such as beer or wine, people exercising regularly or not, people working in adverse environments exposed to pollutants or dangerous materials such as radioactive materials and/or certain chemicals.

Thus, calculating a statistical measure of the quality of the age calculated could take into account whether or not a known chronological age deviates from the calculated biological age significantly more than the entirety of deviations obtained for the reference set and/or more than a plurality of other individuals for whom the levels of the methylation of the sequences of the ensemble has been measured. A difference can be considered to be significantly larger, if the significance is at least 2σ, 3σ, 4σ, 5σ or 6σ.

Also, the statistical measure of quality could be estimated by determining whether or not the reference set of individuals is sufficiently large. This would not be the case for example where a regression is carried out having a Spearman correlation of less than 0.85, preferably less than 0.90, preferably less than 0.91 and preferably 0.92 with a mean average error (MAE) of more than 6 years, preferably more than 5 years, in particular more than 4 years. It will be understood that it is also possible to estimate a confidence interval for each separate age calculated and that calculating a statistical measure might include the determination of a confidence interval of an age calculated. However, calculating a statistical measure of the quality of the age calculated might also be done easier, for example by determining whether the underlying reference group is large enough. It can be determined that the quality is not high enough if the group is considered to be too small. This can be the case if either the number of individuals in the reference group overall is too low and/or if the number of individuals in the reference group is too low in view of the number of genomic DNA sequences or CpG loci respectively in the preselected set of genomic DNA sequences or in the selected ensemble.

It could also be decided that the quality of an age calculated will not be sufficiently high in case not all individuals for whom methylation levels have been determined or not all individuals having certain properties such as being smokers or being female and so forth have been referred to when determining a best way of estimating and calculating an age. In this case, the (statistical) measure would be the number of members in the reference group vis-à-vis the number of individuals for whom levels of methylation and, where applicable, additional information are available.

A calculation of such difference could be done by determining that new data has not been entered into the reference group even prior to calculation of an age of the individual. Then, it should be noted that while at least the age of the individual is outputted in case the quality thereof is judged to be acceptable, it is also possible to output the age even where the quality of the calculation is considered dubious or insufficient. For example, it might be useful to output the age of an individual nonetheless because this allows an operator to check whether any specific problem can be detected that easily explains why the quality of the calculated age is to be considered subpar. For example, it might be that the individual has been grouped wrong so that the age has been determined using an ensemble of genomic DNA sequences and regression coefficients obtained for a group of young male strong smokers while the person is an elderly, non-smoking woman. A plurality of ensembles of CpG loci may be defined based on the set and one of these ensembles may be selected based on specific information either derived from one or more specific methylation level of CpG loci analyzed and/or an additional information provided independently thereof. Some of the CpG loci in the preselected set can be chosen such that a specific ensemble can be selected. Such provision and/or selection of a specific ensemble from several ensembles should be considered inventive per se. Also, it would be possible to output the age calculated with an explanation to the individual and offer a refund as standard or guaranteed qualities have not been achieved.

Also, it would be possible to recalculate the age once a reiteration of the ensemble and/or the best way of obtaining an age based on the levels of methylation of the sequences has been obtained and only then output the age of the individual calculated in an amended manner.

Amending the group of individuals usually would be done by including the one or more individuals for whom individual levels of methylation have additionally been determined. However, it would also be possible to exchange individuals or amend the group of individuals by splitting the group and so forth. For example, a case might occur where an initial group of individuals has been rather small so that a differentiation between smokers and non-smokers, males and females, young and old, persons drinking alcohol or not was not advisable, feasible or reasonable. Then, after some time, a large number of measurements will have been carried out and, in some cases, the additional properties such as individuals being smokers or not will have been determined so that then the group can be amended by adding one or some individuals based on their property and by splitting the groups according to such properties.

It should be noted that the levels of methylation will change in a large number of living organisms in a manner relating to the age of the organism. However, usually, the method of age determination will be used to determine the age of mammals, in particular primates, in particular human beings. Still, at least a rough estimate of age might be useful, e.g. for other living beings where trading animals that are particularly expensive.

In a preferred embodiment, the individual is a human. Of course, this will then also hold for the individuals of the reference group. It has already been indicated above that the numerous steps listed above will require extensive calculations. Therefore, implementing these steps in an automated manner to be executed by a computer is vital. It should be noted that for cases where at least 20 different genomic DNA sequences are considered in the set or the ensemble and where at least 20, preferably 100 individuals form the reference group, calculations without computer implementation are expected to be particularly error-prone so that the results in their entirety must be considered completely useless and unreliable. Also such calculations would neither be affordable in view of costs of computation done by a human being nor acceptable by any individual having to wait for a result. Therefore, executing at least one and preferably all of the calculation and evaluation steps by computers are considered vital.

Regarding the way according to which the levels of methylation of genomic DNA sequences found in the individual are determined, reference is made to the following methods known per se in the art methylation sequencing/bisulfate sequencing, PCR- methods, in particular at least one of methylation specific PCR (MSP), real-time methylation specific PCR, quantitative methylation specific PCR (QMSP), COLD-PCR, PCR using a methylated DNA-specific binding protein, targeted multiplex PCR, real-time PCR and microarray-based PCR, high resolution melting analysis (HRM), methylation-sensitive single-nucleotide primer extension (MS-SnuPE), methylation-sensitive single-strand conformation analysis, methyl-sensitive cut counting (MSCC), base-specific cleavage/MALDI-TOF, e.g. Agena, combined bisulfate restriction analysis (COBRA), methylated DNA immunoprecipitation (MeDIP), micro array-based methods, bead array-based methods, pyrosequencing, direct sequencing without bisulfate treatment (nanopore technology).

It is anticipated by the inventors that, using upcoming technologies or technologies that are known but thus far have found little use or market acceptance, further ways of determination of methylation levels may become available. Therefore, the list of methods given is not exclusive. Also, it might be possible to use different methods of determining methylation levels for different CpGs. Also, it might be possible to use different methods of determining methylation levels for a preselection and for a selection.

Among those methods of detecting the levels of methylation in a manner usable for the present invention, the following are currently particularly preferred: methylation sequencing/bisulfate sequencing, methylation specific PCR (MSP), real-time methylation specific PCR, quantitative methylation specific PCR (QMSP), COLD-PCR, base-specific cleavage/MALDI-TOF, e.g. Agena, micro array-based methods, bead array-based methods, pyrosequencing.

The group of individuals for whom levels of methylation are initially determined may be sufficiently large to obtain calculated ages that remain sufficiently stable even if the self- learning still leads to significant process. In other words, while an initial training of the process by reiterating the ensemble selection and/or the best way of obtaining results should relate on at least 50 individuals, so as to have sufficiently stable values for initial reference, it usually is preferred to have larger numbers such as 100 or 200 individuals in a reference group before starting actual measurements. As has been indicated above, reiterating the composition of the ensemble and all the best way of calculating an age therefrom can be postponed after a sufficiently large number of additional individuals can be additionally considered or added to the reference group.

The number of genomic DNA sequences in the preselected set can be made rather small while still allowing to amend the ensemble in a useful manner.

The preselected set may on the one hand comprise at least 90 CpG loci, preferably at least 100 CpG loci, particularly preferred at least 140 CpG loci, in particular at least 150 CpG loci.

It should be noted that where a broad spectrum of individuals is to be examined, a larger number of CpG loci in the preselected set is advisable, whereas measuring methylation levels in clearly specified, well defined groups might rely on a smaller number of see CpG loci in the preselected set, sometimes even requiring 90 CpG loci or less. On the other hand, the preselected set shall not be excessive for a variety of reasons. First of all, determination of methylation levels of CpG loci is more costly and more complex if more CpG loci are to be examined with respect to the methylation level.

Accordingly, a method relying on a large number of CpG loci is costly and reducing the number of CpG loci in an ensemble or in a preselected set does reduce the cost significantly. Also, the data processing is significantly simplified if less CpG loci need to be considered. This holds both for a reiteration of the CpG loci in the ensemble and for the best way of processing methylation obtained for such loci. It is noted that generally, the calculation expands a particular of reiterating the ensemble or best way should be considered to grow in a highly non-linear manner with a number of genomic DNA sequences considered. Therefore, it is preferred from a data analysis perspective as well to reduce the number of CpG loci considered. However, even where only 350, 170, 150 or even 100 CpG loci are considered in a preselected set, the overall computational effort of a multivariate analysis such as a multilinear regression, a principal component analysis, a partial least square analysis and so forth to determine the most important CpG loci methylation levels without over-determining the system will at any rate not be processable without a computer implementation.

It is considered necessary to provide the methylation levels determined in an electronic, automated manner, e.g. by establishing an electronic record or file for the methylation levels that can be used when processing the data even where such data processing is not immediately done after determination of methylation levels; not using computer interfaces for data transmission between the final stage used for obtaining the methylation levels from the samples and the stage used for data analysis would introduce a source of errors that must be considered unacceptable.

Therefore, it should be noted that the method generally is a computer implemented method having computer implemented steps and that at least some steps must necessarily be executed using a computer.

The selected ensemble may have a number of CpG loci rather small, in particular comprising less than 150 CpG loci, in particular less than 110 CpG loci, in particular less than 100 CpG loci, and in particular less than 90 CpG loci. It has been found that such a relatively small number of CpG loci considered still allows to factor in a large number of different influences, for example from lifestyle, for example due to the food, folate and vitamine intake such as vitamin B12 intake, polyphenols, selenium intake, obesity and/or physical activity, tobacco smoke, alcohol consumption, environmental pollutants such as arsenic and air pollution, aromatic hydrocarbons and other organic pollutants, psychological stress, shift work and so forth. In this respect, reference is made to the paper "Alegria-Torres et al., Epigenomics, 2011 June; 3(3): 267-277". These authors have shown that lifestyle has a significant influence on epigenetics for a large number of factors and that DNA methylation is influenced by lifestyle.

On the other hand, while it is sufficient to have a rather small number of CpG loci considered in the ensemble, the ensemble should not be too small. Otherwise, there is a risk that the age or the deviation of the age determined vis-à-vis the chronological age is affected by measurement errors, an insufficient database in the reference group and so forth.

It should be noted that the numbers indicated above to be suitable for the ensemble are valid after one or more reiterations of the best way of data determination from methylation levels of the see CpG loci of the ensemble.

When reiterating the ensemble, the number of members in the ensemble after reiteration may be different from the number of members in the ensemble prior to reiteration.

However, by such re-iteration, the number of CpG loci in the ensemble may vary optionally, i.e. a mere replacement of one or more CpG loci and the ensemble against one or more other CpG loci in the ensemble is not forbidden.

As has been indicated above, usually, the best way to determine an age from the methylation levels of the CpG loci of the ensemble may rely on coefficients obtained by a multiple regression (preferable: multiple linear regression) of the methylation levels against known chronological ages of individuals in the group. Methylation levels may be used by considering values that vary between 0% for a minimum methylation of a given CpG locus and 100% of a given CpG locus, the later value being used when the methylation level corresponds to the maximum methylation possible for a given CpG locus. In other words, the methylation level of values may be centered and normalized. Of course, rather than using a percentage varying between 0% and 100%, a value between 0 and 1 could also be used. While other ranges of values could be used, using values between 0 and 1 or 0% and 100% is particularly intuitive when assessing results and so forth.

As has been stated above, the age of the individual calculated may be outputted prior to re-selection of the ensemble independent of the judgement of quality of the measurement.

Furthermore, a possibility exists, wherein if the age of the individual calculated is judged to not be acceptable; and an age is outputted only after a re-selection of the ensemble of genomic DNA sequences has been effected and after an age has been recalculated for the re-selected ensemble.

Regarding the statistical analysis of the methylation levels or the values relating to the methylation levels, in principle different methods could be used. However, it has been found to be suitable to effect the statistical analysis using at least one regression method, for example, as disclosed for reference, a principal component analysis searching for the main components responsible for the deviation of the calculated age, a least square regression, a partial least square regression, a LASSO/elastic net regression and/or an XPG Boost method for identification of relevant CpGs. Of note, as explained further above, LASSO and elastic net are different regression methods, at least because LASSO does not comprise a Ridge regression and/or in elastic net, the L1 regularization parameter is not 1.

Further disclosed is a kit that can be used when a method according to the invention is to be executed, that is a kit for use in such a method.

In particular, such a kit may comprise at least a container for biological material of an individual obtained and/or prepared in a manner allowing determination of age according to a method as disclosed herein; the kit also comprising an information carrier carrying information relating to the identification of the patient or individual; the kit further comprising either instructions to execute a method of the invention and/or instructions how to have such a method of the invention executed, e.g. by sending in the probe to a specific lab with a voucher, and/or to provide data for the production of a data carrier comprising age related data determined by a method according to the present invention and/or to provide a data carrier comprising age related data determined by a method according to the present invention.

As has been indicated above, while frequently the absolute age of an individual needs to be determined, for example because biological material with DNA from a perpetrator has been sampled at a crime scene in order to provide an estimate of the chronological age of the perpetrator, frequently, it will be preferred to compare the age determined to a known chronological age.

Also, it might be useful to assess a difference between a chronological age and a biological age in view of the methylation levels of specific CpG loci for which methylation levels have been determined. It should be noted that these specific CpG loci need not constitute part of the ensemble. For example, certain CpG loci might have a methylation level highly dependent on whether or not a person smokes and whether or not the smoker is a particularly strong smoker.

It might not be advisable to include such methylation levels in the ensemble when calculating standard biological age for an individual, but it might be useful to indicate to the individual that certain methylation levels are indicative for environmental or other stress of the individual.

For example, the biological age of an individual might be determined by using an ensemble of CpG loci that is particularly useful for non-smokers; this might be useful where the individual has indicated to be a non-smoker. However, a case might occur where a non-smoker has been forced to passively smoke over a long period, for example because of growing up with smoking parents. In that case, the methylation levels of specific CpG loci might have been subjected to substantive change vis-à-vis a true non-smoker so even if otherwise, a correct biological age is determined, it might be useful to indicate to the individual that certain CpG methylation levels indicative of smoking behavior indicate that the person has suffered strongly from (passive) smoking.

This shows that in certain cases the preselected set may include additional CpG loci that while not representative for a biological age in a large reference group might still be relevant for a specific individual.

It should be noted that given the association of aging behavior and methylation levels, it might be helpful to alter the behavior of the methylation levels. This might be done using appropriate means; inter alia, it is reasonable to assume that drugs might constitute part of such means. Accordingly, where the methylation level has changed for a certain CpG locus vis-à-vis a control group, and it has been found that this change relates to adverse influences, a drug might help to prevent the biochemical adverse effects causing the change of methylation level or to undo the changes.

Understanding this, a method of drug screening is also suggested wherein a number of molecules are screened with respect to effecting aging comprising the steps of determining for specific CpG loci whether a molecule of the large number of molecules screened has a positive effect on the methylation levels of the CpG loci. This can be done in particular by a determination effected at least in part in-silico.

Thus, it is possible in a method of age determination disclosed herein that after a first ensemble of genomic DNA sequences has been selected and ages are determined for a series of individuals, and wherein for at least some individuals of the series methylation levels of genomic DNA sequences additional to those in the ensemble are determined, the group of individuals is amended to include at least some individuals from the series and a determination is made as to whether the ensemble of genomic DNA sequences should be altered in view of methylation levels obtained for additional genomic DNA sequences that were determined for at least some individuals of the series.

Accordingly, the determination of a biological age is altered repeatedly during the course of measurements using more and more data obtained during in the series even if each single determination of the series yields an acceptable result that is a result that has a rather small and easily acceptable confidence interval. The reiteration that is repeatedly executed may, as has been indicated before, relate to only the amendment of regression parameters obtained from a statistical analysis and used in the calculation of an age of an individual, using the methylation levels obtained for the individual or may decide that the ensemble overall should be altered, i.e. additional DNA sequences should be added and/or DNA sequences currently considered should be disconsidered.

Even where the results per se are acceptable, it will be understood that the overall quality will improve. However, where the ensemble itself is to be changed by adding additional DNA sequences and where the number of the available genomic DNA sequences from which such a selection into the ensemble can be made is small, care should be taken to define the DNA sequences that form of pool or set from which the selection can be made in a manner so that adding additional sequences actually is helpful. Therefore, at least in some cases, it is considered useful to start with a very large number of genomic DNA sequences having methylation levels associable with age and to then reduce this large number of genomic DNA sequences to be considered so that the selection, particularly if done repeatedly and often during standard measurements such as every can 8th, 10th or 100th individual, or after having x% more individuals that could be added to a reference group such as x=10%, 20%, 25%, 33%. 50%, 66%, 75%, 100%. Thus, the set should be carefully selected and a multi selection step for determining a useful preselection oftentimes may be advisable.

For example, first, the methylation levels of genomic DNA sequences of a few hundred individuals could be measured for some 800,000 (800000) different genomic DNA sequences. From the data set obtained, a few thousand genomic DNA sequences could be selected, for example in view of a principal component analysis determining the main components of the data set of methylation levels obtained versus the actual ages of the patient. Then, for the selected few thousand genomic DNA sequences, additional measurements could be effected for several hundred or more, e.g. a few thousand individuals and from the data set thus generated, several hundred genomic DNA sequences could be selected, for example of 384 DNA sequences that will have methylation levels detectable by a DNA chip having 384 different or oligonucleotide spots.

Again, the reduction of the number of genomic DNA sequences from the few thousand to 384 genomic DNA sequences could be made in view of a further principal component analysis, in view of the values of the respective methylation levels, in view of several methylation levels of different genomic DNA sequences being highly correlated and so forth.

After the final selection is made and the set of genomic DNA sequences is sufficiently small to allow a cheap determination of all methylation levels, which could be the case for 384 different genomic DNA sequences or 96 genomic DNA sequences, from those remaining genomic DNA sequences, the ensemble could be determined, but the determination of methylation levels can be determined for all of the remaining methylation DNA sequences without excessive costs.

When deciding whether or not the ensemble or the best way of determining an age in view of methylation levels obtained should be altered, the decision may be made based on a set of individuals as large as possible. Therefore, it is possible to provide additional data other than the methylation levels of the ensemble for at least some individuals in addition to the individuals of a presently used reference group. Then, a decision as to whether or not the ensemble or the best way of determining an age should be altered is made (also) in view of the methylation levels obtained for the additional individuals.

It should be noted that usually, the information relating to the additional individuals is used in such a decision as to the best way of calculating the age or as to the selection of genomic DNA sequences into the ensemble or out of the ensemble by simply enlarging a given group of individuals. However, there may be certain cases where it is useful to simultaneously delete individuals from the reference group or to split the reference group into several groups, each group having individuals with specific properties. One reason to exclude individuals from the reference group previously used could be in that a large number of additional individuals is added to the reference group and by doing so, when analyzing the entire group of both previous and added individuals, previously present individuals might be found to have methylation levels that now constitute statistical outliers.

Furthermore, a case might occur where a preselection has been made using a first detection method for detecting methylation levels such as a detection method measuring the methylation levels of some 850000 CpG loci while the actual measurement is performed with a method that is capable of determining the methylation levels of only way less CpG loci, and the methylation levels for these CpG loci show a behavior different from the behavior of the methylation levels of the same CpGs in a cross comparison. Here, while it might be useful to initially rely on the initial measurements obtained by a first means, once a sufficiently large database for the exact second method actually used in providing the methylation levels of the ensemble is available, those data obtained with the more complex first method can be deleted. Other reasons why a deletion might be useful is if a sufficiently large number of individuals have finally been sampled that share a common property and the individual to be deleted from the reference group does not share this property. For example, it is possible to delete individuals that are obese from an initial reference group if after some time it is decided that the ensemble and best way of determining an age should be determined such that best results are obtained for perfectly trained athletes that are not obese.

While it is possible to amend the ensemble and/or the best way of age determination based on methylation levels only once data from a large number of individuals is available, it can additionally and/or alternatively be decided that a re-evaluation of the ensemble and/or the best way should be carried out in view of methylation levels for a specific individual if at least one of the following conditions have been met: some or all methylation levels detected in the genomic DNA sequences are considered to be too low, the predicted age of a single individual deviates too far from a known chronological age of the individual, the predicted ages of a number of individuals show a systematic deviation from the known chronological ages of a number of individuals, the predicted ages of a number of individuals are scattered around the known chronological ages of the individuals with a variance considered too large, the predicted ages of a number of individuals show a systematic deviation from the known chronological ages of the individuals, the number of individuals for whom an age has been determined based on a given ensemble has reached a predetermined number, a specified time has elapsed since a previous re-selection.

It is possible to decide that reiteration or re-evaluation of the ensemble and/or the best way is necessary immediately and/or it can be decided that such reiteration is postponed until data from a sufficiently large number of such individuals is available where the above-mentioned condition is met. Another reason to postpone reiteration would be that such reiteration is only carried out in specific intervals; basically, in all these cases, information relating to the individuals, in particular the methylation levels detected in the genomic DNA sequences and, preferably the chronological ages of the individuals where known are stored prior to reiteration; then, a reiteration using all stored information is effected.

Judgment of the quality of a determined age may be done in that a comparison is made with a known chronological age. In most cases, a confidence interval is known that can be taken as a measure of quality. A very broad confidence interval might indicate that the determined age is unreliable. Also, once a large group of individuals has been examined, it is likely that the age determined does not deviate too far from ages of other individuals previously determined. In other words, once a large reference group has been examined and a new individual has a determined biological age that shows an aging behavior way faster or way slower than other individuals aging fast or slow, for whom previously data have been analyzed, it is not unlikely that an error has occurred, in particular if no additional factors influencing aging are known. In such a case, it could be decided that while the age might be correct, the quality thereof cannot be assessed in satisfying manner. Nonetheless, in such a case, the age determined would be indicated to the individual because, even though it cannot be assured that the age determined is reliable, it might be advisable for the individual to act as if the age determined would be reliable. For example, where a particularly fast aging behavior previously not observed in a large group of individuals is observed, so that the quality of the high age relative to the actual chronological age cannot be assessed, it might be necessary for the individual to consult a medical doctor.

Further, a method of determination of an age indicator for an individual in a series of individuals based on levels of methylation of genomic DNA sequences is disclosed for reference, wherein an ensemble of genomic DNA sequences is selected and an age indicator for the individual is provided in a manner continuously improving a statistical evaluation of previous measurements to obtain a better model.

### Brief description of the Figures

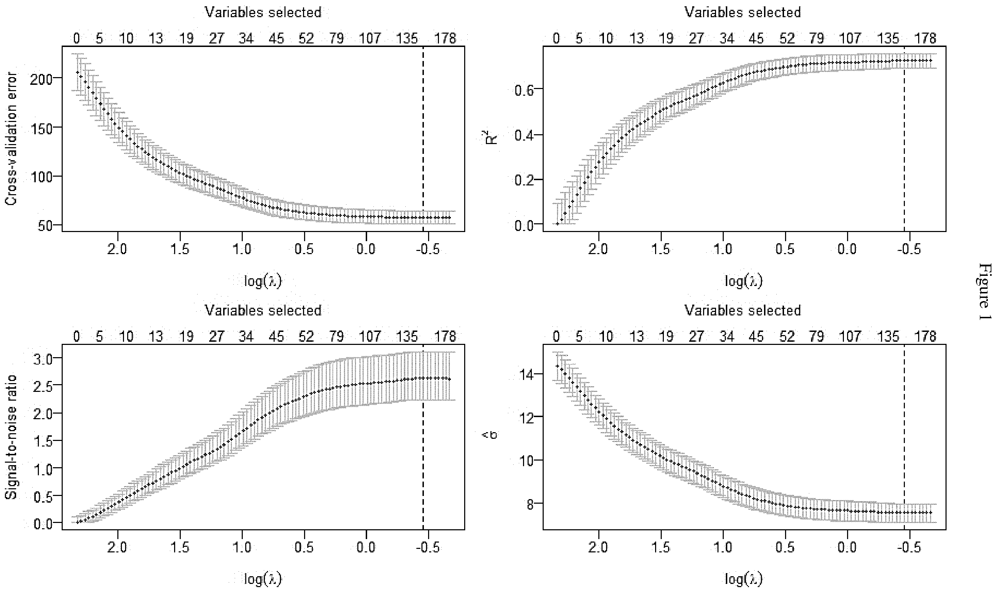

Figure 1. Performance of LASSO. A set of 148 cg sites was determined as optimal. Shown are four plots referring to Lasso regression and its performances. In all four plots a vertical dotted line represents the automatic threshold chosen for the number of variables selected. All plots report mean values plus range intervals produced by 20 cross validation runs. The different axes show different model metrics according to the biglasso package. The two upper plots report sums of cross-validated errors and coefficient of determination (R²), while the bottom two plots report two particular parameter from R implementation of LASSO regression: signal-to-noise ratio and <bs>. Details are in https://cran.rstudio.com/web/packages/biglasso/biglasso.pdf

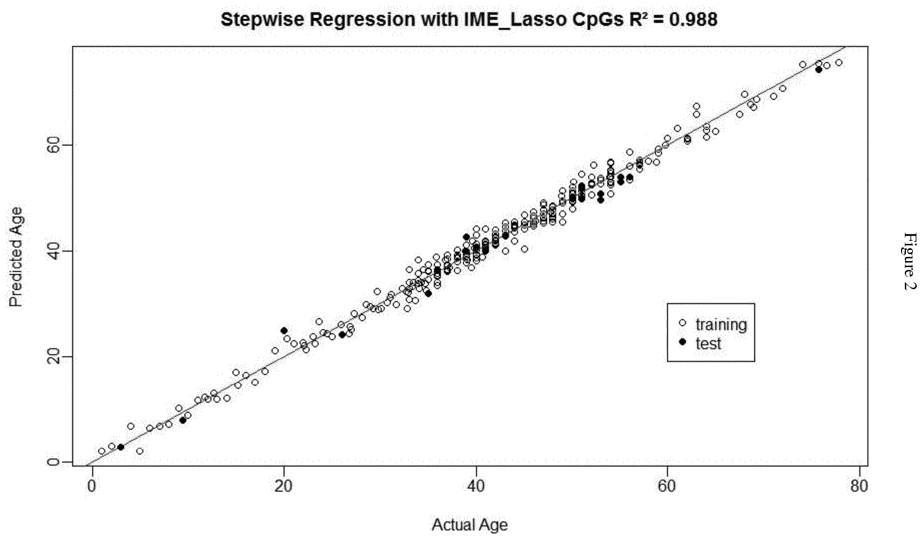

Figure 2, Performance of the age indicator obtained by LASSO and subsequent stepwise regression. Shown are the chronological age (actual age) and the determined age (predicted age) of 259 individuals of the training data set and 30 individuals of the test data set. No relevant or significant differences between training and test data set were observed. The shown coefficient of variation R² is based on the training and test data merged.

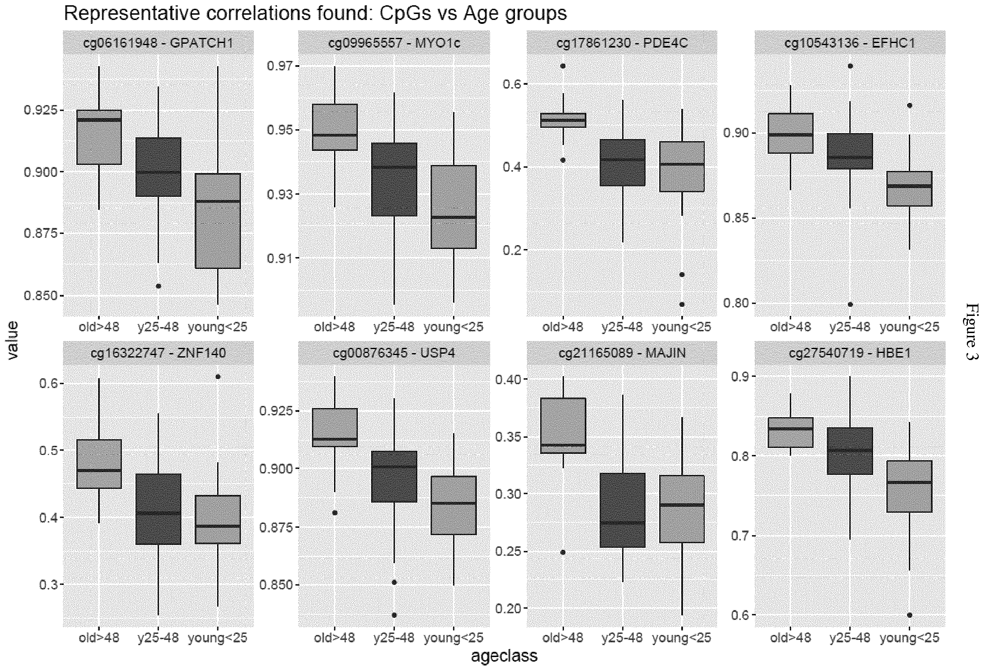

Figure 3, Correlations of representative CpG sites with the chronological age. Individuals of training and test data merged were grouped based on their chronological age (> 48 years, 25-48 years, and < 25 years; "old", "mid" and "young", respectively). The distributions of DNA methylation levels ("value") are shown for 8 representative CpG sites per age group. The genes comprised in the CpG sites are annotated.

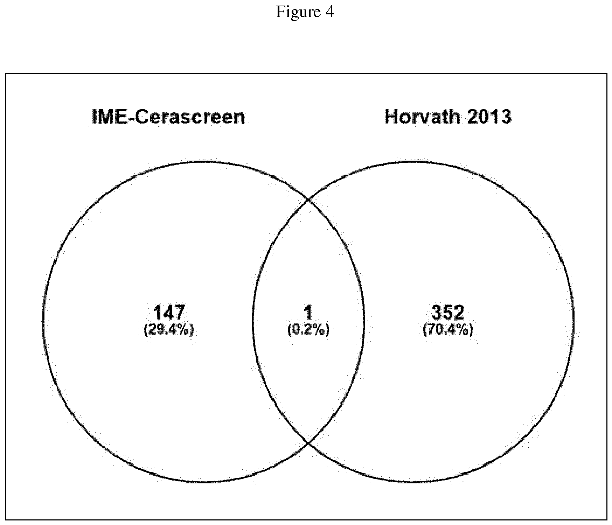

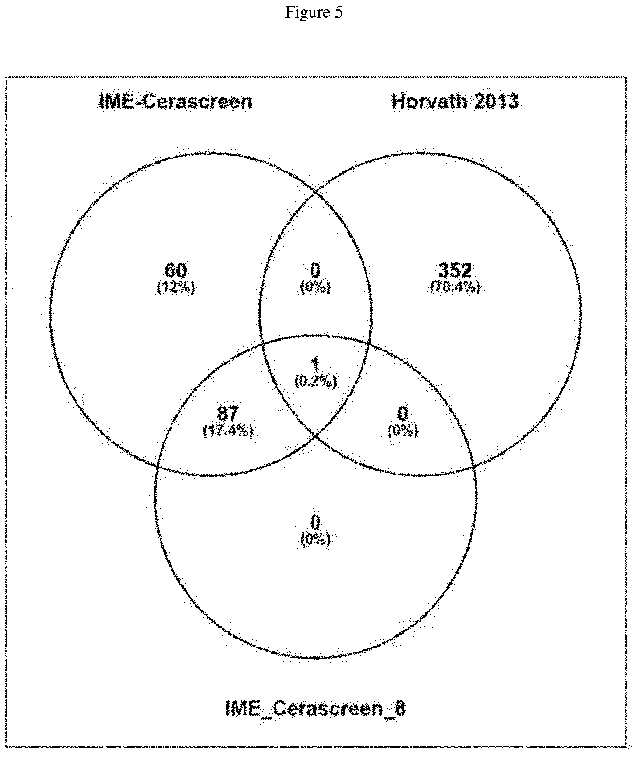

Figure 4, Overlap of CG sites with the set of CG sites as described by Horvath in Genome Biology 2013, 14:R115. The Venn diagram reports the amount of overlap between the set of 148 genomic DNA sequences (CpGs) determined herein by applying LASSO (IME-Cerascreen) and the 353 CpG List reported by Horvath in Genome Biology 2013, 14:R115. See also Figure 5.
Figure 5, Overlap of CG sites determined herein by applying LASSO (IME-Cerascreen) and subsequent stepwise regression (IME_Cerascreen_8). Also shown is the overlap with the set of CG sites as described by Horvath in Genome Biology 2013, 14:R115. See also Figure 4.

### Examples

### Example 1: Measuring CpG methylation levels of DNA from biological samples

For a very large number of app. 850.000 (850000) CpGs, the respective methylation levels have been measured in the following way:
Buccal cells were collected from a number of test persons with buccal swabs and genomic DNA was purified from the buccal cells using a QIAamp 96 DNA Swab BioRobot Kit (Qiagen, Hilden, Germany). The purified genomic DNA was treated with sodium bisulfite using the Zymo EZ DNA Methylation Kit (Zymo, Irvine, CA, USA). This treatment converts unmethylated cytosines to uracil, while methylated cytosines remain unchanged.

All further steps were performed with components from the Infinium MethylationEPIC Kit (Illumina^{™}, San Diego, CA, USA) according to the manufacturer's instructions. In short, bisulfite-treated samples were denatured and neutralized to prepare them for amplification. The amplified DNA was then isothermally amplified in an overnight step and enzymatically fragmented. Fragmented DNA was precipitated with isopropanol, collected by centrifugation at 4°C and resuspended in hybridization buffer. The fragmented, resuspended DNA samples were then dispensed onto an Infinium MethylationEPIC BeadChip (Illumina^{™}) and the BeadChip was incubated overnight in the Illumina^{™} Hybridization Oven to hybridize the samples onto the BeadChip by annealing the fragments to locus-specific 50mers that are covalently linked to the beads.

Unhybridized and nonspecifically hybridized DNA was washed away and the BeadChip was prepared for staining and extension in a capillary flow-through chamber. Single-base extension of the oligos on the BeadChip, using the captured DNA as a template, incorporates fluorescent labels on the BeadChip and thereby determines the methylation level of the query CpG sites. The BeadChip was scanned with the iScan System, using a laser to excite the fluorophore of the single-base extension product on the beads and recording high resolution images of the light emitted from the fluorophores. The data was analyzed using the GenomeStudio Methylation Module (Illumina^{™}), which allows the calculation of beta-values for each analyzed CpG.

With this procedure, the methylation levels of more than 850'000 (850000) different Illumina^{™} defined CpGs were measured per sample and person and a numerical value for each methylation level of the more than 850'000 (850000) different CpGs was provided. This was done for a large number of samples, each sample from a different individual. The numerical values have been normalized such that 0 corresponds to minimum methylation possible for a CpG and 1 corresponds to the maximum methylation for the CpG. Of note, 1 also corresponds to 100% or full methylation.

### Example 2: Measuring CpG methylation levels by base-specific cleavage/MALDI-TOF (Agena)

To determine methylation levels of a pre-selected set of several hundred different CpGs, the EpiTYPER DNA Methylation Analysis Kit from Agena Bioscience (San Diego, CA, USA) was used. In the example, 384 methylation levels of 384 different CpGs have been determined.

Again, Buccal cells were collected from a number of persons with buccal swabs and genomic DNA was purified from the buccal cells using a QIAamp 96 DNA Swab BioRobot Kit (Qiagen, Hilden, Germany). The purified genomic DNA was treated with sodium bisulfite using the Zymo EZ DNA Methylation Kit (Zymo, Irvine, CA, USA). This treatment converts unmethylated cytosines to uracil, while methylated cytosines remain unchanged.

Subsequently, the target regions containing the CpGs of interest were amplified by PCR using a specific primer pair per target region, each containing a T7-promoter-tagged reverse primer, respectively.

The PCR products were then treated with shrimp alkaline phosphatase to remove the unreacted nucleotides from the sample and in vitro transcribed using T7 RNA polymerase. The resulting RNA transcripts were specifically cleaved at uracil residues and dispensed onto a SpectroCHIP Array. This chip was placed into a MALDI-TOF mass spectrometer for data acquisition and the resulting data was analyzed with EpiTYPER software.

From the results, a numerical value for each methylation levels of the 384 different CpGs was provided. The numerical value was again normalized such that 0 corresponds to minimum methylation possible for a CpG and 1 (100%) corresponds to the maximum methylation for the CpG.

While methylation levels of 384 different genomic DNA sequences were determined by the method of Example 2, compared to the app. 850.000 (850000) different genomic DNA sequences, it is noted that the cost of an analysis according to Example 2 is significantly lower, amounting to less than 1/5 of the costs at the time of application.

### Example 3: Measuring CpG methylation levels by methylation specific PCR (msPCR)

To determine methylation levels of a pre-selected set of 192 different CpGs, real-time quantitative methylation specific PCR (msPCR) was performed in the following manner:
For each of the 192 CpG-containing target regions to be analyzed, a specific set of three oligonucleotides was designed, containing one forward primer and two reverse primers. The two reverse primers were designed such that one is having a G at the 3' end that is complementary to the methylated, unchanged C while the second forward primer is having an A at the 3' end that is complementary to the converted uracil.

Then, buccal cells were collected from a number of persons with buccal swabs and genomic DNA was purified from the buccal cells using a QIAamp 96 DNA Swab BioRobot Kit (Qiagen, Hilden, Germany). The purified genomic DNA was treated with sodium bisulfite using the Zymo EZ DNA Methylation Kit (Zymo, Irvine, CA, USA). This treatment converts unmethylated cytosines to uracil, while methylated cytosines remain unchanged.

To determine methylation levels of CpGs contained in the sample, for each set of three oligonucleotides two PCR reactions were initiated, the first PCR reaction using the forward and the first of the two reverse primers, the second PCR reaction using the forward and the second of the two reverse primers. The methylation level of each CpG was determined, using real-time quantitative msPCR with TaqMan probes specific for each amplified target region.

From the results, a numerical value for each methylation levels of the 192 different CpGs was provided. The numerical value was again normalized such that 0 corresponds to minimum methylation possible for a CpG and 1 (100%) corresponds to the maximum methylation for the CpG.

While the number of different genomic DNA sequences is lower than in the method of Example 2, the method is extremely competitive with respect to costs.

### Example 4: Generation of an age predictor using LASSO

DNA methylation levels of 289 individuals (259 for the training data set and 30 for the test data set) have been determined as described in Example 1 unless noted differently. In brief, the DNA methylation levels of 850000 different genomic DNA sequences have been determined from buccal swab samples using the Infinium MethylationEPIC BeadChip (Illumina^{™}). The methylation levels were normalized as beta values using program R v3.4.2, and thus could have a value between 0 and 1. The data set, i.e. the training data set, was a data matrix with a structure as in Table 1.

**Table 1**

| ID | Chronological age | CG1 | CG2 | ... | CG850000 |
|---|---|---|---|---|---|
| Individual 1 | 28 | 0.2 | 1.0 | ... | 0.1 |
| Individual 2 | 8 | ... | ... | ... | ... |
| ... | ... | ... | ... | ... | ... |
| Individual 259 | 65 | ... | ... | ... | ... |

Using the statistical software R v3.4.1 and the biglasso package, a LASSO regression was performed using the command
cvfit <- cv.biglasso(Vars800bm, Age, seed = 2401, nfolds = 20),
wherein Vars800bm is the training data set which relates to an exemplarily matrix as shown in Table 1, wherein the cg sites are the independent variables and the age is the dependent variable to be modeled; seed is a number used by random generator; and nfolds is the number of cross-validation repetition which the model has to be build with. The value 20 was used for cross-validation. The biglasso package was: "The biglasso Package: A Memory- and Computation-Effic Solver for LASSO Model Fitting with Big Data in R" by Yaohui Zeng and Patrick Breheny in arXiv:1701.05936v2 [statCO] 11 March 2018.

The formula of the obtained model (age indicator) upon LASSO regression was:Age = + 53.9126*cg27320127 + 43.1588*cg16267121 + 31.5464*cg00831672 + 30.4384*cg27173374 + 26.5197*cg16867657 + 20.9302*cg14681176 + 19.0975*cg25606723 + 16.8674*cg11607603 + 16.6092*cg08097417 + 15.0595*cg11330075 + 14.5786*cg12333719 + 14.1955*cg10543136 + 13.6743*cg21807065 + 12.4988*cg19851481 + 12.1954*cg08224787 + 11.7822*cg19702785 + 1 1.7706*cg1375993 1 + 11.6845*cg19112204 + 11.4521*cg07955995 + 10.869*cg18815943 + 10.829*cg24724428 + 10.7537*cg22101188 + 10.4571*cg19215678 + 9.551*cg22519947 + 9.5225*cg06161948 + 9.3932*cg16677512 + 9.2647*cg05396610 + 8.9059*cg21628619 + 8.7864*cg15609017 + 8.6846*cg24954665 + 8.5015*cg25642673 + 8.284*cg07802350 + 7.9408*cg05087008 + 7.8335*cg12548216 + 7.7144*cg09965557 + 7.6203*cg16999154 + 7.6057*cg12238343 + 7.5126*cg08044253 + 7.0673*cg16465695 + 6.939*cg13206721 + 6.6733*cg09001642 + 6.1215*cg11176990 + 6.0675*cg07625177 + 6.0657*cg05292016 + 5.9961*cg16593468 + 5.9511*cg07291317 + 5.5409*cg18506897 + 5.4739*cg07120630 + 5.2279*cg08662753 + 5.1938*cg24088134 + 5.1655*cg00097800 + 4.8623*cg16950671 + 4.6431*cg16245716 + 4.6364*cg06279276 + 4.6224*cg08686931 + 4.1089*cg27540719 + 4.0082*cg07529089 + 3.9294*cg06945504 + 3.8147*cg23677767 + 3.7304*cg07766948 + 3.7296*cg00876345 + 3.541*cg05972734 + 3.5305*cg22540792 + 3.4169*cg08118942 + 3.1845*cg02032962 + 3.1329*cg09460489 + 3.0723*cg22444338 + 3.0498*cg08856941 + 2.8317*cg03741619 + 2.7707*cg03230469 + 2.6979*cg06153788 + 2.6678*cg10522765 + 2.6533*cg14911690 + 2.5934*cg06186727 + 2.5488*cg03526652 + 2.5152*cg01520297 + 2.4409*cg09805798 + 2.3836*cg07513002 + 2.3539*cg08960065 + 2.3285*cg06335143 + 2.3044*cg16673857 + 2.2379*cg05990274 + 2.0254*cg04525002 + 1.9303*cg13154327 + 1.8016*cg07494888 + 1.7889*cg03388189 + 1.7543*cg08478427 + 1.7476*cg18768299 + 1.6312*cg21165089 + 1.6196*cg17665505 + 1.613*cg13460409 + 1.5347*cg14305139 + 1.4346*cg12804730 + 1.2032*cg04875128 + 1.2025*cg05211227 + 1.1767*cg18737844 + 1.1712*cg21460868 + 1.15*cg26430984 + 1.135*cg10321869 + 1.0067*cg14756158 + 1.0021*cg16322747 + 0.9948*cg17343879 + 0.9605*cg22077936 + 0.7994*cg18339380 + 0.5436*cg00087368 + 0.3003*cg05812299 + 0.281*cg12732998 + 0.0507*cg16456442 + 0.0277*cg17760405 + 0.0165*cg12658720 - 0.2038*cg08457029 - 0.4098*cg21962791 - 0.4232*cg15761531 - 0.4506*cg19810954 - 0.4626*cg20425444 - 0.5866*cg23128025 - 0.6731*cg25845463 - 0.6945*cg03324695 - 1.0445*cg01636910 - 1.4555*cg12650870 - 1.8012*cg01820962 - 2.2813*cg07782620 - 2.4468*cg04320377 - 2.6024*cg09275691 - 2.6286*cg15008041 - 2.7124*cg20576243 - 3.4046*cg13973351 - 3.5199*cg08194377 - 3.5713*cg07381960 - 4.0608*cg10240079 - 4.2758*cg14231565 - 4.8117*cg24319133 - 4.8449*cg03680898 - 5.694*cg19301963 - 6.83*cg03473532 - 7.515*cg13333913 - 8.0702*cg05106770 - 8.3397*cg04287203 - 9.4713*cg27394136 - 9.4931*cg10501210 - 10.8424*cg19432688 - 12.9786*cg02536625 - 13.2229*cg04028695 - 14.2271*cg16781885 - 14.728*cg15743533 - 14.9252*cg04733826 - 15.7917*cg20088545 - 16.5954*cg06831571 - 367.4866.

This age indicator comprised 148 terms such as + 16.6092*cg08097417, wherein a positive sign indicated that the methylation level positively correlated with age, and a negative sign indicated that the methylation level negatively correlated with age. A numbered cg refers to a genomic DNA sequence according to the Infinium MethylationEPIC BeadChip; and the absolute value of the coefficient with which the cg is multiplied indicates the importance of this cg. Various model performance checks confirmed that the selection of 148 cg sites was optimal (Figure 1).

This age indicator had the following performances: R² = 0.72, variable selected = 148 (non-zero coefficients), wherein R² is the coefficient of determination. The statistics have been determined with an independent test data set consisting of data of 30 individuals (about 10%) which were different from the 259 (289 -30) individuals used for the training data set but which were drawn from the same population as said 289 individuals.

Furthermore, LASSO has been applied on the data of 64 or 150 individuals from the 289 individuals (Table 2).

**Table 2**

| Size of training data set | Number of selected variables in age indicator | Performance of age indicator with test data set (R²) |
|---|---|---|
| 64 | 30 | 0.39 |
| 150 | 105 | 0.6 |
| 259 | 148 | 0.72 |

This suggested that the performance of the LASSO increased when data of further individuals were iteratively added to the data set and the age indicator was iteratively updated.

### Example 5: Generation of an age predictor using LASSO and subsequent stepwise regression

Stepwise regression was applied on a reduced training data set obtained after performing LASSO (Example 4) to distill the best significant set of cg sites/CpGs and thereby optimize the model. The reduced training data set (IME_blasso[,-1]) was the same as the training data set used in Example 4 except that it retained only the 148 columns relating to the 148 cg sites selected by LASSO.

Stepwise regression was performed using the statistical software R v3.4.1 and the following command:
model_blasso <- step(lm(Age ~ . , data = IME_blasso[,-1]), direction = "both"), wherein the direction for removing not significant variables was "both", meaning that both adding and removing variables was allowed.

The formula of the obtained model (age indicator) upon LASSO regression and subsequent stepwise regression was:Age = + 66.2822*cg11330075 + 65.203*cg00831672 + 55.7265*cg27320127 + 44.4116*cg27173374 + 38.3902*cg14681176 + 37.8069*cg06161948 + 36.6564*cg08224787 + 31.9397*cg05396610 + 30.1919*cg15609017 + 28.089*cg09805798 + 27.9392*cg19215678 + 27.8502*cg12333719 + 27.226*cg03741619 + 27.0323*cg16677512 + 25.9599*cg03230469 + 25.3932*cg19851481 + 24.5374*cg10543136 + 22.5525*cg07291317 + 21.8666*cg26430984 + 20.3621*cg16950671 + 20.3269*cg16867657 + 19.7973*cg22077936 + 18.7137*cg08044253 + 18.2047*cg12548216 + 18.1936*cg05211227 + 18.0812*cg13759931 + 17.6857*cg08686931 + 17.5303*cg07955995 + 16.1143*cg07529089 + 14.8703*cg01520297 + 14.6684*cg00087368 + 14.4397*cg05087008 + 14.4361*cg24724428 + 14.3055*cg19112204 + 14.2968*cg04525002 + 14.2302*cg08856941 + 13.3831*cg16465695 + 11.8127*cg08097417 + 11.7798*cg21628619 + 11.3523*cg09460489 + 11.2461*cg13460409 + 10.6268*cg25642673 + 10.4347*cg19702785 + 9.7844*cg18506897 + 9.5931*cg21165089 + 9.093*cg27540719 + 8.9361*cg21807065 + 8.8577*cg18815943 + 8.6138*cg23677767 + 7.1699*cg07802350 + 7.0528*cg11176990 + 6.5416*cg10321869 + 6.5049*cg17343879 + 5.8296*cg08662753 + 5.696*cg14911690 + 3.2983*cg12804730 + 3.1388*cg16322747 - 4.8653*cg14231565 - 5.5608*cg10501210 - 6.047*cg09275691 - 6.35*cg15008041 - 9.1942*cg05812299 - 9.3144*cg24319133 - 9.4566*cg12658720 - 9.8704*cg20576243 - 10.4082*cg03473532 - 10.6429*cg07381960 - 11.1592*cg05106770 - 12.0021*cg04320377 - 12.3296*cg19432688 - 12.9858*cg22519947 - 13.7116*cg06831571 - 13.8029*cg08194377 - 13.8668*cg01636910 - 14.6975*cg14305139 - 15.0408*cg04028695 - 16.3295*cg15743533 - 16.3314*cg03680898 - 18.6196*cg20088545 - 19.0952*cg13333913 - 19.3068*cg19301963 - 21.5752*cg13973351 - 23.0892*cg16781885 - 26.0415*cg04287203 - 32.3606*cg27394136 - 48.0918*cg10240079 - 50.0227*cg02536625 - 63.4434*cg23128025 - 519.3495.

The meaning of the terms and statistics is as explained in Example 4. Further details on the cg sequences and the coefficients can be found in Table 6.

Thus, the number of variables selected was further reduced upon applying the stepwise regression. In fact, the age indicator contained only 88 genomic DNA sequences (cg sites/CpGs).

Moreover, the performance of the age indicator obtained by LASSO and subsequent stepwise regression was:
R² = 0.9884 with the training data; and R² = 0.9929 (with the test data set containing the data of 30 test individuals as explained in Example 4). Thus, the performance was enhanced over the age indicator obtained by LASSO without stepwise regression.

The performance on the test data was as good as on the training data set which suggests that the age indicator has an outstanding performance (Figure 2). Moreover, such a high coefficient of determination value indicates a significant improvement over prior art age indicators.

By grouping individuals (training and test data sets merged) based on their chronological age, it could be confirmed that the methylation level of representative cg sites selected by the regression analysis correlated well with the age groups (Figure 3).

The age indicator and its determination was then compared to the age indicator of Horvath, Genome Biology 2013, 14:R115 in Table 3:

**Table 3**

| Characteristics | Horvath, Genome Biology 2013, 14:R115 | Present invention |
|---|---|---|
| Sample | Various cell types | Buccal swabs |
| Starting number of cg sites/ Illumina^{™} chip | 450000 | 850000 |
| Algorithm | Elastic net | LASSO + stepwise regression |
| No. of cg sites used in model | 353 | 88 |
| No. of cross-validation runs | Unknown | 20 |
| Coefficient of determination (R²) | 0.83 (buccal epithelium) 0.83 (saliva) | 0.996 |
| Median absolute deviation (years) | 0.8 (buccal epithelium) 2.7 (saliva) | 1.0 |
| p-value of coefficients | Unknown | p<0.05 |

This confirmed that the age indicator obtained by LASSO + stepwise regression performed as least as good as a relevant prior art age indicator, or even better, despite having only about 25% of the number of genomic DNA sequences (independent variables).

The small set of genomic DNA sequences comprised in the age indicator allows to use alternative, i.e. simpler, methods (see Examples 2 and 3) to determine the DNA methylation levels of individuals for whom the age is to be determined.

Moreover the set of cg sites determined by LASSO alone or with LASSO + subsequent stepwise regression had very little overlap with the cg sites determined in Horvath, Genome Biology 2013, 14:R115 (Figures 4 and 5).

### Example 6: Determination of gene sets from the sets of cg sites/CpGs

The list of cg sites determined by applying LASSO (Example 4) or LASSO + stepwise regression (Example 5) was filtered for those cg sites which were fully contained within a gene. In a first list (Table 4), 106 (partially redundant) coding sequences and non-coding sequences such as miRNAs or long non-coding RNAs were selected based on the 148 CpGs determined by LASSO:

**Table 4**

| Illumina ID | UCSC_RefGene_Accession | Name of first accession No. |
|---|---|---|
| cg00087368 | NM_005068 | SIM bHLH transcription factor 1 (SIM1) |
| cg12548216 | NM_030885; | microtubule associated protein 4 (MAP4) |
| | NM_001134364; | |
| | NM_002375 | |
| cg25845463 | NM_001033582; | protein kinase C zeta (PRKCZ) |
| | NM_002744; | |
| | NM_001033581 | |
| cg05087008 | NM_001077243; | glutamate ionotropic receptor AMPA type subunit 4 (GRIA4) |
| | NM_001112812; | |
| | NM_001077244; | |
| | NM_000829 | |
| cg05396610 | NR_046356; | glutamate ionotropic receptor AMPA type subunit 4 (GRIA4) |
| | NM_001077243; | |
| | NM_000829 | |
| cg01636910 | NM_003921 | BCL10, immune signaling adaptor (BCL10) |
| cg01820962 | NM_152729 | 5'-nucleotidase domain containing 1 (NTSDC1) |
| cg07529089 | NM_018412; | suppression of tumorigenicity 7 (ST7) |
| | NM_021908 | |
| cg02032962 | NM_006255 | protein kinase C eta (PRKCH) |
| cg03230469 | NM_000514 | glial cell derived neurotrophic factor (GDNF) |
| cg03473532 | NM_001145354 | muskelin 1 (MKLN1) |
| cg03526652 | NM_015189 | exocyst complex component 6B (EXOC6B) |
| cg03680898 | NM_000313 | protein S (PROS1) |
| cg05990274 | NM_000720; | calcium voltage-gated channel subunit alphal D (CACNA1D) |
| | NM_001128840; | |
| | NM_001128839 | |
| cg04320377 | NM_020782 | kelch like family member 42 (KLHL42) |
| cg04875128 | NM_130901 | OTU deubiquitinase 7A (OTUD7A) |
| cg17665505 | NM_004394 | death associated protein (DAP) |
| cg05211227 | NM_001195637 | coiled-coil domain containing 179 (CCDC179) |
| cg05292016 | NM_000793; | iodothyronine deiodinase 2 (DIO2) |
| | NR_038355 | |
| cg03741619 | NM_145068 | transient receptor potential cation channel subfamily V member 3 (TRPV3) |
| cg05812299 | NM_001190478 | MT-RNR2 like 5 (MTRNR2L5) |
| cg05972734 | NM_001164319; | filamin B (FLNB) |
| | NM_001164318; | |
| | NM_001457; | |
| | NM_001164317 | |
| cg07381960 | NM_002569; | furin, paired basic amino acid cleaving enzyme (FURIN) |
| | NM_001289823 | |
| cg06153788 | NR_104238; | solute carrier family 25 member 17 (SLC25A17) |
| | NR_104235; | |
| | NR_104237; | |
| | NR_104236; | |
| | NM_006358; | |
| | NM_001282727; | |
| | NM_001282726 | |
| cg06161948 | NM_018025 | G-patch domain containing 1 (GPATCH1) |
| cg06279276 | NM_033309 | UDP-GlcNAc:betaGal beta-1,3-N-acetylglucosaminyltransferase 9 (B3GNT9) |
| cg06335143 | NM_001004339 | zyg-11 family member A, cell cycle regulator (ZYG11A) |
| cg06945504 | NM_001184776; | seizure related 6 homolog like (SEZ6L) |
| | NM_001184775; | |
| | NM_001184774; | |
| | NM_001184777; | |
| | NM_001184773; | |
| | NM_021115 | |
| cg07291317 | NM_012334 | myosin X (MYO10) |
| cg16677512 | NM_198838; | acetyl-CoA carboxylase alpha (ACACA) |
| | NM_198837; | |
| | NM_198839; | |
| | NM_198836; | |
| | NM_198834 | |
| cg08044253 | NM_002069; | G protein subunit alpha i1 (GNAI1) |
| | NM_001256414 | |
| cg07766948 | NM_024040 | CUE domain containing 2 (CUEDC2) |
| cg07802350 | NM_000523 | homeobox D13 (HOXD13) |
| cg07955995 | NM_138693 | Kruppel like factor 14 (KLF14) |
| cg19112204 | NM_004171 | solute carrier family 1 member 2 (SLC1A2) |
| cg08097417 | NM_138693 | Kruppel like factor 14 (KLF14) |
| cg08118942 | NM_023928 | acetoacetyl-CoA synthetase (AACS) |
| cg08194377 | NM 015245 | ankyrin repeat and sterile alpha motif domain containing 1A (ANKS1A) |
| cg08478427 | NR_106988; | microRNA 7641-2 (MIR7641-2) |
| | NM_001145522; | |
| | NM_001145521; | |
| | NM_001145520; | |
| | NM_015577; | |
| | NM_001145523; | |
| | NM_001145525 | |
| cg08662753 | NM_001278074; | collagen type V alpha 1 chain (COLSA1) |
| | NM_000093 | |
| cg08856941 | NM_020682 | arsenite methyltransferase (AS3MT) |
| cg08960065 | NM_206885; | solute carrier family 26 member 5 (SLC26A5) |
| | NM_206884; | |
| | NM_206883; | |
| | NR_120443; | |
| | NR_120442; | |
| | NR_120441; | |
| | NM_001167962; | |
| | NM_198999 | |
| cg09275691 | NM_020401; | nucleoporin 107 (NUP107), |
| | NR_038930 | |
| cg09805798 | NR_110265; | long intergenic non-protein coding RNA 1797 (LINC01797) |
| | NR_110264 | |
| cg09965557 | NM_001080779; | myosin IC (MYO1C) |
| | NM_033375; | |
| | NM_001080950 | |
| cg10240079 | NM_181726 | ankyrin repeat domain 37 (ANKRD37) |
| cg17861230 | NM_000923 | phosphodiesterase 4C (PDE4C) |
| cg10543136 | NM_018100 | EF-hand domain containing 1 (EFHC1) |
| cg11176990 | NR_028386; | uncharacterized LOC375196 (LOC375196) |
| | NM_001145451 | |
| cg16867657 | NM_017770 | ELOVL fatty acid elongase 2 (ELOVL2) |
| cg12333719 | NM_006646; | WAS protein family member 3 (WASF3) |
| | NM001291965 | |
| cg24724428 | NM_017770 | ELOVL fatty acid elongase 2 (ELOVL2) |
| cg12658720 | NM_203425 | chromosome 17 open reading frame 82 (C17orf82) |
| cg13206721 | NM_020752; | G protein-coupled receptor 158 (GPR158) |
| | NR_027333 | |
| cg13333913 | NM_012304; | F-box and leucine rich repeat protein 7 (FBXL7) |
| | NM_001278317 | |
| cg13460409 | NM_018962 | ripply transcriptional repressor 3 (RIPPLY3) |
| cg13973351 | NM_017966 | VPS37C subunit of ESCRT-I (VPS37C) |
| cg14231565 | NM_001034845 | polypeptide N-acetylgalactosaminyltransferase like 6 (GALNTL6) |
| cg14305139 | NM_014957 | DENN domain containing 3 (DENND3) |
| cg19215678 | NM_006312; | nuclear receptor corepressor 2 (NCOR2) |
| | NM_001077261 | |
| cg00097800 | NM_001430 | endothelial PAS domain protein 1 (EPAS1) |
| cg14911690 | NM_025245 | PBX homeobox 4 (PBX4) |
| cg15609017 | NR_040046 | long intergenic non-protein coding RNA 1531 (LINC01531) |
| cg15743533 | NM_207121; | family with sequence similarity 110 member A (FAM110A) |
| | NM_001042353 | |
| cg15761531 | NM_001010983; | glycosyltransferase 8 domain containing 1 (GLT8D1) |
| | NM_152932; | |
| | NM_018446; | |
| | NM_152932 | |
| cg27173374 | NM_053064 | G protein subunit gamma 2 (GNG2), transcript variant 1 |
| cg16267121 | NM_001190472; | MT-RNR2 like 3 (MTRNR2L3) |
| | NM_001015885; | |
| | NM_003610 | |
| cg16322747 | NM_003440; | zinc finger protein 140 (ZNF140) |
| | NM_001300777; | |
| | NM_001300778; | |
| | NM_001300776 | |
| cg16465695 | NM_014238 | kinase suppressor of ras 1 (KSR1) |
| cg16593468 | NR_028444; | protein disulfide isomerase family A member 5 (PDIA5) |
| | NM_006810 | |
| cg16673857 | NM_018418; | spermatogenesis associated 7 (SPATA7) |
| | NM_001040428 | |
| cg17343879 | NM_148977; | pantothenate kinase 1 (PANK1) |
| | NM_138316; | |
| | NM_148978; | |
| | NR_029524 | |
| cg16781885 | NM_001034845 | polypeptide N-acetylgalactosaminyltransferase like 6 (GALNTL6) |
| cg00876345 | NM_003363; | ubiquitin specific peptidase 4 (USP4) |
| | NM_199443 | |
| cg14756158 | NM_002072 | G protein subunit alpha q (GNAQ) |
| cg19702785 | NM_002251 | potassium voltage-gated channel modifier subfamily member 1 (KCSN1) |
| cg27394136 | NM_007215 | DNA polymerase gamma 2, accessory subunit (POLG2) |
| cg18339380 | NM_020225 | storkhead box 2 (STOX2) |
| cg18506897 | NR_073547; | neurexin 3 (NRXN3) |
| | NM_004796 | |
| cg18768299 | NM_014753 | BMS1, ribosome biogenesis factor (BMS1) |
| cg18815943 | NM_012186 | forkhead box E3 (FOXE3) |
| cg10522765 | NM_004544 | NADH:ubiquinone oxidoreductase subunit A10 (NDUFA10) |
| cg12238343 | NM_016568 | relaxin family peptide receptor 3 (RXFP3) |
| cg19301963 | NM_032638; | GATA binding protein 2 (GATA2) |
| | NR_125398 | |
| cg00831672 | NM_001101426; | isoprenoid synthase domain containing (ISPD) |
| | NM_001101417 | |
| cg19810954 | NM_015833; | adenosine deaminase, RNA specific B1 (ADARB1) |
| | NM_001160230; | |
| | NM_001112; | |
| | NR_027674; | |
| | NR_027672; | |
| | NM_015834; | |
| | NR_027673 | |
| cg20088545 | NM_058238 | Wnt family member 7B (WNT7B) |
| cg20425444 | NM_015310 | pleckstrin and Sec7 domain containing 3 (PSD3) |
| cg21165089 | NM_001300803; | membrane anchored junction protein (MAJIN) |
| | NM_001037225 | |
| cg21962791 | NM_024854 | pyridine nucleotide-disulphide oxidoreductase domain 1 (PYROXD1) |
| cg22101188 | NM_001252335; | cingulin like 1 (CGNL1), transcript variant 1 |
| | NM_032866 | |
| cg22444338 | NM_001134395; | chromosome 7 open reading frame 50 (C7orf50) |
| | NM_032350; | |
| | NM_001134396 | |
| cg22519947 | NM_024848 | MORN repeat containing 1 (MORN1) |
| cg22540792 | NR_024191; | atlastin GTPase 2 (ATL2), transcript variant 3 |
| | NM_001308076; | |
| | NM_001135673; | |
| | NM_022374 | |
| cg23128025 | NM_052950 | WD repeat and FYVE domain containing 2 (WDFY2) |
| cg23677767 | NM_001198675; | transmembrane protein 136 (TMEM136) |
| | NM_001198674; | |
| | NM_001198673; | |
| | NM_001198672; | |
| | NM_001198671; | |
| | NM_001198670; | |
| | NM_174926 | |
| cg01520297 | NM_005539 | inositol polyphosphate-5-phosphatase A (INPPSA) |
| cg25606723 | NM_015130 | TBC1 domain family member 9 (TBC1D9) |
| cg25642673 | NM_002199 | interferon regulatory factor 2 (IRF2) |
| cg14681176 | NM_016538 | sirtuin 7 (SIRT7) |
| cg26430984 | NM_173465 | collagen type XXIII alpha 1 chain (COL23A1) |
| cg02536625 | NM_003875; | guanine monophosphate synthase (GMPS) |
| | NM_003875 | |
| cg27320127 | NM_022055 | potassium two pore domain channel subfamily K member 12 (KCNK12) |
| cg16245716 | NM_021238; | SIN3-HDAC complex associated factor (SINHCAF) |
| | NM_001135811; | |
| | NM_001135812 | |
| cg27540719 | NM_005330 | hemoglobin subunit epsilon 1 (HBE1) |
| cg16950671 | NM_198795 | tudor domain containing 1 (TDRD1) |

In a reduced gene set (Table 5), druggable gene targets have been selected from Table 4. In particular, the genes have been selected if an *in vitro* assay for determining the activity or function of the encoded protein was known in the art.

**Table 5**

| UCSC_RefGene_Accession | Name of first accession No. |
|---|---|
| NM_030885; | microtubule associated protein 4 (MAP4) |
| NM_001134364; | |
| NM_002375 | |
| NM_001033582; | protein kinase C zeta (PRKCZ) |
| NM_002744; | |
| NM_001033581 | |
| NM_001077243; | glutamate ionotropic receptor AMPA type subunit 4 (GRIA4) |
| NM_001112812; | |
| NM_001077244; | |
| NM_000829 | |
| NR_046356; | glutamate ionotropic receptor AMPA type subunit 4 (GRIA4) |
| NM_001077243; | |
| NM_000829 | |
| NM_018412; | suppression of tumorigenicity 7 (ST7) |
| NM_021908 | |
| NM_006255 | protein kinase C eta (PRKCH) |
| NM_000720; | calcium voltage-gated channel subunit alpha1D (CACNA1D) |
| NM_001128840; | |
| NM_001128839 | |
| NM_004394 | death associated protein (DAP) |
| NM_145068 | transient receptor potential cation channel subfamily V member 3 (TRPV3) |
| NM_002569; | furin, paired basic amino acid cleaving enzyme (FURIN) |
| NM_001289823 | |
| NM_198838; | acetyl-CoA carboxylase alpha (ACACA) |
| NM_198837; | |
| NM_198839; | |
| NM_198836; | |
| NM_198834 | |
| NM_002069; | G protein subunit alpha i1 (GNAI1) |
| NM_001256414 | |
| NM_004171 | solute carrier family 1 member 2 (SLC1A2) |
| NM_000923 | phosphodiesterase 4C (PDE4C) |
| NM_017770 | ELOVL fatty acid elongase 2 (ELOVL2) |
| NM_017770 | ELOVL fatty acid elongase 2 (ELOVL2) |
| NM_006312; | nuclear receptor corepressor 2 (NCOR2) |
| NM_001077261 | |
| NM_001430 | endothelial PAS domain protein 1 (EPAS1) |
| NM_053064 | G protein subunit gamma 2 (GNG2) |
| NM_148977; | pantothenate kinase 1 (PANK1) |
| NM_138316; | |
| NM_148978; | |
| NR_029524 | |
| NM_003363; | ubiquitin specific peptidase 4 (USP4) |
| NM_199443 | |
| NM_002072 | G protein subunit alpha q (GNAQ) |
| NM_002251 | potassium voltage-gated channel modifier subfamily S member 1 (KCNS1) |
| NM_007215 | DNA polymerase gamma 2, accessory subunit (POLG2) |
| NM_004544 | NADH:ubiquinone oxidoreductase subunit A10 (NDUFA10) |
| NM_016568 | relaxin family peptide receptor 3 (RXFP3) |
| NM_001101426; | isoprenoid synthase domain containing (ISPD) |
| NM_001101417 | |
| NM_005539 | inositol polyphosphate-5-phosphatase A (INPPSA) |
| NM_016538 | sirtuin 7 (SIRT7) |
| NM_003875; | guanine monophosphate synthase (GMPS) |
| NM_003875 | |
| NM_021238; | SIN3-HDAC complex associated factor (SINHCAF) |
| NM_001135811; | |
| NM_001135812 | |
| NM_198795 | tudor domain containing 1 (TDRD1) |

Finally, a list with 68 (partially redundant) coding sequences and non-coding sequences such as miRNAs or long non-coding RNAs was selected from the 88 CpGs determined by LASSO + stepwise regression (Table 6). The table further shows the coefficients of the respective age indicator and their standard errors (see Example 5).

**Table 6**

| Coefficient | +/- Std. Error | ID | UCSC_Ref_Gene |
|---|---|---|---|
| 66.2822 | 9.8319 | cg11330075 | |
| 65.203 | 12.7828 | cg00831672 | ISPD |
| 55.7265 | 7.5377 | cg27320127 | KCNK12 |
| 44.4116 | 8.4185 | cg27173374 | GNG2 |
| 38.3902 | 11.4848 | cg14681176 | SIRT7 |
| 37.8069 | 7.8695 | cg06161948 | GPATCH1 |
| 36.6564 | 9.964 | cg08224787 | |
| 31.9397 | 8.4487 | cg05396610 | GRIA4 |
| 30.1919 | 9.7667 | cg15609017 | LINC01531 |
| 28.089 | 8.4046 | cg09805798 | LOC101927577 |
| 27.9392 | 6.4631 | cg19215678 | NCOR2 |
| 27.8502 | 6.5183 | cg12333719 | WASF3 |
| 27.226 | 11.4717 | cg03741619 | TRPV3 |
| 27.0323 | 8.3075 | cg16677512 | ACACA |
| 25.9599 | 6.5411 | cg03230469 | GDNF |
| 25.3932 | 7.5404 | cg19851481 | |
| 24.5374 | 9.2886 | cg10543136 | EFHC 1 |
| 22.5525 | 10.8777 | cg07291317 | MYO10 |
| 21.8666 | 13.0388 | cg26430984 | COL23A1 |
| 20.3621 | 4.083 | cg16950671 | TDRD1 |
| 20.3269 | 4.3239 | cg16867657 | ELOVL2 |
| 19.7973 | 11.6224 | cg22077936 | |
| 18.7137 | 3.9634 | cg08044253 | GNAI1 |
| 18.2047 | 6.1215 | cg12548216 | MAP4 |
| 18.1936 | 4.9361 | cg05211227 | CCDC179 |
| 18.0812 | 6.0906 | cg13759931 | |
| 17.6857 | 5.0036 | cg08686931 | |
| 17.5303 | 4.5192 | cg07955995 | KLF14 |
| 16.1143 | 6.2049 | cg07529089 | ST7 |
| 14.8703 | 8.1841 | cg01520297 | INPPSA |
| 14.6684 | 4.3239 | cg00087368 | SIM1 |
| 14.4397 | 9.0743 | cg05087008 | GRIA4 |
| 14.4361 | 3.4811 | cg24724428 | ELOVL2 |
| 14.3055 | 5.5169 | cg19112204 | SLC1A2 |
| 14.2968 | 4.1059 | cg04525002 | |
| 14.2302 | 9.571 | cg08856941 | AS3MT |
| 13.3831 | 8.8481 | cg16465695 | KSR1 |
| 11.8127 | 8.6353 | cg08097417 | KLF14 |
| 11.7798 | 7.2263 | cg21628619 | |
| 11.3523 | 5.5046 | cg09460489 | |
| 11.2461 | 3.2763 | cg13460409 | DSCR6 |
| 10.6268 | 4.8908 | cg25642673 | IRF2 |
| 10.4347 | 7.2693 | cg19702785 | KCNS1 |
| 9.7844 | 7.4354 | cg18506897 | NRXN3 |
| 9.5931 | 5.0988 | cg21165089 | C11orf85 |
| 9.093 | 3.9039 | cg27540719 | HBE1 |
| 8.9361 | 6.2141 | cg21807065 | |
| 8.8577 | 3.708 | cg18815943 | FOXE3 |
| 8.6138 | 2.8016 | cg23677767 | TMEM136 |
| 7.1699 | 3.726 | cg07802350 | HOXD13 |
| 7.0528 | 4.2489 | cg11176990 | LOC375196 |
| 6.5416 | 1.9413 | cg10321869 | |
| 6.5049 | 3.478 | cg17343879 | PANK1 |
| 5.8296 | 2.8652 | cg08662753 | COL5A1 |
| 5.696 | 3.7948 | cg14911690 | PBX4 |
| 3.2983 | 1.8057 | cg12804730 | |
| 3.1388 | 2.007 | cg16322747 | ZNF140 |
| -4.8653 | 3.4742 | cg14231565 | GALNTL6 |
| -5.5608 | 2.5813 | cg10501210 | |
| -6.047 | 2.4969 | cg09275691 | NUP107 |
| -6.35 | 3.4617 | cg15008041 | |
| -9.1942 | 6.2636 | cg05812299 | MTRNR2L5 |
| -9.3144 | 3.8416 | cg24319133 | |
| -9.4566 | 4.137 | cg12658720 | C17orf82 |
| -9.8704 | 3.0654 | cg20576243 | |
| -10.4082 | 3.2632 | cg03473532 | MKLN1 |
| -10.6429 | 7.4387 | cg07381960 | FURIN |
| -11.1592 | 3.2236 | cg05106770 | |
| -12.0021 | 4.6698 | cg04320377 | KLHL42 |
| -12.3296 | 2.7158 | cg19432688 | |
| -12.9858 | 10.2914 | cg22519947 | MORN1 |
| -13.7116 | 2.9505 | cg06831571 | |
| -13.8029 | 3.2707 | cg08194377 | ANKS1A |
| -13.8668 | 4.4903 | cg01636910 | BCL10 |
| -14.6975 | 11.6384 | cg14305139 | DENND3 |
| -15.0408 | 2.9644 | cg04028695 | |
| -16.3295 | 7.5252 | cg15743533 | FAM110A |
| -16.3314 | 5.0278 | cg03680898 | PROS1 |
| -18.6196 | 4.4565 | cg20088545 | WNT7B |
| -19.0952 | 3.3737 | cg13333913 | FBXL7 |
| -19.3068 | 7.0512 | cg19301963 | GATA2 |
| -21.5752 | 6.8028 | cg13973351 | VPS37C |
| -23.0892 | 4.2648 | cg16781885 | GALNTL6 |
| -26.0415 | 6.6199 | cg04287203 | NRP1 |
| -32.3606 | 8.9103 | cg27394136 | POLG2 |
| -48.0918 | 10.9191 | cg10240079 | ANKRD37 |
| -50.0227 | 10.3763 | cg02536625 | GMPS |
| -63.4434 | 21.7615 | cg23128025 | WDFY2 |

### Example 7: Iterative updating of the age indicator

The age indicator was automatically updated with cases (probands; individuals) based on the decision if the domain boundaries of the test data were outside the domain boundaries of the training set of age indicator. The domain boundaries were the minimum and maximum DNA methylation levels of each genomic DNA sequence comprised in the age indicator. The minimum and maximum DNA methylation levels were found in the original training data set which has been used for determining the age indicator. These values change any time if the values of further individuals come in and replace the original min and max values for each of the CpGs. Min values will consequently diminish (if min is not yet 0) and max values will increase (if not yet 1) per CpG. In doing so the domain boundaries of the age indicator will expand to optimal values and it will be increasingly improbable that the age indicator is further updated.

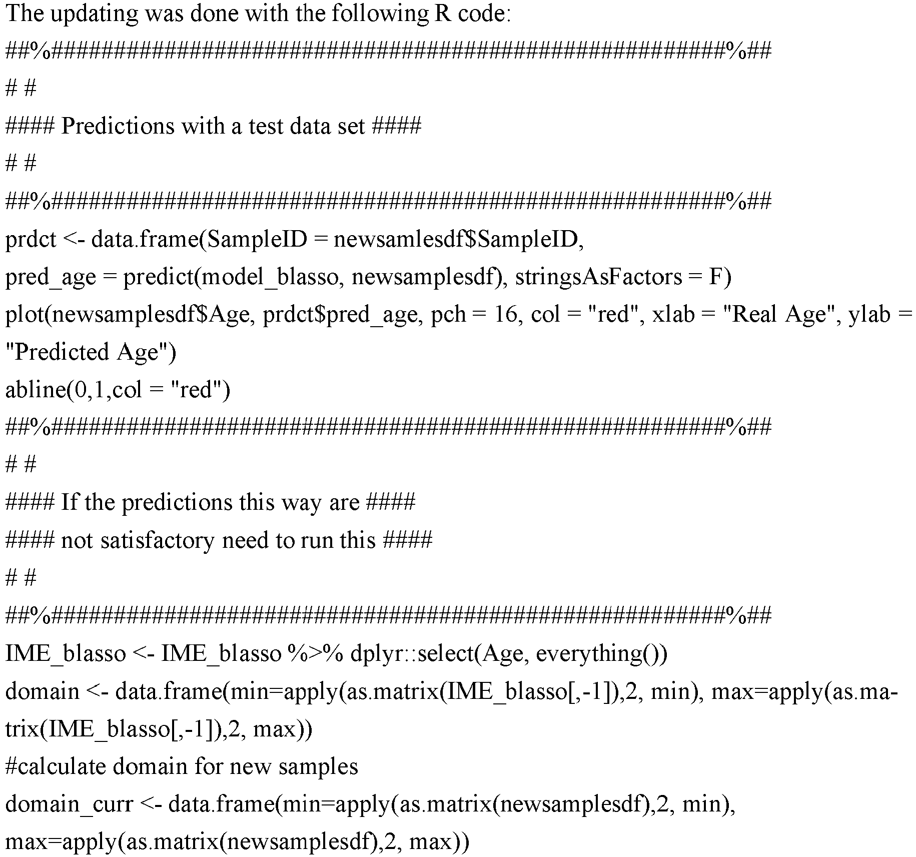

##%##########################################################%##
# #
#### operative check for prediction ####
##
# # ##%##########################################################%##
if(sum((domain$min-domain_curr$min)<0 & (domain$max-domain_curr$max)>0)){
nnew <- NROW(newsamplesdf)
nn <- NROW(IME_blasso)
# add new probands to the training set
newIME_blasso <- rbind(IME_blasso, newsamplesdf) # concatenate the two set
# rerun the model
model_blasso_new <- step(lm(Age ~ . , data = newIME_blasso), direction = "both")
sstep <- summary(model_blasso_new)
sstep
##check
par(mfrow=c(1,1))
plot(newIME_blasso$Age, model_blasso_new$fitted.values,
xlab = "Real Age [red points = new points]", ylab = "Predicted Age",
main = paste("Stepwise Regression with IME_newModel CpGs R² = ",
round(sstep$r.squared,3), sep = ""), pch = 1)
abline(0,1,col = "red")
errs <- newIME_blasso$Age - model_blasso_new$fitted.values
mae(errs)
postResample(newIME_blasso$Age,
as.vector(model_blasso_new$fitted.values))
points(newIME_blasso$Age[nn:(nn+nnew)],
as.vector(model_blasso_new$fitted.values[nn:(nn+nnew)]), col = "red",
pch = 16)
##
predictions <- data.frame(Age = newIME_blasso$Age[nn:(nn+nnew)],
PredAge = model_blasso_new$fitted.values[nn:(nn+nnew)])
write.csv(predictions, "predictions.csv")
save(model_blasso_confy_new, file = "model_blasso_new.lm")
#rm(newIME_blasso)
}else{
predicted <- predict.lm(model_blasso_confy, newsamplesdf)
plot(newsamplesdf$Age, predicted, pch=12, main="Predictions with IME_model")
abline(coef = c(model_blasso_new,1), col = "red")

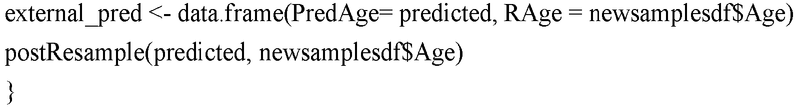

### Example 8: Further statistical Analyses of Data and Prediction of Age

DNA has been sampled from app. 200 individuals. These samples have all been obtained in northern Germany, but in order to have a broad database, care was taken to not exclude any individual in view of factors such as chronological age, general health state, obesity, level of physical fitness, drug consumption including drugs such as nicotine and alcohol. Therefore, the group is considered to be representative for the general population.

CpG methylation levels of the DNA from biological samples of app. 100 individuals have been determined using the method of Example 1, resulting in a large number of app. 850.000 (850000) CpGs for each individual.

In view of the amount of data and the computational expense of its analysis, the data was split into smaller arbitrary groups, and then, the data of these smaller groups was analyzed.

Using the data of a first group of 16 individuals, a principal component analysis has been effected and it was found that about 10 principal components account for almost all of the variance observed in the methylation levels of the CpGs in the groups samples, with the first two components already covering 98% of the variation, clearly indicating that despite the extremely large number of different CpG methylation levels considered, a reduction of the number is advised. Based on the principal component analysis and using regression techniques, a predictor model was established for each group that however basically showed that the model constructed was still suffering from insignificance of some of the coefficients.

It was also determined that even so, a number of the coefficients determined were found to have no statistical significance.

Given this, data from a first larger group of 98 individuals was analysed with the intention of establishing a model having a clearly reduced number of CpGs to be considered while maintaining a high statistical significance of all parameters. To this end, first a LASSO regression was executed; note that LASSO regression is a technique well known in the art and that software packages to implement Lasso regression are readily available. Note that it is possible to distinguish whether or not the methylation levels of a given CpG are of particular statistical relevance or not; this allows to consider only CpGs having some relevance. In particular, in this respect, reference is being made to "The biglasso Package: A Memory- and Computation-Effic Solver for LASSO Model Fitting with Big Data in R" by Yaohui Zeng and Patrick Breheny in arXiv:1701.05936v2 [statCO] 11 March 2018. Using a selection of only 50 different CpGs determined to constitute an optimal set by the LASSO regression, an attempt was made to further optimize the model derived. This was done using the XgBoost algorithm. Note that XgBoost is a well known open-source software library which provides a gradient boosting framework for a number of languages. Note that XgBoost serves to amend coefficients used in a statistical model. For further details with respect to the XgBoost algorithm and the implementation thereof, reference is made to "XGBoost: A Scalable Tree Boosting System", by T. Chen and C. Guestrin, arXiv: 1603.02754v3, 10. Juni 2016. The contents of the cited documents is enclosed herein in its entirety for purposes of disclosure.

It was found that a performant model could be obtained yielding good regression coefficients.

However, rather than contenting oneself with having achieved a high regression coefficient for the group considered, and maintaining the performant model as is, data from another 98 individuals were analyzed in the same manner as before. It was found that for the second group, about 78 CpGs should be considered in a model, with 8 of the 78 CpGs overlapping with the 50 CpGs selected for the first arbitrary group of 98 individuals.

Then, another run was made and it was determined that in a merged group, 70 CpG would constitute a useful selection of CpG from the initally considered app. 850000 different CpGs. From these 70 CpG, 10 were overlapping with only those of the first group, 12 were were overlapping with only those of the second group and 8 were overlapping with both groups.

The regression performed with XgBoost allowed to maintain the same high performance after 20 rounds of cross-validation.

This shows that by statistical means, in particular a LASSO regression, PCA or other means of distinguishing whether or not a specific CpG of a large number of CpG has statistical relevance, the number of CpGs can be significantly reduced from an overall extremely large set to a rather small set, allowing cheap detection using methods as referred to in Examples 2 and 3 above.

Then, relating only to the small set of CpGs, a useful model can be established that despite the small number of CpGs considered allows a determination of an age with high precision and a small confidence intervall, in particular by re-iterating parameters of a statistical model established.

In this manner, despite an overall small number of CpGs considered, determination of an age will be quite precise initially and will have a reliability increasing with time.

## Claims

1. A computer-implemented method for determining an age indicator comprising the steps of
(a) providing a training data set of a plurality of individuals comprising for each individual
(i) the DNA methylation levels of a set of genomic DNA sequences and
(ii) the chronological age, and
(b) applying on the training data set a regression method comprising
(i) a Least Absolute Shrinkage and Selection Operator (LASSO), thereby determining a reduced training data set, and
(ii) subsequent stepwise regression, thereby determining the age indicator, preferably wherein the stepwise regression is applied on said reduced training data set,
wherein the independent variables are the methylation levels of the genomic DNA sequences and preferably wherein the dependent variable is the age,
wherein the age indicator comprises
(i) a subset of the set of genomic DNA sequences as ensemble and
(ii) at least one coefficient per genomic DNA sequence contained in the ensemble, and
wherein the reduced training data set comprises all data of the training data set except the DNA methylation levels of the genomic DNA sequences which are eliminated by the LASSO.

2. A computer-implemented method for determining the age of an individual comprising the steps of
(a) providing a training data set of a plurality of individuals comprising for each individual
(i) the DNA methylation levels of a set of genomic DNA sequences and
(ii) the chronological age, and
(b) applying on the training data set a regression method comprising
(i) a Least Absolute Shrinkage and Selection Operator (LASSO), thereby determining a reduced training data set, and
(ii) subsequent stepwise regression, thereby determining the age indicator, preferably wherein the stepwise regression is applied on said reduced training data set,
wherein the independent variables are the methylation levels of the genomic DNA sequences and preferably wherein the dependent variable is the age,
wherein the age indicator comprises
(i) a subset of the set of genomic DNA sequences as ensemble and
(ii) at least one coefficient per genomic DNA sequence contained in the ensemble, and
wherein the reduced training data set comprises all data of the training data set except the DNA methylation levels of the genomic DNA sequences which are eliminated by the LASSO, and
(c) providing the DNA methylation levels of the individual for whom the age is to be determined of the genomic DNA sequences comprised in the age indicator, and
(d) determining the age of the individual based on its DNA methylation levels and the age indicator.

3. The method of claims 1 or 2, wherein the stepwise regression is a bidirectional elimination, wherein statistically insignificant independent variables are removed, preferably wherein the significance level is 0.05.

4. The method of any of claims 1 to 3, wherein the regression method does not comprise a Ridge regression (L2 regularization) or the L2 regularization parameter/lambda parameter is 0 and/or the LASSO L1 regularization parameter/alpha parameter is 1.

5. The method of any of claims 1 to 4, wherein the age indicator is iteratively updated comprising adding the data of at least one further individual to the training data in each iteration, thereby iteratively expanding the training data set.

6. The method of claim 5, wherein one updating round comprises applying the LASSO on the expanded training data set, thereby determining an updated reduced training data set, and applying the stepwise regression on the updated reduced training data set, thereby determining an updated age indicator.

7. The method of claims 5 or 6, wherein the quality of the age indicator is determined, wherein the determination of said quality comprises the steps of
(a) providing a test data set of a plurality of individuals who have not contributed data to the training data set comprising for each said individual
(i) the DNA methylation levels of the set of genomic DNA sequences comprised in the age indicator and
(ii) the chronological age; and
(b) determining the quality of the age indicator by statistical evaluation and/or evaluation of the domain boundaries,
wherein the statistical evaluation comprises
(i) determining the age of the individuals comprised in the test data set,
(ii) correlating the determined age and the chronological age of said individual(s) and determining a statistical parameter describing this correlation, and
(iii) judging if the statistical parameter indicates an acceptable quality of the age indicator or not, wherein the statistical parameter is a coefficient of determination (R²), and wherein an R² of greater than 0.90, preferably greater than 0.98, indicates an acceptable quality, and
wherein evaluation of the domain boundaries comprises
(iv) determining the domain boundaries of the age indicator,
wherein the domain boundaries are the minimum and maximum DNA methylation levels of each genomic DNA sequence comprised in the age indicator and
wherein said minimum and maximum DNA methylation levels are found in the training data set which has been used for determining the age indicator, and
(v) determining if the test data set exceeds the domain boundaries, wherein not exceeding the domain boundaries indicates an acceptable quality;
and wherein the age indicator is updated when its quality is not acceptable.

8. The method of any of claims 1 to 7, wherein the age indicator comprises at least 80 genomic DNA sequences, and/or wherein the age indicator comprises less than 300, preferably less than 150, preferably less than 110, preferably less than 100, preferably less than 90 genomic DNA sequences.

9. The method of any of claims 1 to 8, wherein the DNA methylation levels of the genomic DNA sequences of an individual are measured in a sample of biological material of said individual comprising said genomic DNA sequences, preferably wherein the sample comprises buccal cells.

10. The method for determining the age of an individual of any of claims 2 to 9, wherein the training data set further comprises at least one life-style factor associable with the individual(s) such as drug consumption, environmental pollutants, shift work and stress, and wherein the method further comprises a step of determining at least one life-style factor which is associated with the difference between the determined and the chronological age of said individual.

11. A computer-implemented method for determining the age of an individual comprising the steps of
(a) providing an age indicator comprising
(I) an ensemble of genomic DNA sequences comprising
at least 70 DNA sequences selected from the group consisting of: cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, and cg23128025; and
(II) at least one coefficient per genomic DNA sequence contained in the ensemble,
wherein said age indicator comprises less than 300 genomic DNA sequences, and preferably wherein said age indicator is obtained by the method for determining an age indicator according to any one of claims 1, or 3 to 9,
(b) providing the DNA methylation levels of the individual for whom the age is to be determined of the genomic DNA sequences comprised in the age indicator, and
(c) determining the age of the individual based on its DNA methylation levels and the age indicator.

12. The method for determining the age of an individual of any one of claims 2 to 11, wherein the determined age is an indicator of the biological age of the individual which relates to the health state and/or fitness state of the individual;
preferably wherein the health state relates to the state of at least one ageing-related disease such as Alzheimer's disease, Parkinson's disease, atherosclerosis, cardiovascular disease, cancer, arthritis, cataracts, osteoporosis, type 2 diabetes, hypertension, Age-Related Macular Degeneration and/or Benign Prostatic Hyperplasia, at least one phenotype associated with said ageing-related disease(s), and/or cancer; and
preferably wherein the fitness state comprises the blood pressure, body weight, level of immune cells, level of inflammation and/or the cognitive function of the individual.

13. Computer-implemented use of an age indicator for diagnosing the health state of an individual, said age indicator comprising
(I) an ensemble of genomic DNA sequences comprising at least 70 DNA sequences selected from the group consisting of: cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, and cg23128025; and
(II) at least one coefficient per genomic DNA sequence contained in the ensemble; and wherein said age indicator comprises less than 300 genomic DNA sequences; and preferably, wherein the health state relates to the state of at least one ageing-related disease such as Alzheimer's disease, Parkinson's disease, atherosclerosis, cardiovascular disease, cancer, arthritis, cataracts, osteoporosis, type 2 diabetes, hypertension, Age-Related Macular Degeneration and/or Benign Prostatic Hyperplasia, at least one phenotype associated with said ageing-related disease(s), and/or cancer.

14. A chip comprising an ensemble of genomic DNA sequences comprising at least 70 DNA sequences selected from the group consisting of: cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, and cg23128025, wherein the chip comprises less than 500 spots and each sequence is contained in a separate spot, and wherein the chip is suitable for detecting the DNA methylation levels of said ensemble of genomic DNA sequences.

15. A kit comprising (i) the chip of claim 14 and optionally (ii) a container for biological material, material for a buccal swab, a spin column and/or an enzyme for extracting, purifying and/or amplifying genomic DNA from a biological sample, and/or hydrogen sulfite.

## Patentansprüche

1. Computer-implementiertes Verfahren zum Bestimmen eines Altersindikators, umfassend die Schritte:
(a) Bereitstellen eines Trainingsdatensatzes einer Vielzahl von Individuen, umfassend für jedes Individuum
(i) die DNA-Methylierungsgrade eines Satzes genomischer DNA-Sequenzen und
(ii) das chronologische Alter, und
(b) Anwenden eines Regressionsverfahrens auf den Trainingsdatensatz, umfassend
(i) einen Least Absolute Shrinkage and Selection Operator (LASSO), wodurch ein reduzierter Trainingsdatensatz bestimmt wird, und
(ii) darauffolgende schrittweise Regression, wodurch der Altersindikator bestimmt wird, bevorzugt wobei die schrittweise Regression auf den reduzierten Trainingsdatensatz angewendet wird,
wobei die unabhängigen Variablen die Methylierungsgrade der genomischen DNA-Sequenzen sind und bevorzugt wobei die abhängige Variable das Alter ist,
wobei der Altersindikator umfasst:
(i) eine Teilmenge des Satzes der genomischen DNA-Sequenzen als Ensemble und
(ii) mindestens einen Koeffizienten pro im Ensemble enthaltener genomischer DNA-Sequenz, und
wobei der reduzierte Trainingsdatensatz alle Daten des Trainingsdatensatzes mit Ausnahme der DNA-Methylierungsgrade der genomischen DNA-Sequenzen umfasst, die durch LASSO eliminiert werden.

2. Computer-implementiertes Verfahren zum Bestimmen des Alters eines Individuums, umfassend die Schritte:
(a) Bereitstellen eines Trainingsdatensatzes einer Vielzahl von Individuen, umfassend für jedes Individuum
(i) die DNA-Methylierungsgrade eines Satzes genomischer DNA-Sequenzen und
(ii) das chronologische Alter, und
(b) Anwenden eines Regressionsverfahrens auf den Trainingsdatensatz, umfassend
(i) einen Least Absolute Shrinkage and Selection Operator (LASSO), wodurch ein reduzierter Trainingsdatensatz bestimmt wird, und
(ii) darauffolgende schrittweise Regression, wodurch der Altersindikator bestimmt wird, bevorzugt wobei die schrittweise Regression auf den reduzierten Trainingsdatensatz angewendet wird,
wobei die unabhängigen Variablen die Methylierungsgrade der genomischen DNA-Sequenzen sind und bevorzugt wobei die abhängige Variable das Alter ist,
wobei der Altersindikator umfasst:
(i) eine Teilmenge des Satzes der genomischen DNA-Sequenzen als Ensemble und
(ii) mindestens einen Koeffizienten pro im Ensemble enthaltener genomischer DNA-Sequenz, und
wobei der reduzierte Trainingsdatensatz alle Daten des Trainingsdatensatzes mit Ausnahme der DNA-Methylierungsgrade der genomischen DNA-Sequenzen umfasst, die durch LASSO eliminiert werden, und
(c) Bereitstellen der DNA-Methylierungsgrade des Individuums, dessen Alter bestimmt werden soll, der im Altersindikator umfassten genomischen DNA-Sequenzen, und
d) Bestimmen des Alters des Individuums auf der Grundlage seiner DNA-Methylierungsgrade und des Altersindikators.

3. Verfahren nach Anspruch 1 oder 2, wobei die schrittweise Regression eine bidirektionale Eliminierung ist, wobei statistisch unbedeutende unabhängige Variablen entfernt werden, bevorzugt wobei das Signifikanzniveau 0,05 ist.

4. Verfahren nach einem der Ansprüche 1 bis 3, wobei das Regressionsverfahren keine Ridge-Regression (L2-Regularisierung) umfasst oder der L2-Regularisierungsparameter/Lambda-Parameter 0 ist und/oder der LASSO-L1-Regularisierungsparameter/Alpha-Parameter 1 ist.

5. Verfahren nach einem der Ansprüche 1 bis 4, wobei der Altersindikator iterativ aktualisiert wird, umfassend das Hinzufügen der Daten mindestens eines weiteren Individuums zu den Trainingsdaten in jeder Iteration, wodurch der Trainingsdatensatz iterativ erweitert wird.

6. Verfahren nach Anspruch 5, wobei eine Aktualisierungsrunde das Anwenden des LASSO auf den erweiterten Trainingsdatensatz umfasst, wodurch ein aktualisierter reduzierter Trainingsdatensatz bestimmt wird, und das Anwenden der schrittweisen Regression auf den aktualisierten reduzierten Trainingsdatensatz, wodurch ein aktualisierter Altersindikator bestimmt wird.

7. Verfahren nach Anspruch 5 oder 6, wobei die Qualität des Altersindikators bestimmt wird, wobei die Bestimmung der Qualität die Schritte umfasst:
(a) Bereitstellen eines Testdatensatzes einer Vielzahl von Individuen, die keine Daten zum Trainingsdatensatz beigetragen haben, umfassend für jedes dieser Individuen
i) die DNA-Methylierungsgrade des im Altersindikator umfassten Satzes genomischer DNA-Sequenzen und
(ii) das chronologische Alter, und
(b) Bestimmen der Qualität des Altersindikators durch statistische Auswertung und/oder Auswertung der Domänengrenzen,
wobei die statistische Auswertung umfasst:
(i) Bestimmen des Alters der im Testdatensatz umfassten Individuen,
(ii) Korrelieren des bestimmten Alters und des chronologischen Alters des/der betreffenden Individuums/Individuen und Bestimmen eines statistischen Parameters, der diese Korrelation beschreibt, und
(iii) Beurteilen, ob der statistische Parameter auf eine akzeptable Qualität des Altersindikators hinweist oder nicht, wobei der statistische Parameter ein Determinationskoeffizient (R²) ist, und wobei ein R² größer als 0,90, bevorzugt größer als 0,98, auf eine akzeptable Qualität hinweist, und
wobei die Auswertung der Domänengrenzen umfasst:
(iv) Bestimmen der Domänengrenzen des Altersindikators,
wobei die Domänengrenzen die minimalen und maximalen DNA-Methylierungsgrade jeder im Altersindikator umfassten genomischen DNA-Sequenz sind, und
wobei die minimalen und maximalen DNA-Methylierungsgrade im Trainingsdatensatz gefunden werden, der zum Bestimmen des Altersindikators verwendet wurde, und
(v) Bestimmen, ob der Testdatensatz die Domänengrenzen überschreitet, wobei das Nichtüberschreiten der Domänengrenzen auf eine akzeptable Qualität hinweist,
und wobei der Altersindikator aktualisiert wird, wenn seine Qualität nicht akzeptabel ist.

8. Verfahren nach einem der Ansprüche 1 bis 7, wobei der Altersindikator mindestens 80 genomische DNA-Sequenzen umfasst, und/oder wobei der Altersindikator weniger als 300, bevorzugt weniger als 150, bevorzugt weniger als 110, bevorzugt weniger als 100, bevorzugt weniger als 90 genomische DNA-Sequenzen umfasst.

9. Verfahren nach einem der Ansprüche 1 bis 8, wobei die DNA-Methylierungsgrade der genomischen DNA-Sequenzen eines Individuums in einer Probe biologischen Materials des Individuums gemessen werden, das die genomischen DNA-Sequenzen umfasst, bevorzugt wobei die Probe bukkale Zellen umfasst.

10. Verfahren zum Bestimmen des Alters eines Individuums nach einem der Ansprüche 2 bis 9, wobei der Trainingsdatensatz des Weiteren mindestens einen Lifestyle-Faktor umfasst, der mit dem/den Individuum/Individuen assoziiert werden kann, wie beispielsweise Drogenkonsum, Umweltschadstoffe, Schichtarbeit und Stress, und wobei das Verfahren des Weiteren einen Schritt des Bestimmens mindestens eines Lifestyle-Faktors umfasst, der mit dem Unterschied zwischen dem bestimmten und dem chronologischen Alters des Individuums assoziiert ist.

11. Computer-implementiertes Verfahren zum Bestimmen des Alters eines Individuums, umfassend die Schritte:
(a) Bereitstellen eines Altersindikators, umfassend
(I) ein Ensemble aus genomischen DNA-Sequenzen, das mindestens 70 DNA-Sequenzen umfasst, die ausgewählt sind aus der Gruppe bestehend aus: cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, und cg23128025; und
(II) mindestens einen Koeffizienten pro im Ensemble enthaltener genomischer DNA-Sequenz,
wobei der Altersindikator weniger als 300 genomische DNA-Sequenzen umfasst, und bevorzugt wobei der Altersindikator durch das Verfahren zum Bestimmen eines Altersindikators nach einem der Ansprüche 1 oder 3 bis 9 erhalten wird,
(b) Bereitstellen der DNA-Methylierungsgrade des Individuums, dessen Alter bestimmt werden soll, der im Altersindikator umfassten genomischen DNA-Sequenzen, und
d) Bestimmen des Alters des Individuums auf der Grundlage seiner DNA-Methylierungsgrade und des Altersindikators.

12. Verfahren zum Bestimmen des Alters eines Individuums nach einem der Ansprüche 2 bis 11, wobei das bestimmte Alter ein Indikator des biologischen Alters des Individuums ist, das den Gesundheitszustand und/oder Fitnesszustand des Individuums betrifft;
bevorzugt wobei der Gesundheitszustand den Zustand mindestens einer altersbedingten Erkrankung wie Alzheimer-Krankheit, Parkinson-Krankheit, Arteriosklerose, Herz-Kreislauf-Krankheit, Krebs, Arthritis, Katarakt, Osteoporose, Typ-2-Diabetes, Bluthochdruck, altersbedingte Makuladegeneration und/oder benigne Prostatahyperplasie, mindestens einen Phänotyp, der mit dieser/diesen altersbedingter/altersbedingten Erkrankung(en) assoziiert ist, und/oder Krebs betrifft, und
bevorzugt wobei der Fitnesszustand den Blutdruck, das Körpergewicht, den Immunzellenspiegel, den Entzündungsgrad und/oder die kognitive Funktion des Individuums umfasst.

13. Computer-implementierte Verwendung eines Altersindikators zur Diagnose des Gesundheitszustands eines Individuums, wobei der Altersindikator umfasst:
(I) ein Ensemble aus genomischen DNA-Sequenzen, das mindestens 70 DNA-Sequenzen umfasst, die ausgewählt sind aus der Gruppe bestehend aus: cg11330075, cg00831672, cg27320127,
cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, und cg23128025; und
(II) mindestens einen Koeffizienten pro im Ensemble enthaltener genomischer DNA-Sequenz; und wobei der Altersindikator weniger als 300 genomische DNA-Sequenzen umfasst; und bevorzugt wobei der Gesundheitszustand den Zustand mindestens einer altersbedingten Erkrankung wie Alzheimer-Krankheit, Parkinson-Krankheit, Arteriosklerose, Herz-Kreislauf-Krankheit, Krebs, Arthritis, Katarakt, Osteoporose, Typ-2-Diabetes, Bluthochdruck, altersbedingte Makuladegeneration und/oder benigne Prostatahyperplasie, mindestens einen Phänotyp, der mit dieser/diesen altersbedingter/altersbedingten Erkrankung(en) assoziiert ist, und/oder Krebs betrifft.

14. Chip, der ein Ensemble genomischer DNA-Sequenzen umfasst, das mindestens 70 DNA-Sequenzen umfasst, die ausgewählt sind aus der Gruppe bestehend aus:
cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg10465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, und cg23128025, wobei der Chip weniger als 500 Spots umfasst und jede Sequenz in einem separaten Spot enthalten ist, und wobei der Chip zum Nachweisen der DNA-Methylierungsgrade des Ensembles aus genomischen DNA-Sequenzen geeignet ist.

15. Kit, der (i) den Chip nach Anspruch 14 und gegebenenfalls (ii) einen Behälter für biologisches Material, Material für einen Wangenabstrich, eine Spin-Säule und/oder ein Enzym zur Extrahierung, Aufreinigung und/oder Amplifizierung genomischer DNA aus einer biologischen Probe, und/oder Hydrogensulfit umfasst.

## Revendications

1. Procédé mis en oeuvre par ordinateur pour déterminer un indicateur d'âge comprenant les étapes suivantes :
(a) obtention d'un ensemble de données d'entraînement d'une pluralité d'individus comprenant, pour chaque individu,
(i) les niveaux de méthylation de l'ADN d'un ensemble de séquences d'ADN génomique et
(ii) l'âge chronologique, et
(b) application sur l'ensemble de données d'entraînement d'un procédé de régression comprenant
(i) un opérateur de sélection et réduction par moindres valeurs absolues (LASSO), ce qui détermine ainsi un ensemble de données d'entraînement réduit, et
(ii) une régression pas à pas ultérieure, ce qui détermine ainsi l'indicateur d'âge, la régression pas à pas étant de préférence appliquée sur ledit ensemble de données d'entraînement réduit,
dans lequel les variables indépendantes sont les niveaux de méthylation des séquences d'ADN génomique et de préférence dans lequel la variable dépendante est l'âge,
dans lequel l'indicateur d'âge comprend
(i) un sous-ensemble de l'ensemble de séquences d'ADN génomique pris comme ensemble et
(ii) au moins un coefficient par séquence d'ADN génomique contenue dans l'ensemble, et
dans lequel l'ensemble de données d'entraînement réduit comprend toutes les données de l'ensemble de données d'entraînement excepté les niveaux de méthylation de l'ADN des séquences d'ADN génomique qui sont éliminées par le LASSO.

2. Procédé mis en oeuvre par ordinateur pour déterminer l'âge d'un individu comprenant les étapes suivantes :
(a) obtention d'un ensemble de données d'entraînement d'une pluralité d'individus comprenant, pour chaque individu,
(i) les niveaux de méthylation de l'ADN d'un ensemble de séquences d'ADN génomique et
(ii) l'âge chronologique, et
(b) application sur l'ensemble de données d'entraînement d'un procédé de régression comprenant
(i) un opérateur de sélection et réduction par moindres valeurs absolues (LASSO), ce qui détermine ainsi un ensemble de données d'entraînement réduit, et
(ii) une régression pas à pas ultérieure, ce qui détermine ainsi l'indicateur d'âge, la régression pas à pas étant de préférence appliquée sur ledit ensemble de données d'entraînement réduit,
les variables indépendantes étant les niveaux de méthylation des séquences d'ADN génomique et la variable dépendante étant de préférence l'âge,
l'indicateur d'âge comprenant
(i) un sous-ensemble de l'ensemble de séquences d'ADN génomique pris comme ensemble et
(ii) au moins un coefficient par séquence d'ADN génomique contenue dans l'ensemble, et
l'ensemble de données d'entraînement réduit comprenant toutes les données de l'ensemble de données d'entraînement excepté les niveaux de méthylation de l'ADN des séquences d'ADN génomique qui sont éliminées par le LASSO, et
(c) obtention des niveaux de méthylation de l'ADN de l'individu pour lequel l'âge doit être déterminé à partir des séquences d'ADN génomique comprises dans l'indicateur d'âge, et
(d) détermination de l'âge de l'individu sur la base de ses niveaux de méthylation de l'ADN et de l'indicateur d'âge.

3. Procédé selon la revendication 1 ou 2, dans lequel la régression pas à pas est une élimination bidirectionnelle dans laquelle les variables indépendantes statistiquement non significatives sont retirées, de préférence dans lequel le seuil de signification est de 0,05.

4. Procédé selon l'une quelconque des revendications 1 à 3, dans lequel le procédé de régression ne comprend pas de régression de Ridge (régularisation L2) ou le paramètre de régularisation L2/paramètre lambda est égal à 0 et/ou le paramètre de régularisation L1/paramètre alpha du LASSO est égal à 1.

5. Procédé selon l'une quelconque des revendications 1 à 4, dans lequel l'indicateur d'âge est actualisé par itérations comprenant l'ajout des données d'au moins un individu supplémentaire aux données d'entraînement à chaque itération, ce qui élargit ainsi par itérations l'ensemble de données d'entraînement.

6. Procédé selon la revendication 5, dans lequel un cycle d'actualisation comprend l'application du LASSO sur l'ensemble de données d'entraînement élargi, ce qui détermine ainsi un ensemble de données d'entraînement réduit actualisé, et l'application de la régression pas à pas sur l'ensemble de données d'entraînement réduit actualisé, ce qui détermine ainsi un indicateur d'âge actualisé.

7. Procédé selon la revendication 5 ou 6, dans lequel la qualité de l'indicateur d'âge est déterminée, la détermination de ladite qualité comprenant les étapes suivantes :
(a) obtention d'un ensemble de données de test d'une pluralité d'individus qui n'ont pas apporté de données à l'ensemble de données d'entraînement comprenant, pour chaque dit individu,
(i) les niveaux de méthylation de l'ADN de l'ensemble de séquences d'ADN génomique comprises dans l'indicateur d'âge et
(ii) l'âge chronologique ; et
(b) détermination de la qualité de l'indicateur d'âge par évaluation statistique et/ou évaluation des limites de domaine,
dans lequel l'évaluation statistique comprend
(i) la détermination de l'âge des individus compris dans l'ensemble de données de test,
(ii) la corrélation de l'âge déterminé et de l'âge chronologique dudit/desdits individu(s) et la détermination d'un paramètre statistique décrivant cette corrélation, et
(iii) le jugement que le paramètre statistique indique une qualité acceptable de l'indicateur d'âge ou non, le paramètre statistique étant un coefficient de détermination (R²), et un R² supérieur à 0,90, de préférence supérieur à 0,98, indiquant une qualité acceptable, et
dans lequel l'évaluation des limites de domaines comprend
(iv) la détermination des limites de domaine de l'indicateur d'âge,
les limites de domaine étant les niveaux de méthylation de l'ADN minimal et maximal de chaque séquence d'ADN génomique comprise dans l'indicateur d'âge et
lesdits niveaux de méthylation de l'ADN minimal et maximal se trouvant dans l'ensemble de données d'entraînement qui a été utilisé pour déterminer l'indicateur d'âge, et
(v) la détermination que l'ensemble de données de test dépasse les limites de domaine ou non, un non-dépassement des limites de domaine indiquant une qualité acceptable ;
et dans lequel l'indicateur d'âge est actualisé quand sa qualité n'est pas acceptable.

8. Procédé selon l'une quelconque des revendications 1 à 7, dans lequel l'indicateur d'âge comprend au moins 80 séquences d'ADN génomique, et/ou dans lequel l'indicateur d'âge comprend moins de 300, de préférence moins de 150, de préférence moins de 110, de préférence moins de 100, de préférence moins de 90 séquences d'ADN génomique.

9. Procédé selon l'une quelconque des revendications 1 à 8, dans lequel les niveaux de méthylation de l'ADN des séquences d'ADN génomique d'un individu sont mesurés dans un échantillon de matière biologique dudit individu comprenant lesdites séquences d'ADN génomique, de préférence dans lequel l'échantillon comprend des cellules buccales.

10. Procédé destiné à déterminer l'âge d'un individu selon l'une quelconque des revendications 2 à 9, dans lequel l'ensemble de données d'entraînement comprend en outre au moins un facteur de style de vie associable à l'individu/aux individus tel que la consommation de drogues, les polluants environnementaux, le travail posté et le stress, et le procédé comprenant en outre une étape de détermination d'au moins un facteur de style de vie qui est associé à la différence entre l'âge déterminé et chronologique dudit individu.

11. Procédé mis en oeuvre par ordinateur pour déterminer l'âge d'un individu comprenant les étapes suivantes :
(a) obtention d'un indicateur d'âge comprenant
(I) un ensemble de séquences d'ADN génomique comprenant au moins 70 séquences d'ADN choisies dans le groupe constitué par : cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, et cg23128025 ; et
(II) au moins un coefficient par séquence d'ADN génomique contenue dans l'ensemble,
ledit indicateur d'âge comprenant moins de 300 séquences d'ADN génomique, et ledit indicateur d'âge étant de préférence obtenu par le procédé destiné à déterminer un indicateur d'âge selon l'une quelconque des revendications 1 et 3 à 9,
(b) obtention des niveaux de méthylation de l'ADN de l'individu pour lequel l'âge doit être déterminé à partir des séquences d'ADN génomique comprises dans l'indicateur d'âge, et
(c) détermination de l'âge de l'individu sur la base de ses niveaux de méthylation de l'ADN et de l'indicateur d'âge.

12. Procédé destiné à déterminer l'âge d'un individu selon l'une quelconque des revendications 2 à 11, dans lequel l'âge déterminé est un indicateur de l'âge biologique de l'individu qui se rapporte à l'état de santé et/ou la forme physique de l'individu ;
de préférence dans lequel l'état de santé se rapporte à l'état d'au moins une maladie liée au vieillissement telle que la maladie d'Alzheimer, la maladie de Parkinson, l'athérosclérose, les maladies cardio-vasculaires, le cancer, l'arthrite, la cataracte, l'ostéoporose, le diabète de type 2, l'hypertension, la dégénérescence maculaire liée à l'âge et/ou l'hypertrophie bénigne de la prostate, au moins un phénotype associé à ladite/auxdites maladie(s) liée(s) au vieillissement, et/ou le cancer ; et
de préférence dans lequel la forme physique comprend la tension artérielle, le poids corporel, le niveau de cellules immunitaires, le niveau d'inflammation et/ou la fonction cognitive de l'individu.

13. Utilisation mise en oeuvre par ordinateur d'un indicateur d'âge pour le diagnostic de l'état de santé d'un individu, ledit indicateur d'âge comprenant
(I) un ensemble de séquences d'ADN génomique comprenant au moins 70 séquences d'ADN choisies dans le groupe constitué par : cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, et cg23128025 ; et
(II) au moins un coefficient par séquence d'ADN génomique contenue dans l'ensemble ; et
dans laquelle ledit indicateur d'âge comprend moins de 300 séquences d'ADN génomique ; et
de préférence dans laquelle l'état de santé se rapporte à l'état d'au moins une maladie liée au vieillissement telle que la maladie d'Alzheimer, la maladie de Parkinson, l'athérosclérose, les maladies cardio-vasculaires, le cancer, l'arthrite, la cataracte, l'ostéoporose, le diabète de type 2, l'hypertension, la dégénérescence maculaire liée à l'âge et/ou l'hypertrophie bénigne de la prostate, au moins un phénotype associé à ladite/auxdites maladie(s) liée(s) au vieillissement, et/ou le cancer.

14. Puce comprenant un ensemble de séquences d'ADN génomique comprenant au moins 70 séquences d'ADN choisies dans le groupe constitué par : cg11330075, cg00831672, cg27320127, cg27173374, cg14681176, cg06161948, cg08224787, cg05396610, cg15609017, cg09805798, cg19215678, cg12333719, cg03741619, cg16677512, cg03230469, cg19851481, cg10543136, cg07291317, cg26430984, cg16950671, cg16867657, cg22077936, cg08044253, cg12548216, cg05211227, cg13759931, cg08686931, cg07955995, cg07529089, cg01520297, cg00087368, cg05087008, cg24724428, cg19112204, cg04525002, cg08856941, cg16465695, cg08097417, cg21628619, cg09460489, cg13460409, cg25642673, cg19702785, cg18506897, cg21165089, cg27540719, cg21807065, cg18815943, cg23677767, cg07802350, cg11176990, cg10321869, cg17343879, cg08662753, cg14911690, cg12804730, cg16322747, cg14231565, cg10501210, cg09275691, cg15008041, cg05812299, cg24319133, cg12658720, cg20576243, cg03473532, cg07381960, cg05106770, cg04320377, cg19432688, cg22519947, cg06831571, cg08194377, cg01636910, cg14305139, cg04028695, cg15743533, cg03680898, cg20088545, cg13333913, cg19301963, cg13973351, cg16781885, cg04287203, cg27394136, cg10240079, cg02536625, et cg23128025, la puce comprenant moins de 500 emplacements et chaque séquence étant contenue dans un emplacement séparé, et la puce étant appropriée pour détecter les niveaux de méthylation de l'ADN dudit ensemble de séquences d'ADN génomique.

15. Kit comprenant (i) la puce selon la revendication 14 et éventuellement (ii) un récipient pour de la matière biologique, du matériel pour un frottis buccal, une colonne de centrifugation et/ou une enzyme pour extraire, purifier et/ou amplifier de l'ADN génomique issu d'un échantillon biologique, et/ou du sulfite d'hydrogène.
